# EUROPEAN PATENT SPECIFICATION

(11) **EP 3 755 814 B1**
(45) Date of publication and mention of the grant of the patent: **09.04.2025**
(21) Application number: 19757342.1
(22) Date of filing: 22.02.2019
(51) Int. Cl.: C12Q 1/686, C12N 15/10, G01N 33/58, G01N 33/60

(54) **ASSAY FOR DETECTION OF ANDROGEN RECEPTOR VARIANTS**
TEST ZUM NACHWEIS VON ANDROGENREZEPTORVARIANTEN
DOSAGE POUR LA DÉTECTION DE VARIANTS DU RÉCEPTEUR DES ANDROGÈNES

(30) Priority: 23.02.2018 US 201862634226 P
(43) Date of publication of application: 30.12.2020
(73) Proprietor: Cornell University, Ithaca, NY 14850 (US)
(72) Inventor: GIANNAKAKOU, Paraskevi, Tenafly, New Jersey 07670 (US); GJYREZI, Ada, Glendale, New York 11385 (US); KIM, Seaho, New York, New York 10044 (US)
(74) Representative: Secerna LLP
(86) International application number: PCT/US2019/019197
(87) International publication number: WO 2019/165242

(56) References cited:
- WO-A1-2011/112581
- WO-A1-2014/047285
- WO-A1-2015/179404
- WO-A1-2016/154600
- WO-A1-2017/181161
- WO-A1-2017/207702
- JP-A- 2004 254 600
- JP-A- 2004 254 601
- JP-A- 2004 254 602
- US-A1- 2013 059 762
- US-A1- 2021 108 273
- GJYREZI ADA ET AL: "Abstract 2736: A digital droplet PCR assay for the quantitation of androgen receptor and splice variant expression in CTCs from metastatic castration resistant prostate cancer patients | Cancer Research", CANCER RESEARCH, 1 July 2017 (2017-07-01), XP055852748, Retrieved from the Internet <URL:https://cancerres.aacrjournals.org/content/77/13_Supplement/2736> [retrieved on 20211019], DOI: :10.1158/1538-7445.AM2017-2736
- YAFENG MA ET AL: "Droplet Digital PCR Based Androgen Receptor Variant 7 (AR-V7) Detection from Prostate Cancer Patient Blood Biopsies", INTERNATIONAL JOURNAL OF MOLECULAR SCIENCES, vol. 17, no. 8, 4 August 2016 (2016-08-04), pages 1 - 11, XP055589963, DOI: 10.3390/ijms17081264
- MARIA THADANI-MULERO ET AL: "Androgen Receptor Splice Variants Determine Taxane Sensitivity in Prostate Cancer", CANCER RESEARCH, vol. 74, no. 8, 20 February 2014 (2014-02-20), US, pages 2270 - 2282, XP055500385, ISSN: 0008-5472, DOI: 10.1158/0008-5472.CAN-13-2876
- TAGAWA SCOTT T ET AL: "Expression of AR-V7 and ARv567es in Circulating Tumor Cells Correlates with Outcomes to Taxane Therapy in Men with Metastatic Prostate Cancer Treated in TAXYNERGY", CLINICAL CANCER RESEARCH, 9 October 2018 (2018-10-09), pages 1880 - 1888, XP055852557, Retrieved from the Internet <URL:https://www.ncbi.nlm.nih.gov/pmc/articles/PMC6432911/pdf/nihms-1509272.pdf> [retrieved on 20211019], DOI: 10.1158/1078-0432.CCR-18-0320
- MA, Y ET AL.: "Droplet Digital PCR Based Androgen Receptor Variant 7 ( AR -V7) Detection from Prostate Cancer Patient Blood Biopsies", INTERNATIONAL JOURNAL OF MOLECULAR SCIENCES, vol. 17, no. 1264, 4 August 2016 (2016-08-04), pages 1 - 11, XP055589963, doi:10.3390/ijms17081264
- GALLETTI, G ET AL.: "Abstract 1713: Transferrin receptor 1 (TfR) as marker for circulating tumor cells (CTCs) identification in NSCLC", CANCER RESEARCH , PROCEEDINGS: AACR ANNUAL MEETING 2017, vol. 77, no. 13, July 2017 (2017-07-01), pages 1 - 4, XP055632309, Retrieved from the Internet <URL:https://cancerres.aacrjournals.org/content/77/13_Supplement/1713>
- KULASINGHE, A ET AL.: "Short term ex-vivo expansion of circulating head and neck tumour cells", ONCOTARGET, vol. 7, no. 37, 9 August 2016 (2016-08-09), pages 60101 - 60109, XP055632314

## Description

### Background

Prostate cancer (PC) is the second most common cancer diagnosed in men worldwide. Unfortunately, upon progression and metastasis, it is also the second leading cause of cancer death in men in the United States (Siegel, 2015). In addition, metastatic prostate cancer is almost always lethal.

The aberrant functioning of androgen receptor signaling is a central driving force behind prostatic tumorigenesis and its transition (or progression) into metastatic castration resistant disease (Feldman, 2001).

Methods for detecting expression of androgen receptor variants have been described previously. By way of example, Gjyrezi Ada et al (Cancer Res (2017) 77 (13_Supplement): 2736, 1 July 2017) describes an assay to measure mRNA expression of the AR full length (AR-FL) and the splice variants Arv7 and Arv567es, by using Droplet Digital PCR in CTCs isolated from CRPC patients. Yafeng ma et al (International Journal of Molecular Sciences, vol. 17, no. 8, (2016-08-04)) describes a screen for AR-V7 mRNA expression directly from circulating tumor cells (CTCs). Thadani-Mulero et al (Cancer Res (2014 Apr 15;74(8):2270-2282) describes an assay in which ARv567 expression confers docetaxel sensitivity *in vivo,* while expression of the microtubule-independent ARv7 variant confers resistance. WO 2014/047285 A1 describes a method for detecting and/or treating a subset of prostate cancer patients who may benefit from taxane treatment, wherein the method comprises testing whether an androgen receptor (AR) splice variant is present in a test sample obtained from the patient, wherein the androgen receptor variant can be ARv5,6,7 or ARv7, or a combination thereof. WO 2017/207702 A1 describes an in vitro method for determining if a tumor is resistant to androgen deprivation therapy, the method comprising determining the presence or absence of an androgen receptor splice variant selected from the group consisting of AR-V5 and AR- V10 in a tumor sample.

Hence, androgen deprivation therapy (ADT) is usually the first line of treatment for prostate cancer patients (Harris (2009); Sridhar (2014)). In addition to ADT, next-generation androgen receptor (AR) signaling inhibitors such as abiraterone, an inhibitor of androgen bio-synthesis, and enzalutamide, an antagonist of AR-ligand binding, are used in the treatment of prostate cancer.

However, many patients become resistant to ADT therapy as well to the next generation AR signaling inhibitors (enzalutamide and abiraterone). When prostate cancer patients progress following androgen receptor targeted therapies they are treated with taxane chemotherapy, the only class of cancer chemotherapy that improves survival of castration-resistant prostate cancer (mCRPC) patients. However, the effectiveness of taxanes can also be impaired by the development of drug resistance. Drug resistance is the number one cause of cancer death in such patients.

Methods of detecting drug resistance allow new therapeutic regimens to be utilized before the prostate cancer has progressed too far.

### Summary

Prostate cancer drug resistance is due to the expression of androgen receptor (AR) splice variants (AR-Vs), which lack the ligand-binding domain and are constitutively active in the nucleus (Antonarakis, 2014). For example, expression of the androgen receptor splice variants AR-v7 or AR-V567 in circulating tumor cells (CTCs) isolated from the blood of prostate cancer patients has been correlated with resistance to enzalutamide and abiraterone. There is also evidence that AR-Vs may convey cross-resistance, not only to enzalutamide and abiraterone, but also to taxanes. Hence, assessment of the presence and/or amounts of different AR variants has clinical utility.

The inventors have shown that the androgen receptor (AR) binds microtubules (MTs), the primary target of taxanes, via its hinge domain and utilizes microtubules as tracks for its nuclear translocation and transcriptional activation of target genes. To assess the chemotherapy effect on targeting the microtubule-androgen receptor (MT-AR) axis and the nuclear accumulation of AR, the mechanism in CTCs derived from chemotherapy-naive mCRPC patients receiving taxane chemotherapy (docetaxel or cabazitaxel) was investigated in a multi institutional clinical trial. The results showed that clinical response to treatment was significantly associated with lower nuclear AR expression in CTCs and such reduced AR expression was predictive of the biochemical response to treatment for the individual patient (Antonarakis, 2017).

Sensitive assay methods are described herein that involve (a) capturing circulating cancer cells (e.g., by any method); (b) extracting mRNA and (c) detecting and quantifying each of AR-FL, AR-V7, AR-V567 in parallel digital droplet PCR assays. Such methods can detect androgen receptor and androgen receptor variants from prostate cancer subjects who may be in need thereof treatment.

The assay methods described herein are sensitive when using mRNA from as little as a half of a cell (0.5 cell). These assay methods are also highly specific for each AR-V transcript and avoid co-detection of other similar variants, especially AR-V9, which is structurally similar to AR-V7. Currently available methods do not employ the highly sensitive methods described herein. For example, currently available methods do not adequately detect AR-V567, and do not have the specificity to distinguish which type of AR-variant, as well as the full-length AR (AR-FL), is being expressed and how much of each AR-variant (including AR-FL) is expressed. Currently available methods sometimes employ pre-amplification of transcripts, which is not needed in the methods described herein. No reference gene or standard curve is needed for the assay methods described herein. Instead of currently available multiplex assay procedures, separate assay mixtures can be used in the methods described herein, which helps to improve the assay sensitivity.

The claimed invention is defined in the appended claims.

The invention provides a method comprising:
a. capturing circulating cancer cells from a sample from a test subject;
b. extracting mRNA to provide a RNA sample; and
c. detecting and quantifying each of full-length androgen receptor (AR-FL), androgen receptor variant 7 (AR-V7), and androgen receptor variant 5,6,7 (AR-V567) in parallel digital droplet PCR assays;
wherein:
(i) the parallel digital droplet PCR assay(s) for detecting and quantifying for AR-FL optionally comprises the following set of primers or probes: a primer according to the sequence as set forth in SEQ ID NO: 7 and a primer according to the sequence as set forth in SEQ ID NO: 8, and optionally a probe according to the sequence as set forth in SEQ ID NO: 9;
(ii) the parallel digital droplet PCR assay(s) for detecting and quantifying AR-V7 comprises the following set of primers or probes: a primer according to the sequence as set forth in SEQ ID NO: 11 and a primer according to the sequence as set forth in SEQ ID NO: 12, and optionally a probe according to the sequence as set forth in SEQ ID NO: 13; and
(iii) the parallel digital droplet PCR assay(s) for detecting and quantifying AR-v567es optionally comprises the following set of primers or probes: a primer according to the sequence as set forth in SEQ ID NO: 15 and a primer according to the sequence as set forth in SEQ ID NO: 16, and optionally a probe according to the sequence as set forth in SEQ ID NO: 17.

In certain embodiments, the method comprises one or more digital droplet PCR assays for detecting and quantifying AR-FL, each digital droplet PCR assay for detecting and quantifying AR-FL performed in a separate droplet comprising a primer according to the sequence as set forth in SEQ ID NO: 7 and a primer according to the sequence as set forth in SEQ ID NO: 8; wherein the droplet can optionally further comprise a probe according to the sequence as set forth in SEQ ID NO: 9.

In certain embodiments, the method comprises one or more digital droplet PCR assays for detecting and quantifying for AR-V7, each digital droplet PCR assay for detecting and quantifying for AR-V7 performed in a separate droplet comprising a primer according to the sequence as set forth in SEQ ID NO: 11 and a primer according to the sequence as set forth in SEQ ID NO: 12; wherein the droplet can optionally further comprise a probe according to the sequence as set forth in SEQ ID NO: 13.

In certain embodiments, the method comprises one or more digital droplet PCR assays for detecting and quantifying AR-v567es, each digital droplet PCR assay for detecting and quantifying AR-v567es performed in a separate droplet comprising a primer according to the sequence as set forth in SEQ ID NO: 15 and a primer according to the sequence as set forth in SEQ ID NO: 16; wherein the droplet optionally further comprises a probe according to the sequence as set forth in SEQ ID NO: 17.

In certain embodiments, the primers or probes are covalently or non-covalently bonded to one or two labels fluorescent, chemiluminescent, or radioactive labels.

In certain embodiments, capturing circulating cancer cells from the sample comprises isolating cells that express transferrin receptor, optionally further comprising depletion of CD45+ cells from the sample before isolating cells that express transferrin receptor.

In certain embodiments, the test subject is healthy.

In certain embodiments, the test subject is suspected of having cancer, or metastatic cancer, or prostate cancer.

In certain embodiments, the method comprises informing the test subject or medical personnel providing medical care for the test subject of detection, quantities, and/or quantification of full-length androgen receptor (AR-FL), androgen receptor variant 7 (AR-V7), or androgen receptor variant 5,6,7 (AR-V567) levels detected or quantified in the parallel digital droplet PCR assays.

The invention provides a taxane for use in a method of treating prostate cancer in a subject, wherein the method comprises:
(i) detecting the absence of AR-V7 in a sample by a method defined herein;
(ii) providing the taxane for administration to the subject.

The invention provides a composition comprising two or more types of primers or probes with at least 15 nucleotides of SEQ ID NO: 7, 8, 9, 11, 12, 13, 15, 16, and 17, wherein one or more type of primer or probe is covalently or non-covalently bound to a label, wherein the composition comprises:
a primer having the sequence as set forth in SEQ ID NO: 11 and a primer comprising at least 15 nucleotides according to the sequence as set forth in SEQ ID NO: 12, wherein the composition can optionally further comprise a probe comprising at least 15 nucleotides according to the sequence as set forth in SEQ ID NO: 13.

In certain embodiments, the composition comprises a primer comprising at least 15 nucleotides according to the sequence as set forth in SEQ ID NO: 7 and a primer comprising at least 15 nucleotides according to the sequence as set forth in SEQ **ID** NO: 8, wherein the composition can optionally further comprise a probe comprising at least 15 nucleotides according to the sequence as set forth in SEQ **ID** NO: 9.

**In** certain embodiments, the composition comprises a primer comprising at least 15 nucleotides according to the sequence as set forth in SEQ **ID** NO: 15 and a primer comprising at least 15 nucleotides according to the sequence as set forth in SEQ **ID** NO: 16, wherein the composition can optionally further comprise a probe comprising at least 15 nucleotides according to the sequence as set forth in SEQ **ID** NO: 17.

**In** certain embodiments, the composition comprises one or more primers or probes are covalently or non-covalently bonded to one or two fluorescent, chemiluminescent, or radioactive labels.

**In** certain embodiments, the method comprises capturing circulating cancer cells from the sample, comprising:
a. depleting the sample obtained from the test subject of CD45+ cells to obtain a CD45+ depleted sample; and
b. staining the CD45+ depleted sample with a labeled reagent that binds to transferrin receptor (TfR) to obtain a TfR-labeled sample;
c. optionally quantifying the TfR-labeled cells in the TfR-labeled sample.

### Description of the Figures

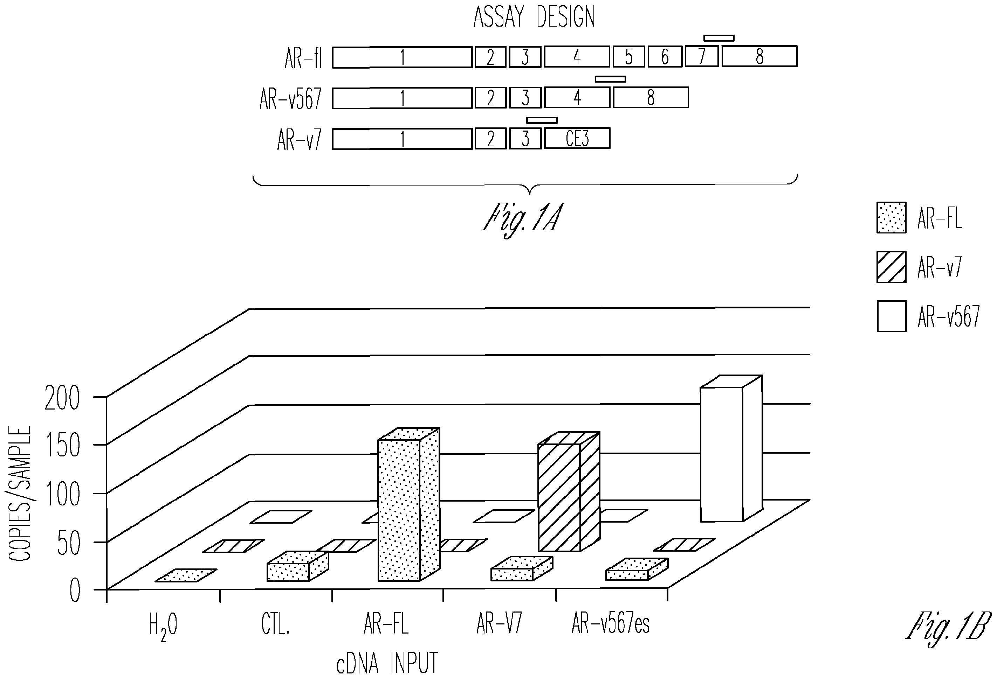

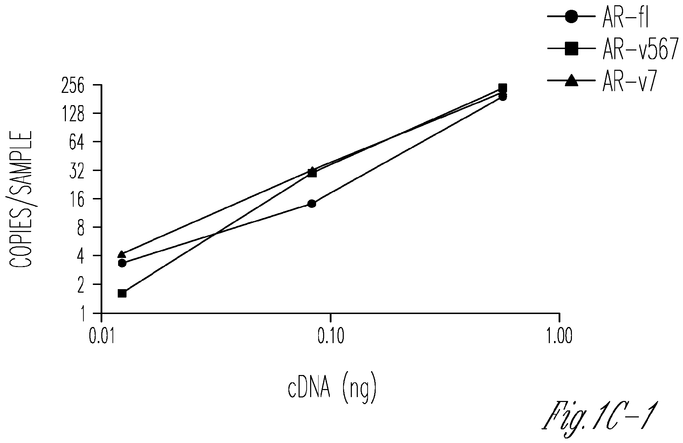

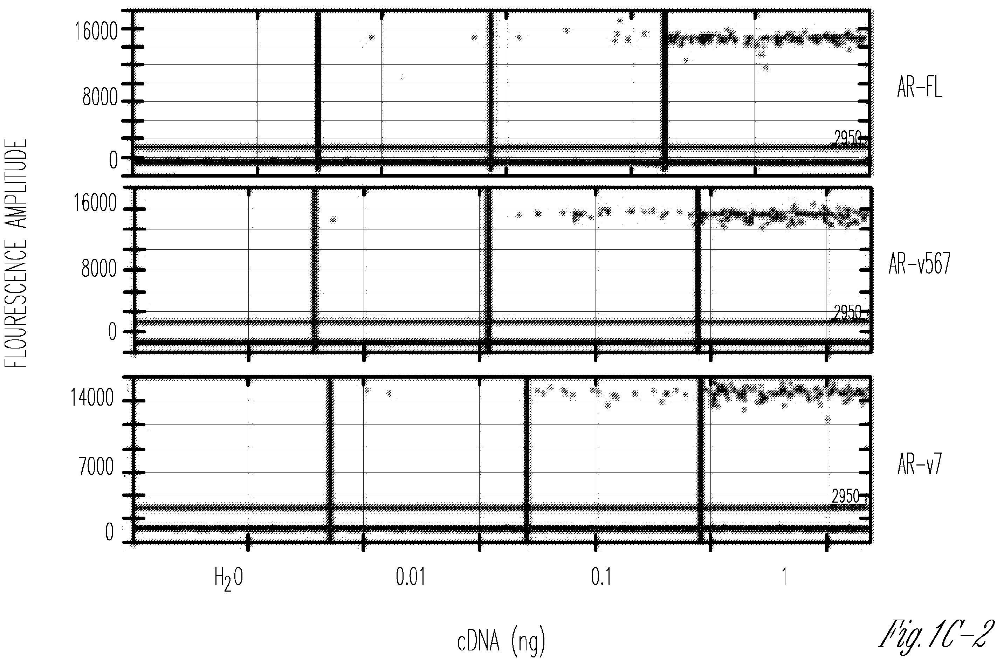

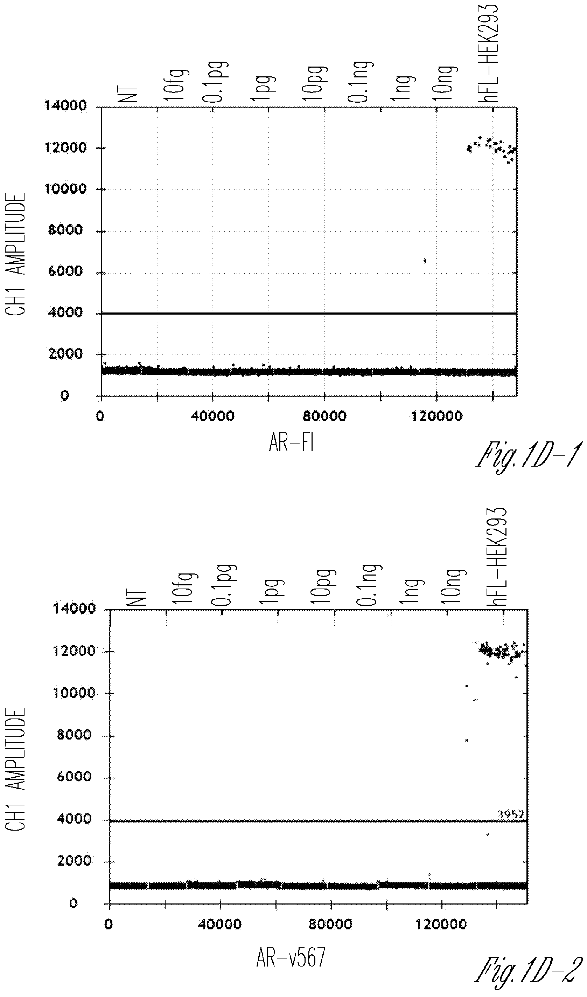

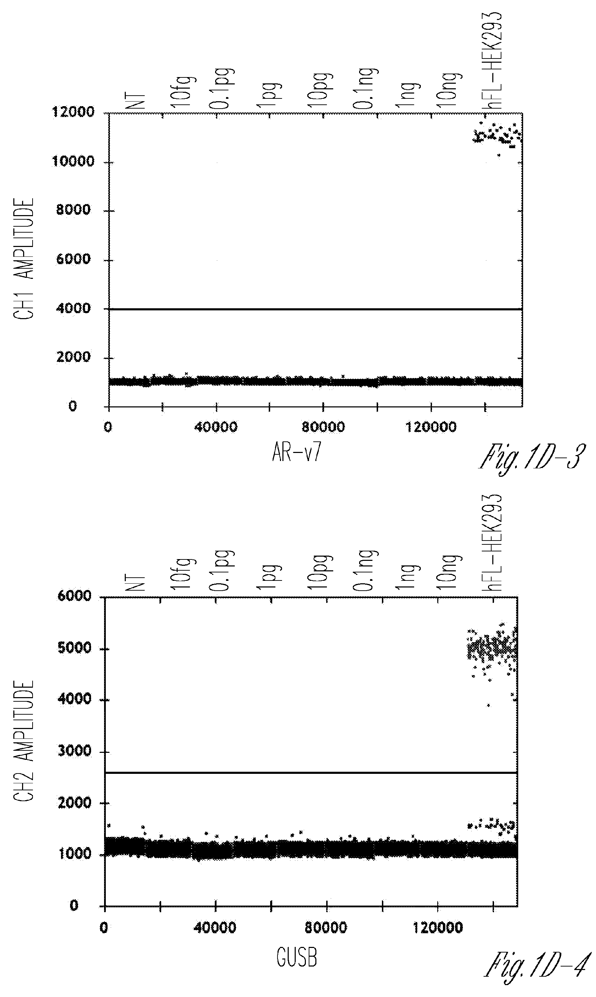

FIGs. 1A-1D illustrate the design of the assay methods described herein as well as their specificity and sensitivity. FIG. 1A schematically illustrates structure of androgen receptor and androgen receptor variants, with the positions of AR-FL, AR-v567 ("exon skipping variant" also referred to as AR-v567es), and AR-v7 primer pairs (bars above diagram). The primers were designed to be at the position of exon junctions to ensure specificity for each respective transcript and avoid non-specific signals from other variants. FIG. 1B graphically illustrates the assay specificity as the concentration (copies/sample) of transcript detected in cells transfected with empty vector (control, Ctl) or with plasmids encoding the indicated AR-Vs (cDNA input). The front row shows detection results for AR-FL, the middle row shows detection results for AR-V7, and the back row shows detection results for the exon-skipping AR variant, AR-v567es. As illustrated the method is highly specific for the AR variant that was transfected into the cells. No variant signal was detected in the non-transfected (control) HEK293T cells, while low levels of endogenous AR-FL was present in the non-transfected (control) HEK293T cells, as expected. FIG. 1C-1 and 1C-2 illustrate the analytical sensitivity of the assay as determined for each individual AR splice variant. FIG. 1C-1 graphically illustrates the sensitivity of serial dilutions of the respective DNA plasmids (1, 0.1, 0.01 ng) in triplicate for each concentration. These results showed linearity over the entire quantification range and correlation coefficients greater than 0.99 in all cases, indicating a precise log-linear relationship. FIG. 1C-2 illustrates fluorescence amplitude/droplet results, illustrating nanogram-level specificity of the assay. FIG. 1D-1 to 1D-4 show that when genomic DNA is used as input, no signal was detected, confirming the specificity of the assay. FIG. 1D-1 shows detection of AR-FL in genomic DNA. FIG. 1D-2 shows detection of AR-v567 in genomic DNA. FIG. 1D-3 shows detection of AR-V7 in genomic DNA. FIG. 1D-4 shows detection of GUSB (control) in genomic DNA.

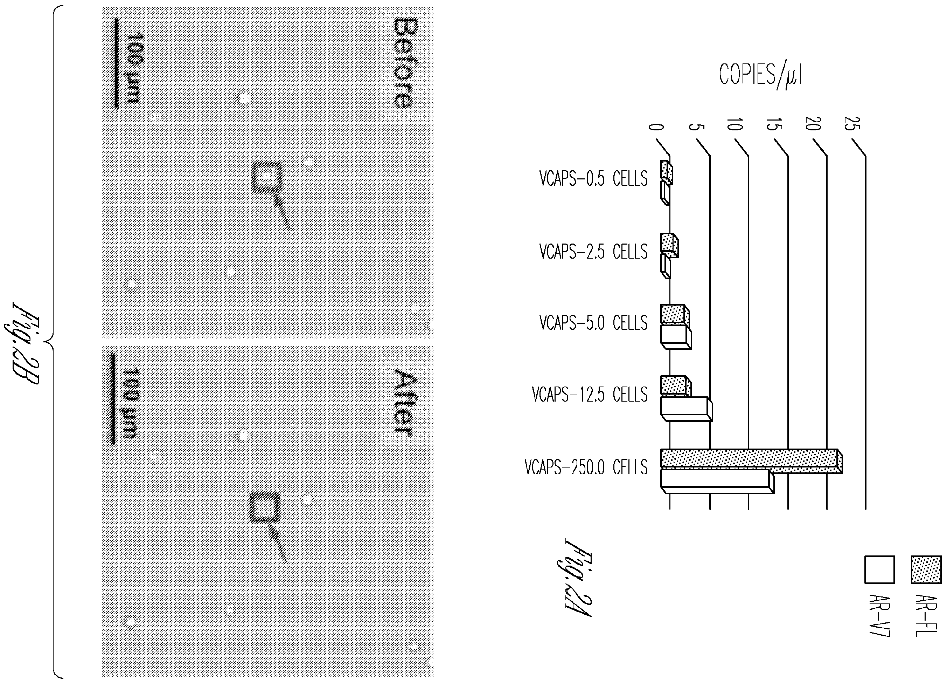

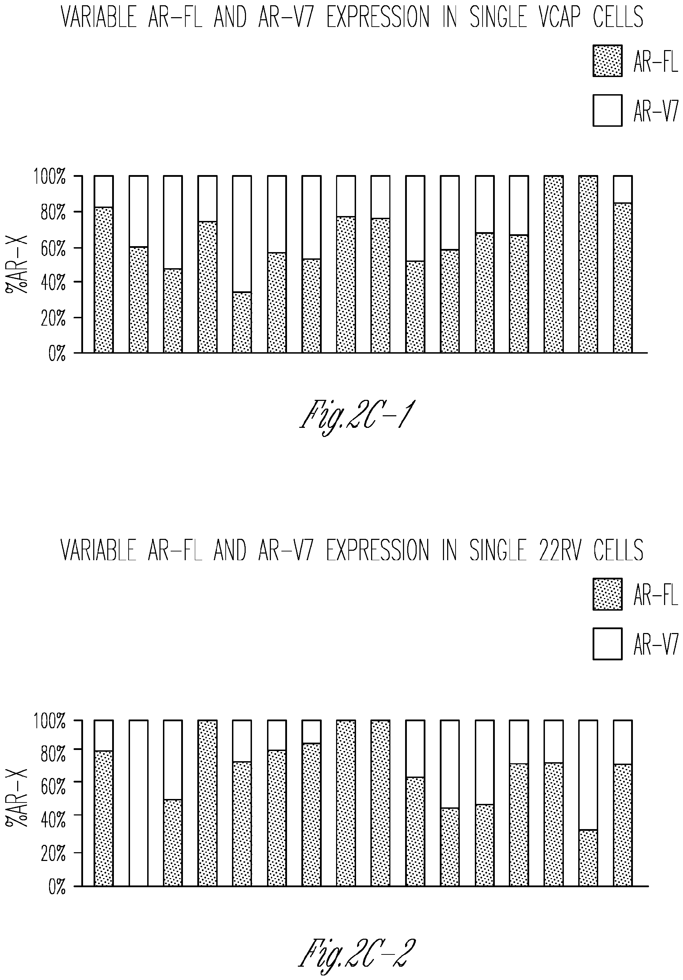

FIGs. 2A-2C illustrate the assay method sensitivity. FIG. 2A graphically illustrates validation that the assay detects low numbers androgen receptor transcripts (Copies/µl) in 0.5 VCaP prostate cancer cells, 2.5 VCaP cells, 5 VCaP cells, 12.5 VCaP cells and 250 VCaP cells. As illustrated, the AR-FL and AR-v7 mRNA transcripts were detected at levels as low as the RNA obtained from a single VCaP cell. AR-v567es transcripts were not detect in the VCaP cells. FIG. 2B illustrates that a single cell can be picked using the Cell Celector system from ALS (bracket identified by arrows). The cells shown on the left ("Before") was picked using the Cell Celector system. FIG. 2C-1 and 2C-2 illustrate the sensitivity of the assay methods by detection of AR-FL and AR-V7 in single cells isolated with Cell Celector, where FIG. 2C-1 shows results for AR-FL and AR-V7 in VCap cells and FIG. 2C-2 shows results for AR-FL and AR-V7 in 22RV cells. Each bar represents RNA input from 1 single cell split into two wells for analysis. Expression of each transcript per single cell is displayed in 100% stacked column format. Please note the cell-to-cell heterogeneity in single-cell ddPCR data.

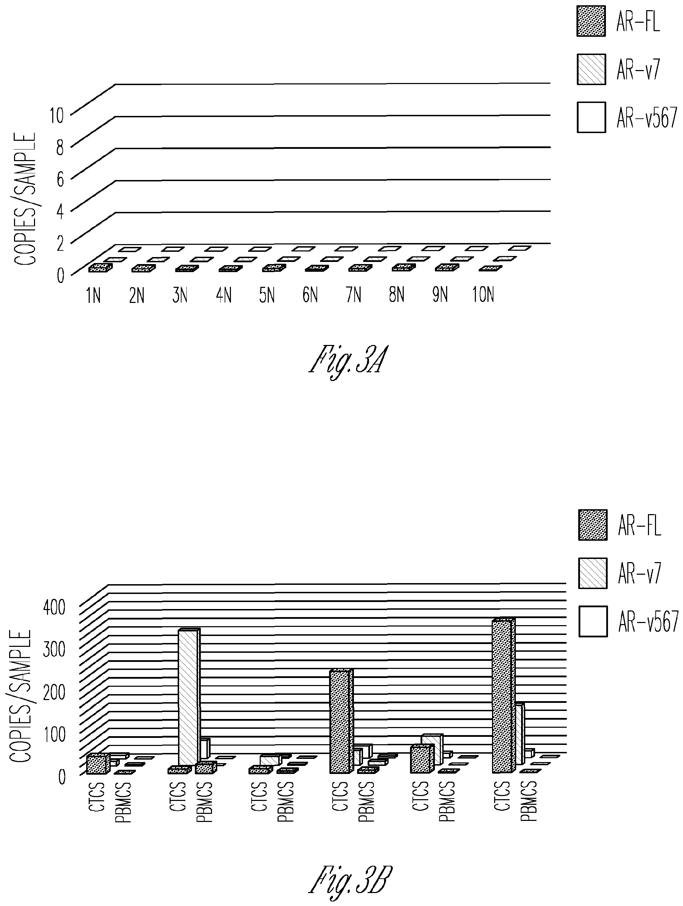

FIGs. 3A-3B illustrate validation of the assay methods in healthy volunteers and in patients with castration-resistant prostate cancer (CRPC). FIG. 3A graphically illustrates that healthy donor control PBMC samples were negative for expression of AR7 and AR-v567es variants. Low levels of AR-FL were detected in healthy donor control PBMC samples. The front row shows AR-FL results; the middle row shows results for AR-V7; and the back row shows results for AR-v567. FIG. 3B graphically illustrates AR variant expression in patient-derived circulating tumor cells (CTCs) with matching PBMC samples. The front row shows detection results for AR-FL, the middle row shows detection results for AR-V7, and the back row shows detection results for AR-v567es. Representative data from six CRPC patient CTC samples are shown in comparison to data from matching PBMCs.

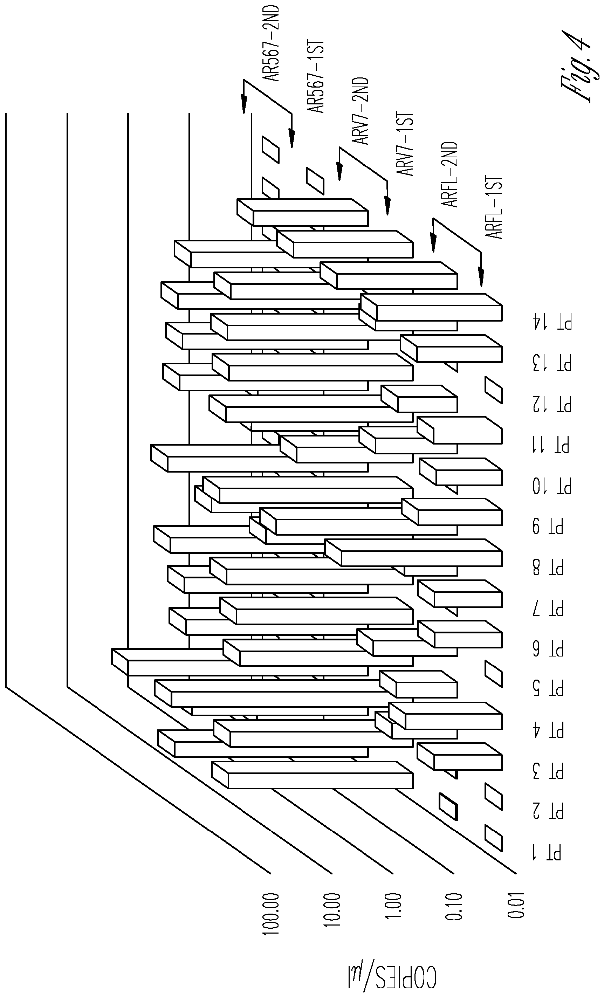

FIG. 4 graphically illustrates reproducibility of the expression levels of the AR-FL, AR-v7 and AR-v567es RNAs in duplicate clinical samples from various patients. CTCs were isolated and enriched by negative selection (Rosette Sep) from fourteen metastatic CRPC patients. RNA was extracted from the CTC samples, the RNA was split into two batches, and then stored frozen. Batch 1 was run by operator 1 and batch 2 was run by a different operator (operator 2) on a different day. ddPCR data show nearly identical results for the expression of each AR transcript when the same patient sample from the same time-point was run twice.

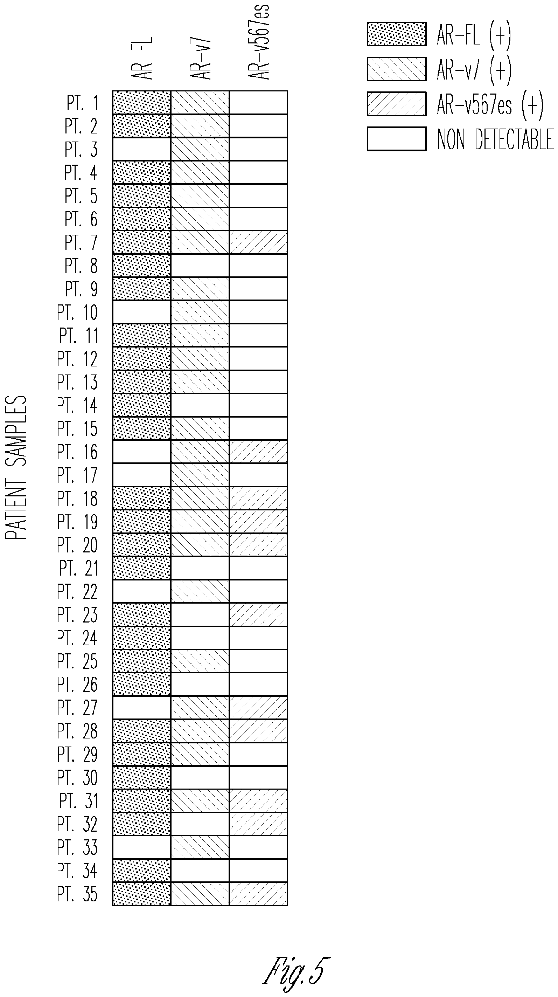

FIG. 5 visually and numerically illustrates AR splice variant expression data observed for different metastatic CRPC patients. A chart is shown illustrating which of the AR-FL, AR-V7, and AR-V567 transcripts were detected in samples from the metastatic CRPC patients. Shaded boxes indicate that an AR variant is present, while non-shaded boxes indicate that no AR variant was detected. Androgen receptor full length and androgen receptor splice variant expression was assessed by ddPCR in CTCs enriched by negative selection from 35 mCRPC patients. AR-FL was expressed in 28/38 patients (74%), AR-V7 was expressed in 26/78 patients (69%), and AR-v567es was expressed in 29% of patients (11/38). Three of the 38 patients did not express any of the three transcripts (8%), 9/38 patients (24%) were positive for both splice variants, and 7/38 (18%) were negative for both.

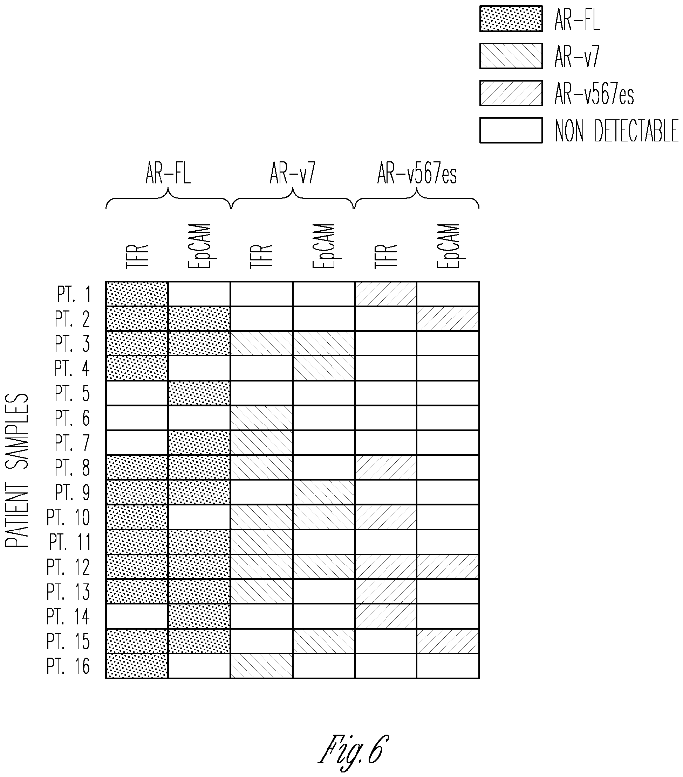

FIG. 6 illustrates that AR-V expressing cells are enriched in transferrin receptor (TfR-positive) CTCs as compared to epithelial cell-adhesion molecule (EpCAM-positive) CTCs obtained from mCRPC patients. Shaded boxes in the chart indicate that an AR variant is present, while non-shaded boxes indicate that no AR variant was detected.

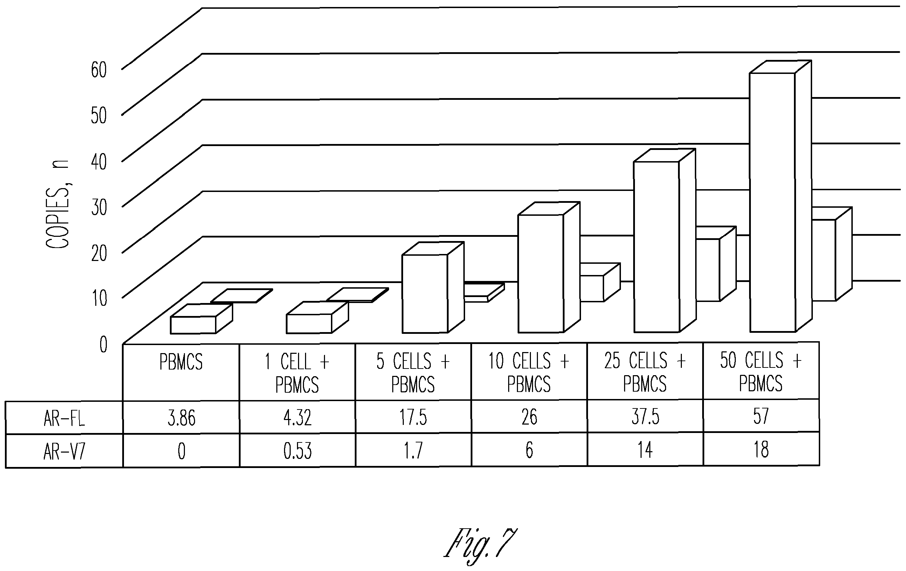

FIG. 7 illustrates AR-FL and AR-V7 mRNA expression (#copies per sample) as determined by ddPCR in varying amounts of 22RV1 cells in the presence of healthy donor blood run through the GEDI device. The front row of bars illustrates the amounts of AR-FL while the back row of bars illustrates the amounts of AR-v7. The assay reliably and reproducibly detects both transcripts in single spiked- in cells. The table below the graph shows the raw data (copy number) for each transcript per condition. Healthy donor blood PBMCs alone were used as a control.

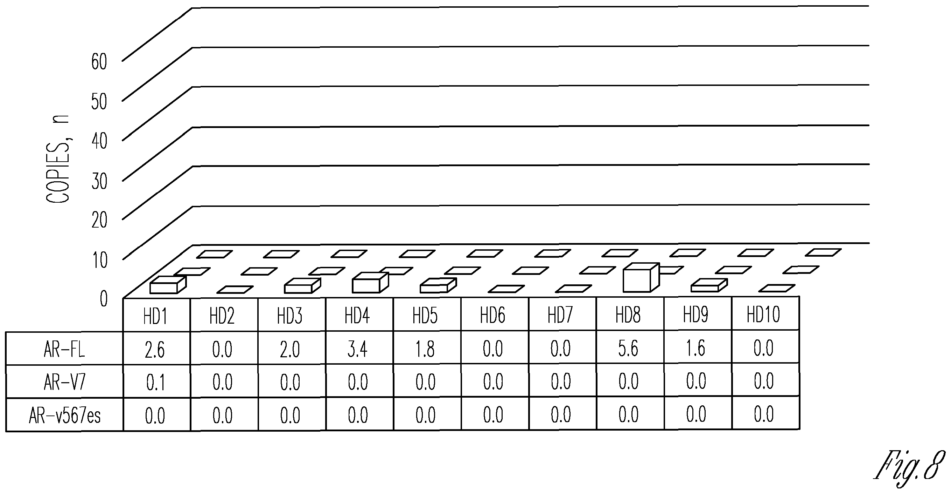

FIG. 8 illustrates mRNA expression levels of AR-FL, AR-v7 and AR-v567es that were analyzed in healthy donor blood from 10 volunteers. The front row of bars illustrates the amounts of AR-FL, the middle row of bars illustrates the amounts of AR-v7, and the back row of bars illustrates the amounts of AR-v567es. One ml of healthy donor peripheral blood was processed through the GEDI microfluidic device. The table below the graph shows the raw data (copy number) for each transcript per sample.

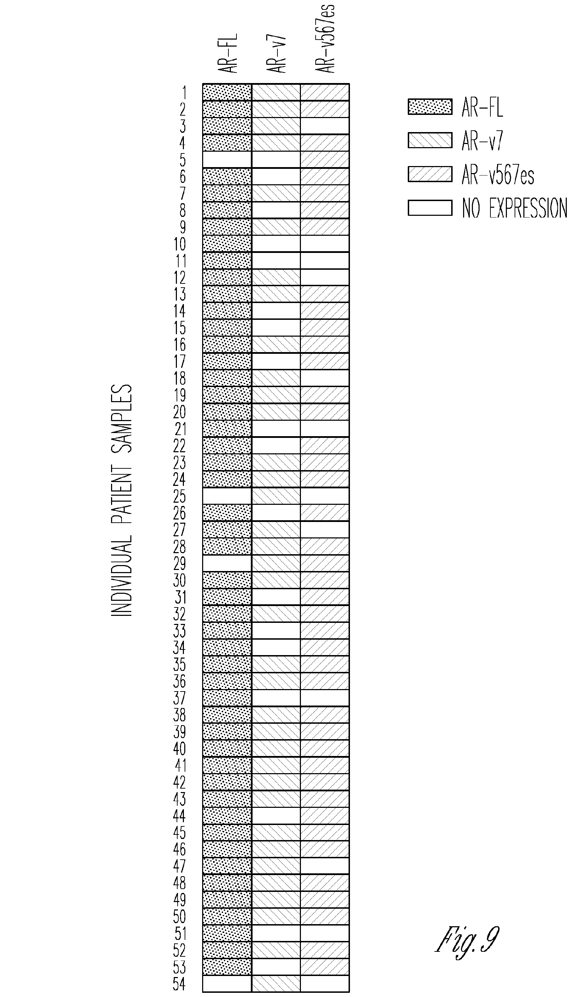

FIG. 9 illustrates which types of AR transcripts were detected in each patient sample. Shaded boxes indicate that AR-FL, AR-V7, and AR-v567es were separately detected in that patient sample, while non-shaded boxes indicate that no AR transcripts of the type identified at the top of the table were detected.

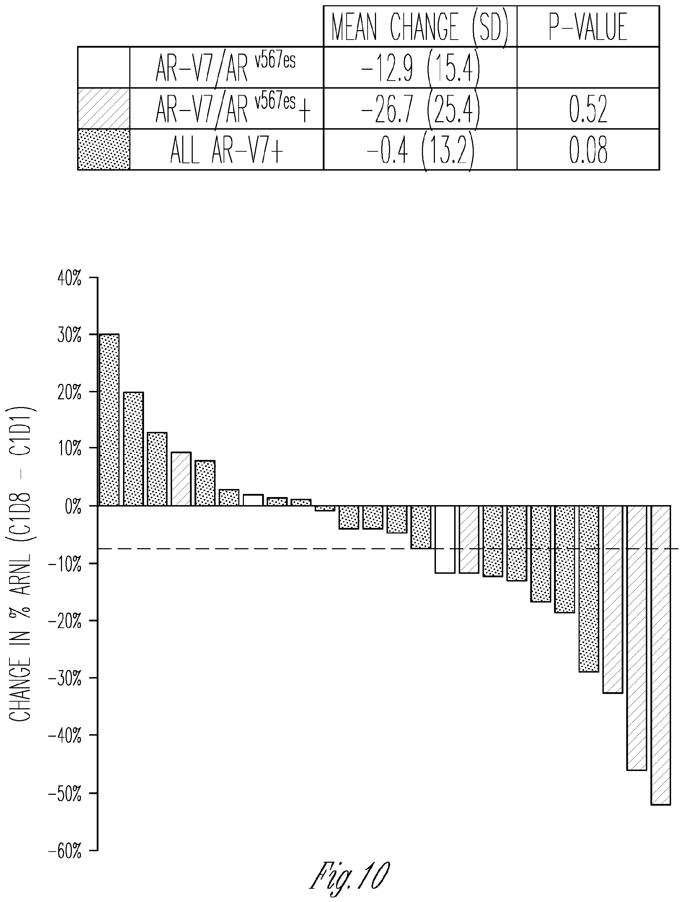

FIG. 10 graphically illustrates the percentage change in AR nuclear localization (ARNL) at treatment Cycle 1 Day 8 (C1D8) compared with treatment Cycle 1 Day 1 (C1D1, baseline) in patients stratified by AR-V status as shown by a waterfall plot, where the dotted line represents the mean change in %ARNL for all patients. The results for AR-V7-negative and AR-v567es-negative patients are shown with white (open) bars; the results for AR-V7-negative and AR-v567es-positive patients are shown with grey bars; and results for all AR-V7-positive patients are shown with dark gray bars.

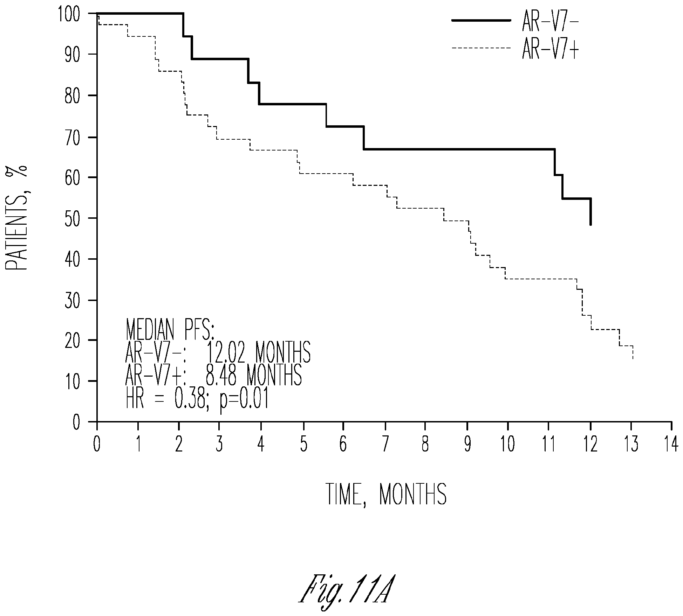

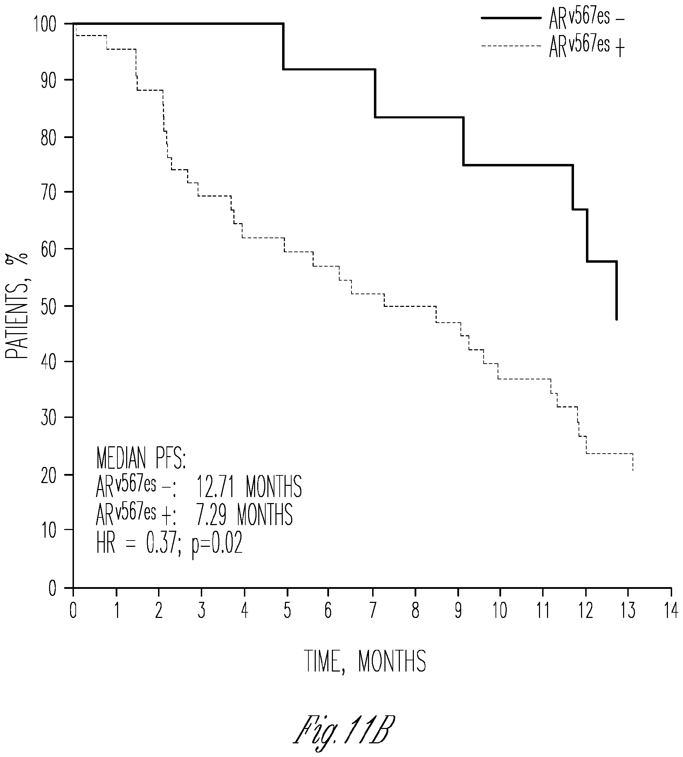

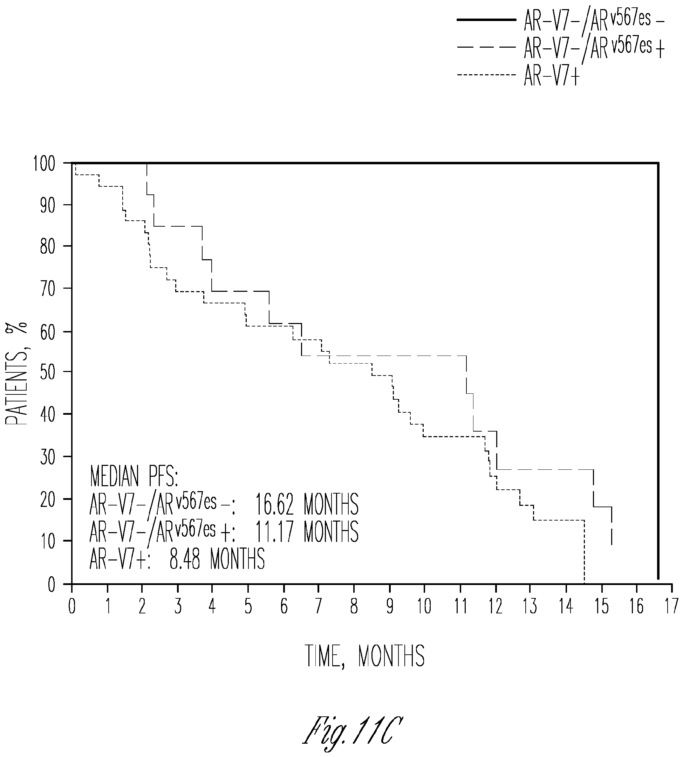

FIG. 11A-11C illustrate progression-free survival for patients expressing various types of AR. FIG. 11A shows a Kaplan-Meier curve of progression-free survival for AR-V7-negative vs AR-V7-positive patients. For AR-V7-negative (regardless of ARv567es status) vs AR-V7-positive, p = 0.01. FIG. 11B shows a Kaplan-Meier curve of progression-free survival for ARv567es-negative vs ARv567es-positive patients. For ARv567es-negative (regardless of AR-V7 status) vs ARv567es-positive, p = 0.02. FIG. 11C shows a Kaplan-Meier curve of progression-free survival for AR-V7-negative /ARv567es-negative vs AR-V7-negative /ARv567es-positive vs all AR-V7-positive. For AR-V7-negative /ARv567es-negative vs AR-V7-negative/ARv567es-positive, p = 0.18; for AR-V7-negative/ARv567es-negative vs AR-V7-positive, p = 0.004; for AR-V7-negative /ARv567es-positive vs AR-V7-positive, p = 0.32. The trend for AR-V7-negative / ARv567es-negative, AR-V7-negative / ARv567es-positive and AR-V7-positive, p = 0.0013. As illustrated, AR-V7-positive have the shortest progression-free survival while AR-V7-negative / ARv567es-negative patients exhibit the longest progression-free survival.

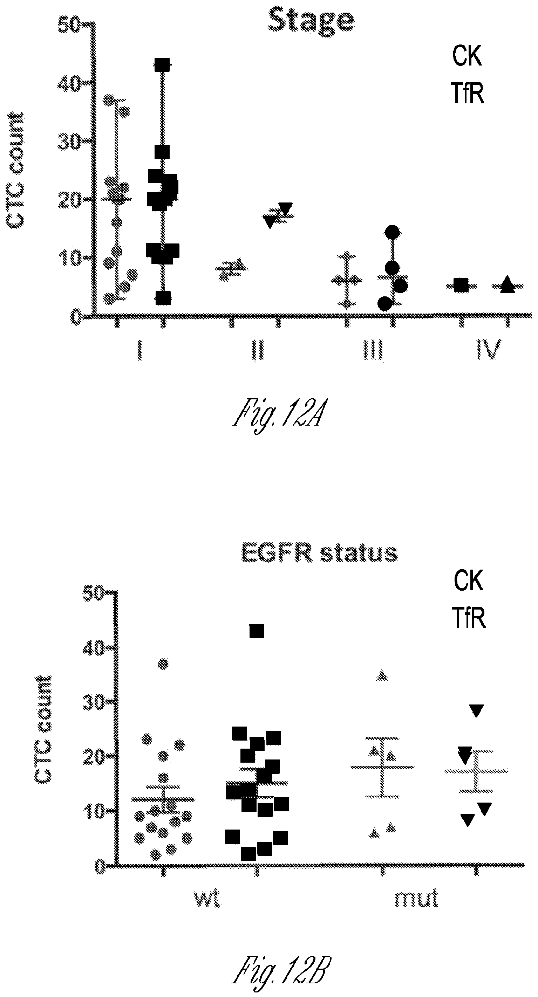

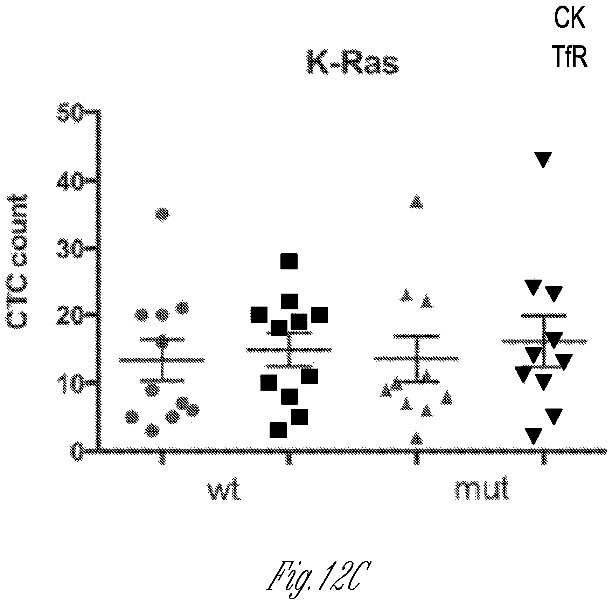

FIG. 12A-12C illustrate that TfR-positive labeling identifies cancer tumor cells (CTCs) in NSCLC patients. FIG. 12A illustrates that TfR-positive labeling identifies cancer tumor cells (CTCs) across cancer stages I-IV in early-stage (I-III) and metastatic (stage IV) NSCLC patients. FIG. 12B illustrates that TfR-positive labeling identifies cancer tumor cells (CTCs) across EGFR mutation status in early-stage NSCLC patients. FIG. 12C illustrates that TfR-positive labeling identifies cancer tumor cells (CTCs) across K-Ras mutation status in early-stage NSCLC patients.

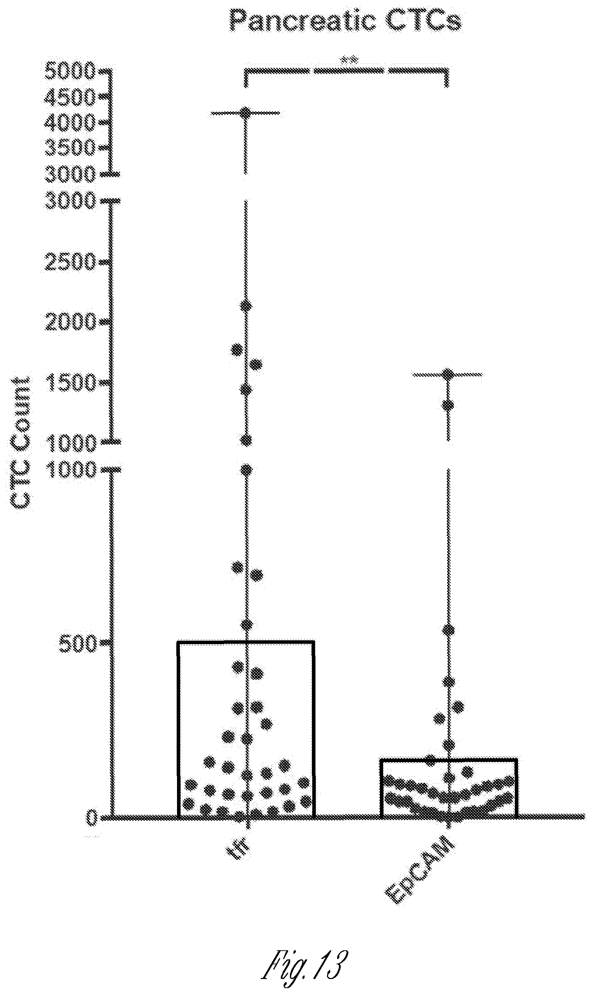

FIG. 13 shows that TfR-positive labeling identifies a more expanded pool of CTCs compared to EpCAM-positive labeling (p<0.01) in the peripheral blood of patients with pancreatic cancer (n= 43 patient samples). TfR+ and EpCAM+ CTCs were labeled and enumerated from the same tube of blood. X axis shows TfR+ CTCs and EpCAM+ CTCs; Y axis indicates CTC count; each dot represents one patient sample sample. The CTC counts according to each surface labeling are: Sample. TfR+/EpCAM- : Median CTC number: 148; range : 2-4182; EpCAM+/TfR-: median CTC number: 68; range: 0-1552

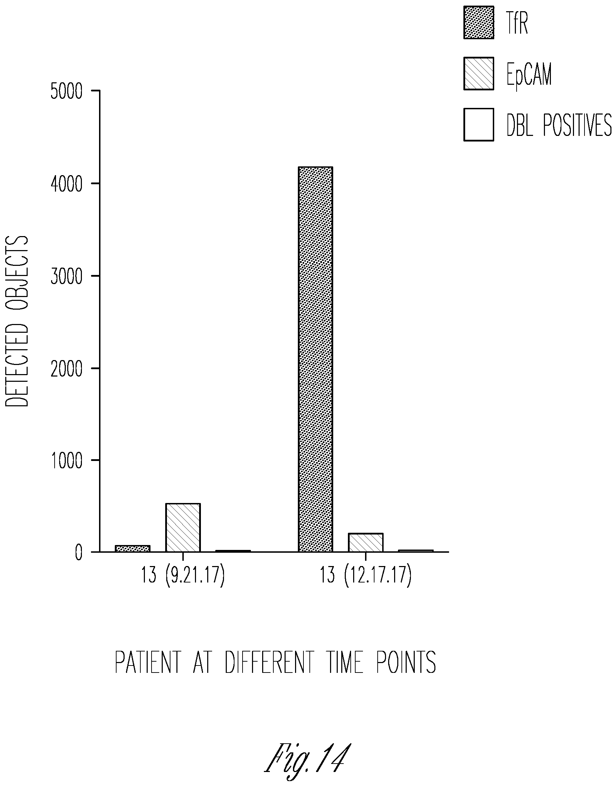

FIG. 14 shows CTC counts from the same patient (patient 13) at two different time points of blood collection. The first time point (9.21.17) was obtained when patient 13 was responding to the standard of care treatment with FOLFIRINOX chemotherapy regimen. The second time point (12.17.17) was obtained at the time of disease progression (pathological progression). TfR (black bars) refers to TfR-positive/EpCAM-negative/CD45-negative CTCs. EpCAM (light gray bars) refers to TfR-negative/EpCAM-positive/CD45-negative CTCs. Double pos (dark gray bars) refers to TfR-positive/EpCAM-positve/CD45-negative CTCs. Note that TfR+ CTCs were significantly higher at progression than EpCAM+ CTCs which decreased. Very few double positive CTCs were detected than either TfR or EpCAM CTCs. Use of transferrin receptor TfR (black bars) more accurately identified pathological progression than EpCAM.

### Detailed Description

Methods are described herein that involve (a) capturing circulating cancer cells (e.g., by any method); (b) extracting mRNA; and (c) detecting and quantifying each of AR-FL, AR-V7, and AR-V567 RNAs in parallel digital droplet PCR assays.

Any disclosures provided herein relating to methods for treatment of the human or animal body by surgery or therapy, or diagnostic methods practiced on the human or animal body, should be regarded as the disclosure of substances or compositions for use in such methods.

### Androgen Receptors

Androgen receptor variants ARv5,6,7 and ARv7 (also known as AR3) appear to be the two most clinically prevalent splice variants. The ARv5,6,7 variant is present in 59% of tumor specimens from castration-resistant prostate cancer patients, and its expression arises in response to androgen deprivation therapy or abiraterone treatment (Sun et al., J Clin Invest 120, 2715 (Aug. 2010); Mostaghel et al., Clin Cancer Res 17, 5913 (Sep 15, 2011)). The ARv7 variant is present in both benign and malignant prostate tissues but is generally enriched in metastatic disease (Gao et al., Cancer Res 69, 2305 (Mar 15, 2009); Hornberg et al., PLoS One 6, e19059 (2011)). Thus, the presence of androgen receptor splice variants is common in castration-resistant prostate cancer patients and is associated with resistance to current androgen deprivation therapies.

Sequences for various androgen receptors are available, for example, from the National Center for Biotechnology Information (see website at ncbi.nlm.nih.gov).

For example, a full length human androgen receptor (AR-FL) sequence is available from the database maintained by the National Center for Biotechnology Information (see website at ncbi.nlm.nih.gov), which has accession number P10275.2 (GI:113830) and is shown below as SEQ ID NO:1.

1 MEVQLGLGRV YPRPPSKTYR GAFQNLFQSV REVIQNPGPR
41 HPEAASAAPP GASLLLLQQQ QQQQQQQQQQ QQQQQQQQET
81 SPRQQQQQQG EDGSPQAHRR GPTGYLVLDE EQQPSQPQSA
121 LECHPERGCV PEPGAAVAAS KGLPQQLPAP PDEDDSAAPS
161 TLSLLGPTFP GLSSCSADLK DILSEASTMQ LLQQQQQEAV
181 SEGSSSGRAR EASGAPTSSK DNYLGGTSTI SDNAKELCKA
241 VSVSMGLGVE ALEHLSPGEQ LRGDCMYAPL LGVPPAVRPT
281 PCAPLAECKG SLLDDSAGKS TEDTAEYSPF KGGYTKGLEG
321 ESLGCSGSAA AGSSGTLELP STLSLYKSGA LDEAAAYQSR
361 DYYNFPLALA GPPPPPPPPH PHARIKLENP LDYGSAWAAA
401 AAQCRYGDLA SLHGAGAAGP GSGSPSAAAS SSWHTLFTAE
441 EGQLYGPCGG GGGGGGGGGG GGGGGGGGGG GGEAGAVAPY
481 GYTRPPQGLA GQESDFTAPD VWYPGGMVSR VPYPSPTCVK
521 SEMGPWMDSY SGPYGDMRLE TARDHVLPID YYFPPQKTCL
561 ICGDEASGCH YGALTCGSCK VFFKRAAEGK QKYLCASRND
601 CTIDKFRRKN CPSCRLRKCY EAGMTLGARK LKKLGNLKLQ
641 EEGEASSTTS PTEETTQKLT VSHIEGYECQ PIFLNVLEAI
681 EPGVVCAGHD NNQPDSFAAL LSSLNELGER QLVHVVKWAK
721 ALPGFRNLHV DDQMAVIQYS WMGLMVFAMG WRSFTNVNSR
761 MLYFAPDLVF NEYRMHKSRM YSQCVRMRHL SQEFGWLQIT
801 PQEFLCMKAL LLFSIIPVDG LKNQKFFDEL RMNYIKELDR
841 IIACKRKNPT SCSRRFYQLT KLLDSVQPIA RELHQFTFDL
881 LIKSHMVSVD FPEMMAEIIS VQVPKILSGK VKPIYFHTQ

The sequence of the androgen receptor can vary somewhat from one patient to another. For example, the number of the repetitive glutamine residues in androgen receptors (amino acids 58-89 of SEQ ID NO:1) can increase or decrease by any number between about 2-25 amino acids. Similarly, the number of repetitive glycine residues in androgen receptors (amino acids 446-472 of SEQ ID NO:1) can increase or decrease by any number between about 2-23 amino acids. Thus, the androgen receptor detected by the methods, reagents and devices described herein can have at least 75% sequence identity, or at least 80% sequence identity, or at least 85% sequence identity, or at least 90% sequence identity, or at least 95% sequence identity, or at least 96% sequence identity, or at least 97% sequence identity, or at least 98% sequence identity, or at least 99% sequence identity to SEQ **ID** NO:1.

Sequence identity can be evaluated using sequence analysis software (e.g., via the NCBI tools, or the Sequence Analysis Software Package of the Genetics Computer Group. University of Wisconsin Biotechnology Center. 1710 University Avenue. Madison, **WI** 53705). Such software matches similar sequences by assigning degrees of sequence identity to various substitutions, deletions, insertions, and other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

Nucleotide sequences for the full-length human androgen receptor are also available from the NCBI database. For example, a cDNA sequence for the full length human androgen receptor is available as accession number M20132.1 (GI:178627), shown below as SEQ **ID** NO:2.

1 TAATAACTCA GTTCTTATTT GCACCTACTT CAGTGGACAC
41 TGAATTTGGA AGGTGGAGGA TTTTGTTTTT TTCTTTTAAG
81 ATCTGGGCAT CTTTTGAATC TACCCTTCAA GTATTAAGAG
121 ACAGACTGTG AGCCTAGCAG GGCAGATCTT GTCCACCGTG
161 TGTCTTCTTC TGCACGAGAC TTTGAGGCTG TCAGAGCGCT
201 TTTTGCGTGG TTGCTCCCGC AAGTTTCCTT CTCTGGAGCT
241 TCCCGCAGGT GGGCAGCTAG CTGCAGCGAC TACCGCATCA
281 TCACAGCCTG TTGAACTCTT CTGAGCAAGA GAAGGGGAGG
321 CGGGGTAAGG GAAGTAGGTG GAAGATTCAG CCAAGCTCAA
361 GGATGGAAGT GCAGTTAGGG CTGGGAAGGG TCTACCCTCG
401 GCCGCCGTCC AAGACCTACC GAGGAGCTTT CCAGAATCTG
441 TTCCAGAGCG TGCGCGAAGT GATCCAGAAC CCGGGCCCCA
481 GGCACCCAGA GGCCGCGAGC GCAGCACCTC CCGGCGCCAG
521 TTTGCTGCTG CTGCAGCAGC AGCAGCAGCA GCAGCAGCAG
561 CAGCAGCAGC AGCAGCAGCA GCAGCAGCAG CAGCAAGAGA
601 CTAGCCCCAG GCAGCAGCAG CAGCAGCAGG GTGAGGATGG
641 TTCTCCCCAA GCCCATCGTA GAGGCCCCAC AGGCTACCTG
681 GTCCTGGATG AGGAACAGCA ACCTTCACAG CCGCAGTCGG

721 CCCTGGAGTG CCACCCCGAG AGAGGTTGCG TCCCAGAGCC
761 TGGAGCCGCC GTGGCCGCCA GCAAGGGGCT GCCGCAGCAG
801 CTGCCAGCAC CTCCGGACGA GGATGACTCA GCTGCCCCAT
841 CCACGTTGTC CCTGCTGGGC CCCACTTTCC CCGGCTTAAG
881 CAGCTGCTCC GCTGACCTTA AAGACATCCT GAGCGAGGCC
921 AGCACCATGC AACTCCTTCA GCAACAGCAG CAGGAAGCAG
961 TATCCGAAGG CAGCAGCAGC GGGAGAGCGA GGGAGGCCTC
1001 GGGGGCTCCC ACTTCCTCCA AGGACAATTA CTTAGGGGGC
1041 ACTTCGACCA TTTCTGACAA CGCCAAGGAG TTGTGTAAGG
1081 CAGTGTCGGT GTCCATGGGC CTGGGTGTGG AGGCGTTGGA
1121 GCATCTGAGT CCAGGGGAAC AGCTTCGGGG GGATTGCATG
1161 TACGCCCCAC TTTTGGGAGT TCCACCCGCT GTGCGTCCCA
1201 CTCCTTGTGC CCCATTGGCC GAATGCAAAG GTTCTCTGCT
1241 AGACGACAGC GCAGGCAAGA GCACTGAAGA TACTGCTGAG
1281 TATTCCCCTT TCAAGGGAGG TTACACCAAA GGGCTAGAAG
1321 GCGAGAGCCT AGGCTGCTCT GGCAGCGCTG CAGCAGGGAG
1361 CTCCGGGACA CTTGAACTGC CGTCTACCCT GTCTCTCTAC
1401 AAGTCCGGAG CACTGGACGA GGCAGCTGCG TACCAGAGTC
1441 GCGACTACTA CAACTTTCCA CTGGCTCTGG CCGGACCGCC
1481 GCCCCCTCCG CCGCCTCCCC ATCCCCACGC TCGCATCAAG
1521 CTGGAGAACC CGCTGGACTA CGGCAGCGCC TGGGCGGCTG
1561 CGGCGGCGCA GTGCCGCTAT GGGGACCTGG CGAGCCTGCA
1601 TGGCGCGGGT GCAGCGGGAC CCGGTTCTGG GTCACCCTCA
1641 GCCGCCGCTT CCTCATCCTG GCACACTCTC TTCACAGCCG
1681 AAGAAGGCCA GTTGTATGGA CCGTGTGGTG GTGGTGGGGG
1721 TGGTGGCGGC GGCGGCGGCG GCGGCGGCGG CGGCGGCGGC
1761 GGCGGCGGCG GCGGCGGCGA GGCGGGAGCT GTAGCCCCCT
1801 ACGGCTACAC TCGGCCCCCT CAGGGGCTGG CGGGCCAGGA
1841 AAGCGACTTC ACCGCACCTG ATGTGTGGTA CCCTGGCGGC
1881 ATGGTGAGCA GAGTGCCCTA TCCCAGTCCC ACTTGTGTCA
1921 AAAGCGAAAT GGGCCCCTGG ATGGATAGCT ACTCCGGACC
1961 TTACGGGGAC ATGCGTTTGG AGACTGCCAG GGACCATGTT
2001 TTGCCCATTG ACTATTACTT TCCACCCCAG AAGACCTGCC
2041 TGATCTGTGG AGATGAAGCT TCTGGGTGTC ACTATGGAGC

2081 TCTCACATGT GGAAGCTGCA AGGTCTTCTT CAAAAGAGCC
2121 GCTGAAGGGA AACAGAAGTA CCTGTGCGCC AGCAGAAATG
2161 ATTGCACTAT TGATAAATTC CGAAGGAAAA ATTGTCCATC
2201 TTGTCGTCTT CGGAAATGTT ATGAAGCAGG GATGACTCTG
2241 GGAGCCCGGA AGCTGAAGAA ACTTGGTAAT CTGAAACTAC
2281 AGGAGGAAGG AGAGGCTTCC AGCACCACCA GCCCCACTGA
2321 GGAGACAACC CAGAAGCTGA CAGTGTCACA CATTGAAGGC
2361 TATGAATGTC AGCCCATCTT TCTGAATGTC CTGGAAGCCA
2401 TTGAGCCAGG TGTAGTGTGT GCTGGACACG ACAACAACCA
2441 GCCCGACTCC TTTGCAGCCT TGCTCTCTAG CCTCAATGAA
2481 CTGGGAGAGA GACAGCTTGT ACACGTGGTC AAGTGGGCCA
2521 AGGCCTTGCC TGGCTTCCGC AACTTACACG TGGACGACCA
2561 GATGGCTGTC ATTCAGTACT CCTGGATGGG GCTCATGGTG
2601 TTTGCCATGG GCTGGCGATC CTTCACCAAT GTCAACTCCA
2641 GGATGCTCTA CTTCGCCCCT GATCTGGTTT TCAATGAGTA
2681 CCGCATGCAC AAGTCCCGGA TGTACAGCCA GTGTGTCCGA
2721 ATGAGGCACC TCTCTCAAGA GTTTGGATGG CTCCAAATCA
2761 CCCCCCAGGA ATTCCTGTGC ATGAAAGCAC TGCTACTCTT
2801 CAGCATTATT CCAGTGGATG GGCTGAAAAA TCAAAAATTC
2841 TTTGATGAAC TTCGAATGAA CTACATCAAG GAACTCGATC
2881 GTATCATTGC ATGCAAAAGA AAAAATCCCA CATCCTGCTC
2921 AAGACGCTTC TACCAGCTCA CCAAGCTCCT GGACTCCGTG
2961 CAGCCTATTG CGAGAGAGCT GCATCAGTTC ACTTTTGACC
3001 TGCTAATCAA GTCACACATG GTGAGCGTGG ACTTTCCGGA
3041 AATGATGGCA GAGATCATCT CTGTGCAAGT GCCCAAGATC
3081 CTTTCTGGGA AAGTCAAGCC CATCTATTTC CACACCCAGT
3121 GAAGCATTGG AAACCCTATT TCCCCACCCC AGCTCATGCC
3161 CCCTTTCAGA TGTCTTCTGC CTGTTATAAC TCTGCACTAC
3201 TCCTCTGCAG TGCCTTGGGG AATTTCCTCT ATTGATGTAC
3241 AGTCTGTCAT GAACATGTTC CTGAATTCTA TTTGCTGGGC
3281 TTTTTTTTTC TCTTTCTCTC CTTTCTTTTT CTTCTTCCCT
3321 CCCTATCTAA CCCTCCCATG GCACCTTCAG ACTTTGCTTC
3361 CCATTGTGGC TCCTATCTGT GTTTTGAATG GTGTTGTATG
3401 CCTTTAAATC TGTGATGATC CTCATATGGC CCAGTGTCAA

3441 GTTGTGCTTG TTTACAGCAC TACTCTGTGC CAGCCACACA

3481 AACGTTTACT TATCTTATGC CACGGGAAGT TTAGAGAGCT

3521 AAGATTATCT GGGGAAATCA AAACAAAAAA CAAGCAAACA

3561 AAAAAAAAA

An mRNA encoding an androgen receptor can have at least 75% sequence identity, or at least 80% sequence identity, or at least 85% sequence identity, or at least 90% sequence identity, or at least 95% sequence identity, or at least 96% sequence identity, or at least 97% sequence identity, or at least 98% sequence identity, or at least 99% sequence identity to an mRNA with or complementary to SEQ **ID** NO:2.

A sequence for androgen receptor variant 5,6,7es [Homo sapiens] is also available from the NCBI database National Center for Biotechnology Information (see website at ncbi.nlm.nih.gov). This androgen receptor variant lacks exons 5,6, and7. The NCBI database provides a sequence for a human androgen receptor variant 5,6,7es with accession number ACZ81436.1 (GI:270358642) (SEQ **ID** NO:3).

1 MEVQLGLGRV YPRPPSKTYR GAFQNLFQSV REVIQNPGPR
41 HPEAASAAPP GASLLLLQQQ QQQQQQQQQQ QQQQQQQQQQ
81 QETSPRQQQQ QQGEDGSPQA HRRGPTGYLV LDEEQQPSQP
121 QSALECHPER GCVPEPGAAV AASKGLPQQL PAPPDEDDSA
161 APSTLSLLGP TFPGLSSCSA DLKDILSEAS TMQLLQQQQQ
201 EAVSEGSSSG RAREASGAPT SSKDNYLGGT STISDNAKEL
241 CKAVSVSMGL GVEALEHLSP GEQLRGDCMY APLLGVPPAV
281 RPTPCAPLAE CKGSLLDDSA GKSTEDTAEY SPFKGGYTKG
321 LEGESLGCSG SAAAGSSGTL ELPSTLSLYK SGALDEAAAY
361 QSRDYYNFPL ALAGPPPPPP PPHPHARIKL ENPLDYGSAW
401 AAAAAQCRYG DLASLHGAGA AGPGSGSPSA AASSSWHTLF
441 TAEEGQLYGP CGGGGGGGGG GGGGGGGGGG GGGGGGGGEA
481 GAVAPYGYTR PPQGLAGQES DFTAPDVWYP GGMVSRVPYP
521 SPTCVKSEMG PWMDSYSGPY GDMRLETARD HVLPIDYYFP
561 PQKTCLICGD EASGCHYGAL TCGSCKVFFK RAAEGKQKYL
601 CASRNDCTID KFRRKNCPSC RLRKCYEAGM TLGARKLKKL
641 GNLKLQEEGE ASSTTSPTEE TTQKLTVSHI EGYECQPIFL
681 NVLEAIEPGV VCAGHDNNQP DSFAALLSSL NELGERQLVH
721 VVKWAKALPD CERAASVHF

The sequence of the androgen receptor splice variant v5,6,7 can vary somewhat from one patient to another. For example, the androgen receptor splice variant v5,6,7 detected by the methods, reagents and devices described herein can have at least 75% sequence identity, or at least 80% sequence identity, or at least 85% sequence identity, or at least 90% sequence identity, or at least 95% sequence identity, or at least 96% sequence identity, or at least 97% sequence identity, or at least 98% sequence identity, or at least 99% sequence identity to SEQ **ID** NO:3.

Nucleotide sequences for the human androgen receptor variant v5,6,7 are also available from the NCBI database. For example, a cDNA sequence for the SEQ **ID** NO:3 human androgen receptor variant v5,6,7 is available as accession number GU208210.1 (GI:270358641), shown below as SEQ **ID** NO:4.

1 AGGATGGAAG TGCAGTTAGG GCTGGGAAGG GTCTACCCTC
41 GGCCGCCGTC CAAGACCTAC CGAGGAGCTT TCCAGAATCT
81 GTTCCAGAGC GTGCGCGAAG TGATCCAGAA CCCGGGCCCC
121 AGGCACCCAG AGGCCGCGAG CGCAGCACCT CCCGGCGCCA
161 GTTTGCTGCT GCTGCAGCAG CAGCAGCAGC AGCAGCAGCA
201 GCAGCAGCAG CAGCAGCAGC AGCAGCAGCA GCAGCAGCAG
241 CAGCAAGAGA CTAGCCCCAG GCAGCAGCAG CAGCAGCAGG
281 GTGAGGATGG TTCTCCCCAA GCCCATCGTA GAGGCCCCAC
321 AGGCTACCTG GTCCTGGATG AGGAACAGCA ACCTTCACAG
361 CCGCAGTCGG CCCTGGAGTG CCACCCCGAG AGAGGTTGCG
401 TCCCAGAGCC TGGAGCCGCC GTGGCCGCCA GCAAGGGGCT
441 GCCGCAGCAG CTGCCAGCAC CTCCGGACGA GGATGACTCA
481 GCTGCCCCAT CCACGTTGTC CCTGCTGGGC CCCACTTTCC
521 CCGGCTTAAG CAGCTGCTCC GCTGACCTTA AAGACATCCT
561 GAGCGAGGCC AGCACCATGC AACTCCTTCA GCAACAGCAG
601 CAGGAAGCAG TATCCGAAGG CAGCAGCAGC GGGAGAGCGA
641 GGGAGGCCTC GGGGGCTCCC ACTTCCTCCA AGGACAATTA
681 CTTAGGGGGC ACTTCGACCA TTTCTGACAA CGCCAAGGAG
721 TTGTGTAAGG CAGTGTCGGT GTCCATGGGC CTGGGTGTGG
761 AGGCGTTGGA GCATCTGAGT CCAGGGGAAC AGCTTCGGGG
801 GGATTGCATG TACGCCCCAC TTTTGGGAGT TCCACCCGCT
841 GTGCGTCCCA CTCCTTGTGC CCCATTGGCC GAATGCAAAG

881 GTTCTCTGCT AGACGACAGC GCAGGCAAGA GCACTGAAGA
921 TACTGCTGAG TATTCCCCTT TCAAGGGAGG TTACACCAAA
961 GGGCTAGAAG GCGAGAGCCT AGGCTGCTCT GGCAGCGCTG
1001 CAGCAGGGAG CTCCGGGACA CTTGAACTGC CGTCTACCCT
1041 GTCTCTCTAC AAGTCCGGAG CACTGGACGA GGCAGCTGCG
1081 TACCAGAGTC GCGACTACTA CAACTTTCCA CTGGCTCTGG
1121 CCGGACCGCC GCCCCCTCCG CCGCCTCCCC ATCCCCACGC
1161 TCGCATCAAG CTGGAGAACC CGCTGGACTA CGGCAGCGCC
1201 TGGGCGGCTG CGGCGGCGCA GTGCCGCTAT GGGGACCTGG
1241 CGAGCCTGCA TGGCGCGGGT GCAGCGGGAC CCGGTTCTGG
1281 GTCACCCTCA GCCGCCGCTT CCTCATCCTG GCACACTCTC
1321 TTCACAGCCG AAGAAGGCCA GTTGTATGGA CCGTGTGGTG
1361 GTGGTGGGGG TGGTGGCGGC GGCGGCGGCG GCGGCGGCGG
1401 CGGCGGCGGC GGCGGCGGCG GCGGCGGCGG CGGCGGCGAG
1441 GCGGGAGCTG TAGCCCCCTA CGGCTACACT CGGCCCCCTC
1481 AGGGGCTGGC GGGCCAGGAA AGCGACTTCA CCGCACCTGA
1521 TGTGTGGTAC CCTGGCGGCA TGGTGAGCAG AGTGCCCTAT
1561 CCCAGTCCCA CTTGTGTCAA AAGCGAAATG GGCCCCTGGA
1601 TGGATAGCTA CTCCGGACCT TACGGGGACA TGCGTTTGGA
1641 GACTGCCAGG GACCATGTTT TGCCCATTGA CTATTACTTT
1681 CCACCCCAGA AGACCTGCCT GATCTGTGGA GATGAAGCTT
1721 CTGGGTGTCA CTATGGAGCT CTCACATGTG GAAGCTGCAA
1761 GGTCTTCTTC AAAAGAGCCG CTGAAGGGAA ACAGAAGTAC
1801 CTGTGCGCCA GCAGAAATGA TTGCACTATT GATAAATTCC
1841 GAAGGAAAAA TTGTCCATCT TGTCGTCTTC GGAAATGTTA
1881 TGAAGCAGGG ATGACTCTGG GAGCCCGGAA GCTGAAGAAA
1921 CTTGGTAATC TGAAACTACA GGAGGAAGGA GAGGCTTCCA
1961 GCACCACCAG CCCCACTGAG GAGACAACCC AGAAGCTGAC
2001 AGTGTCACAC ATTGAAGGCT ATGAATGTCA GCCCATCTTT
2041 CTGAATGTCC TGGAAGCCAT TGAGCCAGGT GTAGTGTGTG
2081 CTGGACACGA CAACAACCAG CCCGACTCCT TTGCAGCCTT
2121 GCTCTCTAGC CTCAATGAAC TGGGAGAGAG ACAGCTTGTA
2161 CACGTGGTCA AGTGGGCCAA GGCCTTGCCT GATTGCGAGA
2201 GAGCTGCATC AGTTCACTTT TGACCTGCTA ATCAAGTCAC

2241 ACATGGTGAG CGTGGACTTT CCGGAAATGA TGGCAGAGAT

2281 CATCTCTGTG CAAGTGCCCA AGATCCTTTC TGGGAAAGTC

2321 AAGCCCATCT ATTTCCACAC CCAGTGAAGC ATTGGAAACC

2361 CTATTTCCCC ACCCCAGCTC ATGCCCCCTT TCAGATGTCT

2401 TCTGCCTGTT ATAACTCTGC ACTACTCCTC TGCAGTGCCT

2441 TG

An mRNA encoding an androgen receptor variant can have at least 75% sequence identity, or at least 80% sequence identity, or at least 85% sequence identity, or at least 90% sequence identity, or at least 95% sequence identity, or at least 96% sequence identity, or at least 97% sequence identity, or at least 98% sequence identity, or at least 99% sequence identity to an mRNA with or complementary to SEQ ID NO:4.

The androgen receptor variant 7 (AR-V7) is a ligand-independent transcription factor that promotes prostate cancer resistance to AR-targeted therapies. A sequence for human androgen receptor variant 7 is available from the NCBI database with accession number ACN39559.1 (GI:224181614) (SEQ ID NO:5).

1 MEVQLGLGRV YPRPPSKTYR GAFQNLFQSV REVIQNPGPR
41 HPEAASAAPP GASLLLQQQQ QQQQQQQQQQ QQQQQQQQQQ
61 QQQQQETSPR QQQQQQGEDG SPQAHRRGPT GYLVLDEEQQ
121 PSQPQSALEC HPERGCVPEP GAAVAASKGL PQQLPAPPDE
161 DDSAAPSTLS LLGPTFPGLS SCSADLKDIL SEASTMQLLQ
201 QQQQEAVSEG SSSGRAREAS GAPTSSKDNY LGGTSTISDN
241 AKELCKAVSV SMGLGVEALE HLSPGEQLRG DCMYAPLLGV
281 PPAVRPTPCA PLAECKGSLL DDSAGKSTED TAEYSPFKGG
321 YTKGLEGESL GCSGSAAAGS SGTLELPSTL SLYKSGALDE
361 AAAYQSRDYY NFPLALAGPP PPPPPPHPHA RIKLENPLDY
401 GSAWAAAAAQ CRYGDLASLH GAGAAGPGSG SPSAAASSSW
441 HTLFTAEEGQ LYGPCGGGGG GGGGGGGGGG GGGGEAGAVA
481 PYGYTRPPQG LAGQESDFTA PDVWYPGGMV SRVPYPSPTC
521 VKSEMGPWMD SYSGPYGDMR LETARDHVLP IDYYFPPQKT
561 CLICGDEASG CHYGALTCGS CKVFFKRAAE GKQKYLCASR
601 NDCTIDKFRR KNCPSCRLRK CYEAGMTLGE KFRVGNCKHL
641 KMTRP

The sequence of the androgen receptor splice variant v7 can vary somewhat from one patient to another. For example, the androgen receptor splice-variant v7 detected by the methods, reagents and devices described herein can have at least 75% sequence identity, or at least 80% sequence identity, or at least 85% sequence identity, or at least 90% sequence identity, or at least 95% sequence identity, or at least 96% sequence identity, or at least 97% sequence identity, or at least 98% sequence identity, or at least 99% sequence identity to SEQ **ID** NO:5.

Nucleotide sequences for the human androgen receptor variant v7 are also available from the NCBI database. For example, a cDNA sequence for the SEQ **ID** NO:5 human androgen receptor variant v7 is available as accession number FJ235916.1 (GI:224181613), shown below as SEQ **ID** NO:6.

1 GACACTGAAT TTGGAAGGTG GAGGATTTTG TTTTTTTCTT
41 TTAAGATCTG GGCATCTTTT GAATCTACCC TTCAAGTATT
81 AAGAGACAGA CTGTGAGCCT AGCAGGGCAG ATCTTGTCCA
121 CCGTGTGTCT TCTTCTGCAC GAGACTTTGA GGCTGTCAGA
161 GCGCTTTTTG CGTGGTTGCT CCCGCAAGTT TCCTTCTCTG
201 GAGCTTCCCG CAGGTGGGCA GCTAGCTGCA GCGACTACCG
241 CATCATCACA GCCTGTTGAA CTCTTCTGAG CAAGAGAAGG
281 GGAGGCGGGG TAAGGGAAGT AGGTGGAAGA TTCAGCCAAG
321 CTCAAGGATG GAAGTGCAGT TAGGGCTGGG AAGGGTCTAC
361 CCTCGGCCGC CGTCCAAGAC CTACCGAGGA GCTTTCCAGA
401 ATCTGTTCCA GAGCGTGCGC GAAGTGATCC AGAACCCGGG
441 CCCCAGGCAC CCAGAGGCCG CGAGCGCAGC ACCTCCCGGC
481 GCCAGTTTGC TGCTGCAGCA GCAGCAGCAG CAGCAGCAGC
521 AGCAGCAGCA GCAGCAGCAG CAGCAGCAGC AGCAGCAGCA
561 GCAGCAGCAG CAGCAGCAGC AAGAGACTAG CCCCAGGCAG
601 CAGCAGCAGC AGCAGGGTGA GGATGGTTCT CCCCAAGCCC
641 ATCGTAGAGG CCCCACAGGC TACCTGGTCC TGGATGAGGA
681 ACAGCAACCT TCACAGCCGC AGTCGGCCCT GGAGTGCCAC
721 CCCGAGAGAG GTTGCGTCCC AGAGCCTGGA GCCGCCGTGG
761 CCGCCAGCAA GGGGCTGCCG CAGCAGCTGC CAGCACCTCC
801 GGACGAGGAT GACTCAGCTG CCCCATCCAC GTTGTCCCTG
841 CTGGGCCCCA CTTTCCCCGG CTTAAGCAGC TGCTCCGCTG
881 ACCTTAAAGA CATCCTGAGC GAGGCCAGCA CCATGCAACT
921 CCTTCAGCAA CAGCAGCAGG AAGCAGTATC CGAAGGCAGC
961 AGCAGCGGGA GAGCGAGGGA GGCCTCGGGG GCTCCCACTT
1001 CCTCCAAGGA CAATTACTTA GGGGGCACTT CGACCATTTC
1041 TGACAACGCC AAGGAGTTGT GTAAGGCAGT GTCGGTGTCC
1081 ATGGGCCTGG GTGTGGAGGC GTTGGAGCAT CTGAGTCCAG

1121 GGGAACAGCT TCGGGGGGAT TGCATGTACG CCCCACTTTT
1161 GGGAGTTCCA CCCGCTGTGC GTCCCACTCC TTGTGCCCCA
1201 TTGGCCGAAT GCAAAGGTTC TCTGCTAGAC GACAGCGCAG
1241 GCAAGAGCAC TGAAGATACT GCTGAGTATT CCCCTTTCAA
1281 GGGAGGTTAC ACCAAAGGGC TAGAAGGCGA GAGCCTAGGC
1321 TGCTCTGGCA GCGCTGCAGC AGGGAGCTCC GGGACACTTG
1361 AACTGCCGTC TACCCTGTCT CTCTACAAGT CCGGAGCACT
1401 GGACGAGGCA GCTGCGTACC AGAGTCGCGA CTACTACAAC
1441 TTTCCACTGG CTCTGGCCGG ACCGCCGCCC CCTCCGCCGC
1481 CTCCCCATCC CCACGCTCGC ATCAAGCTGG AGAACCCGCT
1521 GGACTACGGC AGCGCCTGGG CGGCTGCGGC GGCGCAGTGC
1561 CGCTATGGGG ACCTGGCGAG CCTGCATGGC GCGGGTGCAG
1601 CGGGACCCGG TTCTGGGTCA CCCTCAGCCG CCGCTTCCTC
1641 ATCCTGGCAC ACTCTCTTCA CAGCCGAAGA AGGCCAGTTG
1681 TATGGACCGT GTGGTGGTGG TGGGGGTGGT GGCGGCGGCG
1721 GCGGCGGCGG CGGCGGCGGC GGCGGCGGCG AGGCGGGAGC
1761 TGTAGCCCCC TACGGCTACA CTCGGCCCCC TCAGGGGCTG
1801 GCGGGCCAGG AAAGCGACTT CACCGCACCT GATGTGTGGT
1841 ACCCTGGCGG CATGGTGAGC AGAGTGCCCT ATCCCAGTCC
1881 CACTTGTGTC AAAAGCGAAA TGGGCCCCTG GATGGATAGC
1921 TACTCCGGAC CTTACGGGGA CATGCGTTTG GAGACTGCCA
1961 GGGACCATGT TTTGCCCATT GACTATTACT TTCCACCCCA
2001 GAAGACCTGC CTGATCTGTG GAGATGAAGC TTCTGGGTGT
2041 CACTATGGAG CTCTCACATG TGGAAGCTGC AAGGTCTTCT
2081 TCAAAAGAGC CGCTGAAGGG AAACAGAAGT ACCTGTGCGC
2121 CAGCAGAAAT GATTGCACTA TTGATAAATT CCGAAGGAAA
2161 AATTGTCCAT CTTGTCGTCT TCGGAAATGT TATGAAGCAG
2201 GGATGACTCT GGGAGAAAAA TTCCGGGTTG GCAATTGCAA
2241 GCATCTCAAA ATGACCAGAC CCTGAAGAAA GGCTGACTTG
2281 CCTCATTCAA AATGAGGGCT CTAGAGGGCT CTAGTGGATA
2321 GTCTGGAGAA ACCTGGCGTC TGAGGCTTAG GAGCTTAGGT
2361 TTTTGCTCCT CAACACAGAC TTTGACGTTG GGGTTGGGGG
2401 CTACTCTCTT GATTGCTGAC TCCCTCCAGC GGGACCAATA
2441 GTGTTTTCCT ACCTCACAGG GATGTTGTGA GGACGGGCTG
2481 TAGAAGTAAT AGTGGTTACC ACTCATGTAG TTGTGAGTAT
2521 CATGATTATT GTTTCCTGTA ATGTGGCTTG GCATTGGCAA
2561 AGTGCTTTTT GATTGTTCTT GATCACATAT GATGGGGGCC
2601 AGGCACTGAC TCAGGCGGAT GCAGTGAAGC TCTGGCTCAG
2641 TCGCTTGCTT TTCGTGGTGT GCTGCCAGGA AGAAACTTTG
2681 CTGATGGGAC TCAAGGTGTC ACCTTGGACA AGAAGCAACT
2721 GTGTCTGTCT GAGGTTCCTG TGGCCATCTT TATTTGTGTA

2761 TTAGGCAATT CGTATTTCCC CCTTAGGTTC TAGCCTTCTG
2801 GATCCCAGCC AGTGACCTAG ATCTTAGCCT CAGGCCCTGT
2841 CACTGAGCTG AAGGTAGTAG CTGATCCACA GAAGTTCAGT
2881 AAACAAGGAC CAGATTTCTG CTTCTCCAGG AGAAGAAGCC
2921 AGCCAACCCC TCTCTTCAAA CACACTGAGA GACTACAGTC
2961 CGACTTTCCC TCTTACATCT AGCCTTACTG TAGCCACACT
3001 CCTTGATTGC TCTCTCACAT CACATGCTTC TCTTCATCAG
3041 TTGTAAGCCT CTCATTCTTC TCCCAAGCCA GACTCAAATA
3081 TTGTATTGAT GTCAAAGAAG AATCACTTAG AGTTTGGAAT
3121 ATCTTGTTCT CTCTCTGCTC CATAGCTTCC ATATTGACAC
3161 CAGTTTCTTT CTAGTGGAGA AGTGGAGTCT GTGAAGCCAG
3201 GGAAACACAC ATGTGAGAGT CAGAAGGACT CTCCCTGACT
3241 TGCCTGGGGC CTGTCTTTCC CACCTTCTCC AGTCTGTCTA
3281 AACACACACA CACACACACA CACACACACA CACACACACA
3321 CACACGCTCT CTCTCTCTCT CCCCCCCCAA CACACACACA
3361 CTCTCTCTCT CACACACACA CACATACACA CACACTTCTT
3401 TCTCTTTCCC CTGACTCAGC AACATTCTGG AGAAAAGCCA
3441 AGGAAGGACT TCAGGAGGGG AGTTTCCCCC TTCTCAGGGC
3481 AGAATTTTAA TCTCCAGACC AACAAGAAGT TCCCTAATGT
3521 GGATTGAAAG GCTAATGAGG TTTATTTTTA ACTACTTTCT
3561 ATTTGTTTGA ATGTTGCATA TTTCTACTAG TGAAATTTTC
3601 CCTTAATAAA GCCATTAATA CACCCAAAAA AAAAAAAAAA
3641 A

An mRNA encoding an androgen receptor variant can have at least 75% sequence identity, or at least 80% sequence identity, or at least 85% sequence identity, or at least 90% sequence identity, or at least 95% sequence identity, or at least 96% sequence identity, or at least 97% sequence identity, or at least 98% sequence identity, or at least 99% sequence identity to an mRNA with or complementary to SEQ ID NO:6.

### Samples

Patients who are in need of evaluation for prostate cancer, or for progression of prostate cancer, or who can benefit from modified therapy for prostate cancer can provide samples for evaluation in the methods described herein. For example, patients who may be suffering from prostate cancer and/or patients who may be resistant or non-respondent to various drugs such as enzalutamide, abiraterone, taxanes, or a combination thereof can provide samples for evaluation in the methods described herein.

Taxanes refer to a class of compounds having a core ring system of three rings, A, B and C, as shown below. 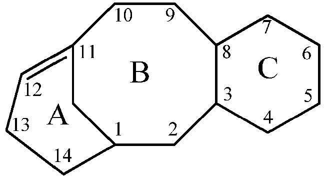 Examples of taxanes include paclitaxel, docetaxel, abraxane, and taxotere.

The sample can, for example, be circulating tumor cells, or prostate tissue sample. The development of metastases in patients with solid tumor malignancies can result from tumor cells entering the circulatory system and migrating to distant organs, where they extravasate and multiply. Circulating tumor cells (CTCs) are rare-as few as one cell per 100 million blood cells.

The samples can be obtained directly from a patient or indirectly from the patient. **In** other words, a sample can be obtained by one person and then tested or evaluated as described herein by a second person.

The sample can also, for example, be a fresh or frozen or archived paraffin-embedded and fixed (e.g. formalin-fixed) tissue sample, routinely prepared and preserved in everyday clinical practice. The sample can be a biological fluid, such as, without limitation, whole blood, peripheral blood, ascites fluid, or a combination thereof.

For example, the samples used in the methods described herein can be peripheral blood samples or the circulating tumor cells (CTCs) obtained from peripheral blood samples. Peripheral blood samples can be collected from mCRPC patients, for example, in EDTA tubes. The collected blood samples ideally are processed within 24 hours of the time of withdrawing the blood.

A variety of technologies has been developed to improve the detection and capture of circulating tumor cells from the peripheral blood. These include density gradient centrifugation, immunomagnetic bead separation using monoclonal antibodies targeting epithelial cell-surface antigens, cell sorting using flow cytometry, filtration-based size separation and microfluidic devices. Although advances in circulating tumor cell capture have been made, the low frequency of circulating tumor cells in cancer patients, their heterogeneity, the lack of organ-specific capture approaches, and the plasticity of the circulating tumor cell population has limited the ability to capture and track all circulating tumor cells. Currently, the epithelial cell-adhesion molecule (EpCAM), represents an antigen of choice for the majority of microfluidic devices that have been developed to capture circulating tumor cells. However, as illustrated herein, capture of CTCs using epithelial cell-adhesion molecule (EpCAM) may not be an optimal method for obtaining CTCs that are useful for evaluating androgen receptor expression levels. Instead, selection of CTCs that express transferrin receptor (TfR) are a better pool for evaluation of androgen receptor expression levels.

In some cases, the sample used for extraction of RNA contains circulating tumor cells (CTCs). Such circulating tumor cells can be obtained from whole blood samples by isolation and/or enrichment of the circulating tumor cells by various methods. For example, in a first method circulating tumor cells can be isolated and enriched from peripheral blood samples by using a CD45 negative depletion Rosette Sep kit. according to the manufacturer's instructions (STEMCELL Technologies Inc., Canada). In another example, a second method for isolating and enriching circulating tumor cells from peripheral blood samples can involve using the prostate-specific membrane antigen (PSMA)-based geometrically enhanced immunocapture (GEDI) as described by Kirby (2012), Galletti (2014).

### RNA Extraction

Total RNA can be extracted from the enriched circulating tumor cells pool using the *RNAeasy Plus Micro kit* (Qiagen) as per manufacturer's instructions. Aliquots of the RNA can be arrayed in separate test vessels. For example, the RNA can be arrayed in plates that have multiple wells, such as 96-well plates.

### Detection and Quantification of Androgen Receptor Variants

Droplet Digital PCR (ddPCR) can be used to quantify the mRNA levels. In many cases, Droplet Digital PCR (ddPCR) is an improved method for distinguishing and quantifying the full-length AR and the various AR variants.

RNA aliquots can be prepared for detection and/or quantitation by mixing the RNA aliquots with nucleotides and one or more RNA/DNA polymerases. For example, the RNA aliquots can be mixed with available multiplexed master mixes of PCR enzyme/buffer from the *One-Step RT ddPCR Advanced Kit for Probes* (Bio-Rad), amplicons for a GUSB control DNA region, and primers that specifically bind to, detect, and can amplify the full-length androgen receptor (AR-FL) and AR-variants.

Specific primers are used that provide improved sensitivity and specificity for the full-length androgen receptor (AR-FL) and AR-variants. Such primer sequences are shown below.

**Table 2: Primers and Probes**

| **Transcript** | **Type** | **Sequence** | **SEQ ID NO:** |
|---|---|---|---|
| AR-FL | Forward | 5'-AATCCCACATCCTGCTCAAG-3' | 7 |
| | Reverse | 5'-GCAGCCTATTGCGAGAGAG-3' | 8 |
| | Probe | 5'-ACCAGCTCACCAAGCTCCTGG-3' | 9 |
| | Fluorophore | FAM | |
| AR-V7 | Forward | 5'-AGGGATGACTCTGGGAGAAA-3' | 11 |
| | Reverse | 5'-AAAGGCTGACTTGCCTCATT-3' | 12 |
| | Probe | 5'-TCCGGGTTGGCAATTGCAAGC-3' | 13 |
| | Fluorophore | FAM | |
| AR-v567es | Forward | 5'-CTTTGCAGCCTTGCTCTCTA-3' | 15 |
| | Reverse | 5'-CTTGCCTGATTGCGAGAGAG-3' | 16 |
| | Probe | 5'-ACACGTGGTCAAGTGGGCCA-3' | 17 |
| | Fluorophore | FAM | |

In some cases, the primers and/or probes are labeled with one or more detectable labels. For example, the primers and/or probes can be labeled with 6-carboxyfluorescein (6-FAM), but other labels can be used. Labels such as 6-carboxyfluorescein (6-FAM), NED^{™} (Applera Corporation), HEX^{™} or VIC^{™} (Applied Biosystems); TAMRA^{™} labels (Applied Biosystems, CA, USA); chemiluminescent markers, Ruthenium probes; or radioactive labels (for example, tritium in the form of tritiated thymidine, ³⁵Sulfur, or ³²Pphosphorus) may also be used.

The primers and probes can have the sequences shown in Table 2. However, they can also have at least one nucleotide difference, or at least two nucleotide differences, or at least three nucleotide differences, or at least four nucleotide differences, or at least five nucleotide differences from the sequences shown in Table 2. In some cases, the primers and probes can be at least one nucleotide, or at least two nucleotides, or at least three nucleotides, or at least four nucleotides, or at least five nucleotides, or at least six nucleotides, or at least seven nucleotides longer or shorter than the sequences shown in Table 2. Typically, the primers are shorter than about 50 nucleotides, or 40 nucleotides, or 35 nucleotides, or 30 nucleotides, or 29 nucleotides, or 28 nucleotides, or 27 nucleotides, or 26 nucleotides, or 25 nucleotides. Primers are typically at least 14 nucleotides in length, or at least 15 nucleotides in length, or at least 16 nucleotides in length, or at least 17 nucleotides in length. Probes can be longer than primers. For example, probes can generally be as long as 200 nucleotides in length. However, probes are conveniently less than 175 nucleotides in length, or less than 150 nucleotides in length, or less than 125 nucleotides in length, or less than 100 nucleotides in length, or less than 75 nucleotides in length, or less than 50 nucleotides in length, or less than 40 nucleotides in length, or less than 30 nucleotides in length, or less than 25 nucleotides in length. Probes generally are at least 15 nucleotides in length, or at least 16 nucleotides in length, or at least 17 nucleotides in length, or at least 18 nucleotides in length.

In some disclosures provided herein, a set of primers can be used that can include one or more of the following pairs of primers:
Set 1: including SEQ ID NO: 7 and 8 (for detecting / quantifying AR-FL);
Set 2: including SEQ ID NO: 11 and 12 (for detecting / quantifying AR-V7);
Set 3: including SEQ ID NO: 15 and 16 (for detecting /quantifying AR-v567es); or combinations thereof.

In some cases, probes can be used that include one or more of the following sequences:
SEQ ID NO:9 (for detecting / quantifying AR-FL);
SEQ ID NO:13 (for detecting / quantifying AR-V7);
SEQ ID NO:17 (for detecting / quantifying AR-v567es); or combinations thereof.

Hence, for example, primers can have between 10 to 30 nucleotides, for example any range between 10 and 30 nucleotides, such as between 10 and 25 nucleotides, between 15 and 30 nucleotides, between 18 and 25 nucleotides, between 18 and 30 nucleotides, between 10 and 20 nucleotides, or between 15 and 20 nucleotides etc.

For example, a "probe" can be an oligonucleotide that forms a hybrid structure with a target sequence contained in a molecule in a sample undergoing analysis, due to the complementarity of at least one sequence in the probe with the target sequence. Probes can include oligonucleotide sequences, for example, that are between 15 to 40 nucleotides, for example any range between 15 and 40 nucleotides, such as between 15 and 30 nucleotides, between 15 and 25 nucleotides, between 18 and 25 nucleotides, between 18 and 30 nucleotides, between 17 and 27 nucleotides, between 18 and 25 nucleotides, or between 17 and 24 nucleotides etc.

Assay methods for detecting and/or quantifying androgen receptors can include methods such as real-time PCR, end-point PCR; end-point PCR with fluorescence detection, quantitative PCR, digital PCR, open- array PCR, digital drop PCR, quantitative digital PCR, quantitative real-time PCR, PCR suitable for high-throughput, and microarray detection / quantification methods.

The term "PCR" relates to polymerase chain reaction which is a procedure involving target amplification. The term "target amplification" relates to an enzymemediated procedure which is capable of producing billions of copies of nucleic acid target sequences. Procedures for PCR as a target amplification method are available to those of ordinary skill in the art. In general, conducting PCR involves mixing a sample of DNA, cDNA or RNA in a solution with at least two oligonucleotide primers that are prepared to be complementary to each strand of a DNA duplex, cDNA duplex, or an RNA:cDNA hybrid template. Nucleotide triphosphates (e.g., dNTPs) and a DNA polymerase, such as Taq polymerase, are used to catalyze the formation of DNA from the oligonucleotide primers and the dNTPs. At least one of the primers is a so called forward primer binding in 5' to 3' direction to the 3' end of the first strand of the DNA; the so called reverse primer is binding in 3' to 5' direction to the 5' end of the second strand of the DNA. The general principle of the PCR procedure foresees that the solution is heated to denature the double- stranded DNA to single-stranded DNA. After cooling down of the solution to the so called annealing temperature, the primers are able to bind to the separated DNA strands and the DNA polymerase catalyzes the generation of a new strand by joining the dNTPs to the primers. This process is repeated in several cycles resulting in a respective amount of amplified PCR products. The term "real-time PCR" relates to the detection of PCR products via fluorescence signals which are generated by cleavage of a dual labeled probe during hybridization of the PCR product. A dual labeled probe has a fluorescence dye and a quencher moiety. Examples of commonly used probes are TAQMAN^{®} probes.

In some cases, a digital PCR system can be employed such as a droplet digital PCR system. The term "droplet digital PCR" refers to a digital PCR system in which the reaction area is a droplet of water in a well. Preferably, the digital PCR system is an emulsion droplet digital PCR system. The term "emulsion droplet digital PCR" refers to a digital PCR system in which the reaction area is a droplet that is formed in a water-oil emulsion. Techniques for performing droplet digital PCR and emulsion droplet digital PCR are available and include, but are not limited to, those described in Hindson et al., Anal Chem, 83:8604-8610 (2011); Pinheiro et al., Anal Chem, 84:1003-1011 (2012); and Jones et al., J. Virological Methods, 202: 46-53 (2014). Droplet digital PCR systems and emulsion droplet digital PCR systems also are commercially available from sources such as, for example, the QX200^{™} DROPLET DIGITAL^{™} PCR system (Bio-Rad Laboratories, Inc., Hercules, Calif.).

There are several intrinsic advantages to ddPCR compared to traditional qPCR (Hindson, Nat Methods, Oct;10(10):1003-5 (2013); Doi, 2015; Huggett, PLoS One 8(9):e75296 (2013); Racki, Plant Methods 10(1):42,014-0042-6 (2014)). First, ddPCR allows absolute quantification without the need for normalization, calibrator or external references (Zhao et al., PLoS One 11(7):e0159004 (2016). This is because Poisson statistics allow direct estimation of template copies. Second, ddPCR provides a direct measurement expressed as number of copies of target per microliter of reaction (with confidence intervals) (Hindson, 2013). Third, because ddPCR is an endpoint binary assay, it is relatively insensitive to technical issues such as PCR inhibitors (Doi, 2015; Huggett, 2013; Racki, 2014). Fourth, unlike traditional qPCR, ddPCR has predicable technical measurement error because the underlying binomial distribution can be used to directly compute confidence intervals (Dube et al. PLoS One, 3(8):e2876 (2008). 5) ddPCR has been shown to have increased precision and sensitivity in detecting low template copies (Brunetto, J Neurovirol. 20(4):341-51 (2014); Sanders, PLoS One 8(9):e75296 (2013); Zhao et al., J Vet Diagn Invest. 27(6):784-8 (2015)). Sixth, ddPCR assays can be predictably and reliably run multiplexed. There are now hundreds of publications that underline the benefits of ddPCR and guidelines have been developed to ensure excellent data quality, precision and reproducibility for this highly sensitive technique (Huggett, 2013).

Due to the high sequence analogy of AR-v7 and AR-v9 recently published by Dehm's group (see, e.g., Kohli et al. Clin Cancer Res 23(16):4704-4715 (2017)), the inventors investigated the expression profiles of AR-v7, AR-v9 and AR-FL in RNA-Seq already published prostate cancer patient data. The inventors acquired access to RNA-Seq data of 556 primary prostate cancer patients from TCGA (The Cancer Genome Atlas) and 98 CRPC patients from Robinson et al. (2015) study. Raw sequencing reads were trimmed using Trimmomatic and aligned to human reference genome (version hg38) using STAR. Determination of expression for AR-v7 was examined based on mapped reads across junction between exon3 and CE3. Reads for AR-v9 were extracted from junction reads between exon 3 and CE5 (Kohli et al., 2017). For AR-fly, expression was determined through counting mapped reads across junction between exon7 and exon8 of AR gene.

**In** the primary prostate cancer samples, 98% of the 556 samples were AR-FL positive, 29% were AR-v7 positive and 4% were AR-v9 positive. Interestingly, in the 98 CRPC samples, 97% were AR-FL positive similar to primary prostate cancer patients. However, the expression of both AR variants was much higher in the CRPC compared to the primary samples: 79.6% and 73.5% were Ar-v7 and Ar-v9 positive respectively. This significant increase in the presence of AR-v7 and Ar-v9 in CRPC patients emphasizes the importance of the AR-V in the development of drug resistance and its role in mCRPC.

One or more androgen receptor full-length and/or androgen receptor variant proteins can therefore be expressed at higher levels in subjects with prostate cancer and/or in subjects with drug-resistant cancers (e.g., drug resistant prostate cancer) than in healthy persons (e.g., in subjects without prostate cancer). For example, androgen receptor full-length and/or androgen receptor variant proteins can be expressed at 10% higher, or 20% higher, or 30% higher, or 50% higher, or 60% higher, or 70% higher, or 80% higher, or 100% higher levels in subjects with prostate cancer and/or in subjects with drug-resistant cancers (e.g., drug resistant prostate cancer) than in healthy persons (e.g., in subjects without prostate cancer). **In** some cases, one or more androgen receptor full-length and/or androgen receptor variant proteins can be expressed at two-fold higher, or three-fold higher, or four-fold higher, or five-fold higher, or seven-fold higher, or eight-fold higher, or ten-fold higher, or twelve-fold higher, or fourteen-fold higher, or fifteen-fold higher, or seventeen-fold higher, or twenty-fold higher, or twenty-two-fold higher, or twenty-five-fold higher, or thirty-fold higher levels in subjects with prostate cancer and/or in subjects with drug-resistant cancers (e.g., drug resistant prostate cancer) than in healthy persons (e.g., in subjects without prostate cancer).

The assay methods described herein (shown in the first row) were compared to those obtained by other methods. Such a comparison is illustrated, for example, in Table 2, where the features of the assay methods described herein are shown in the first row.

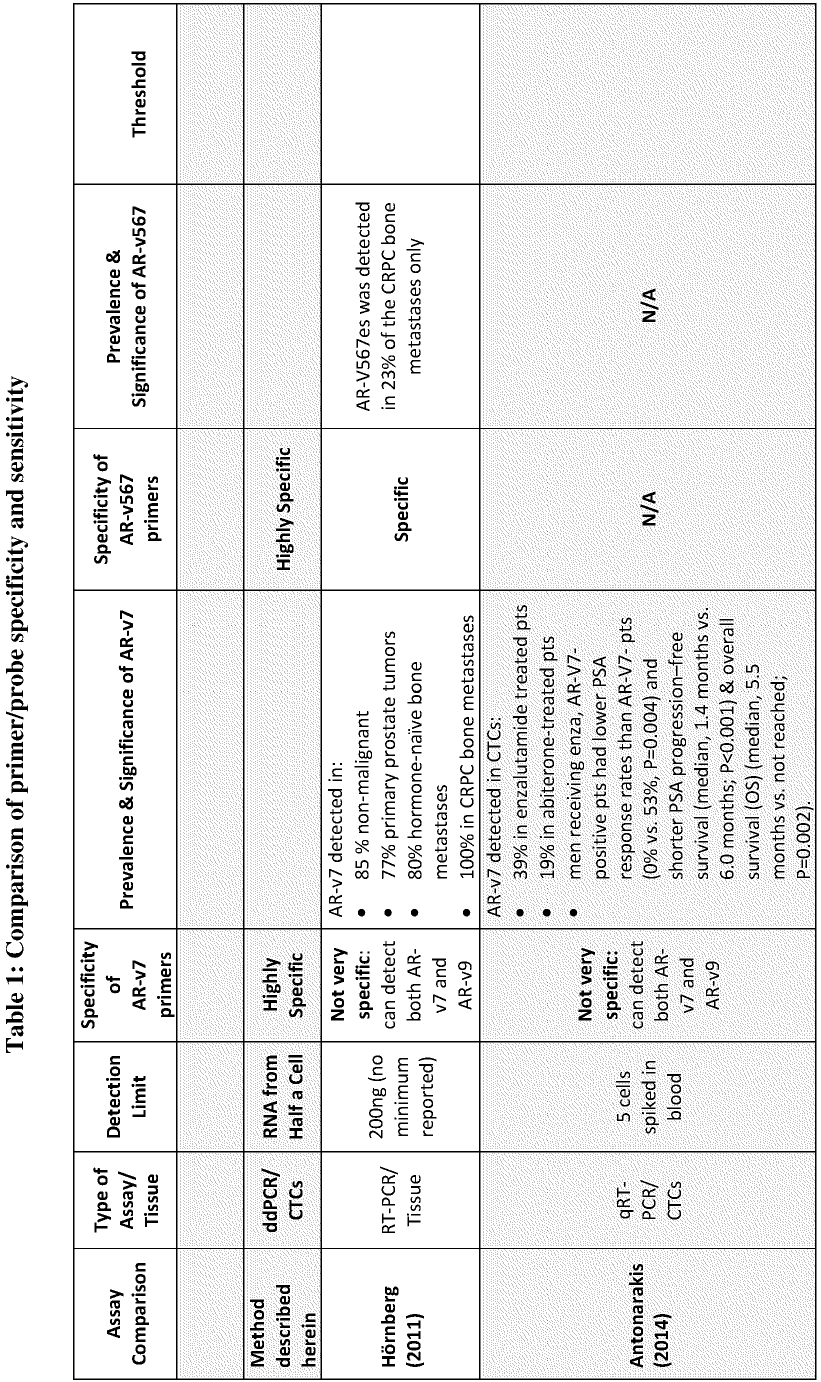

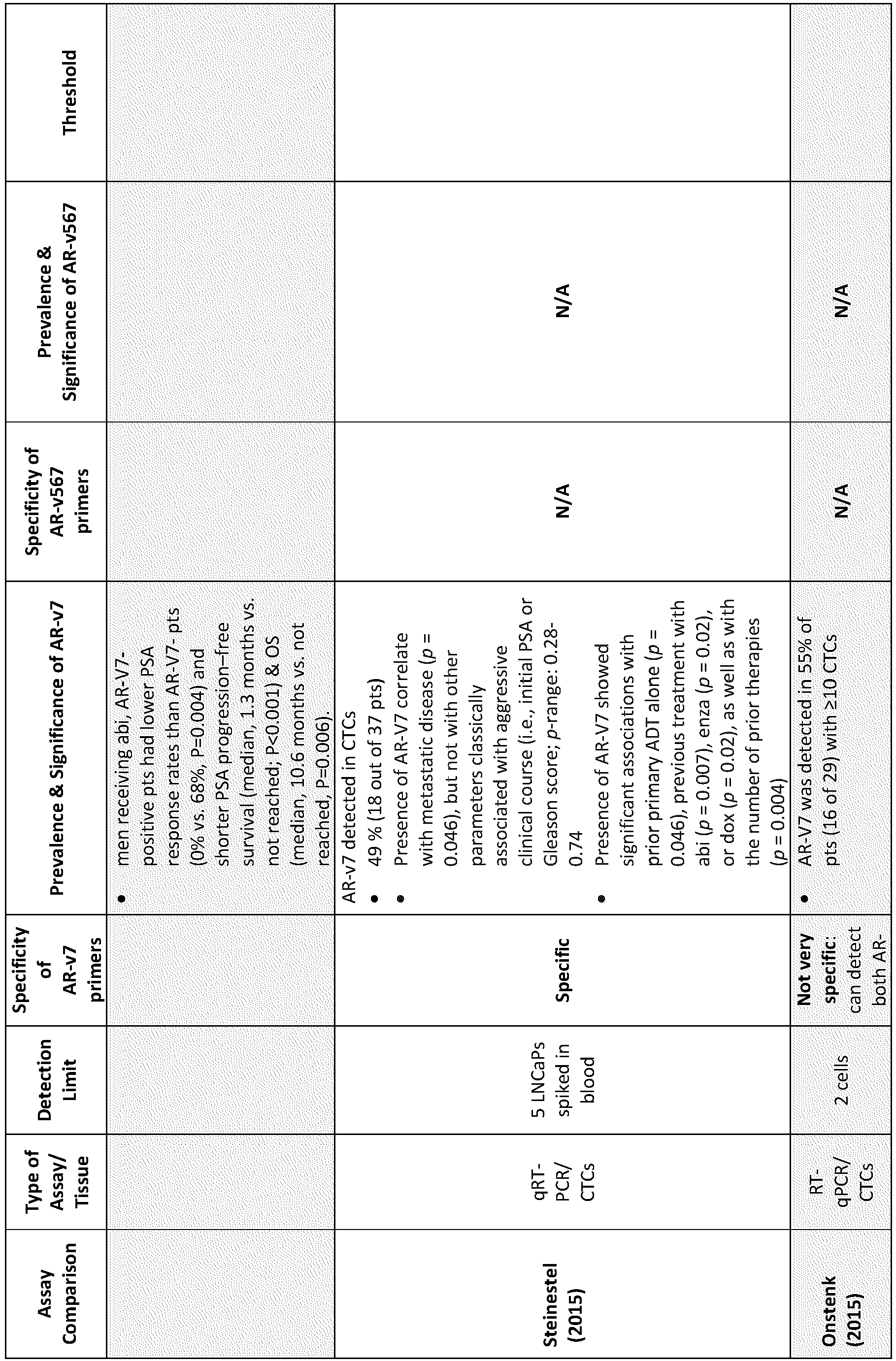

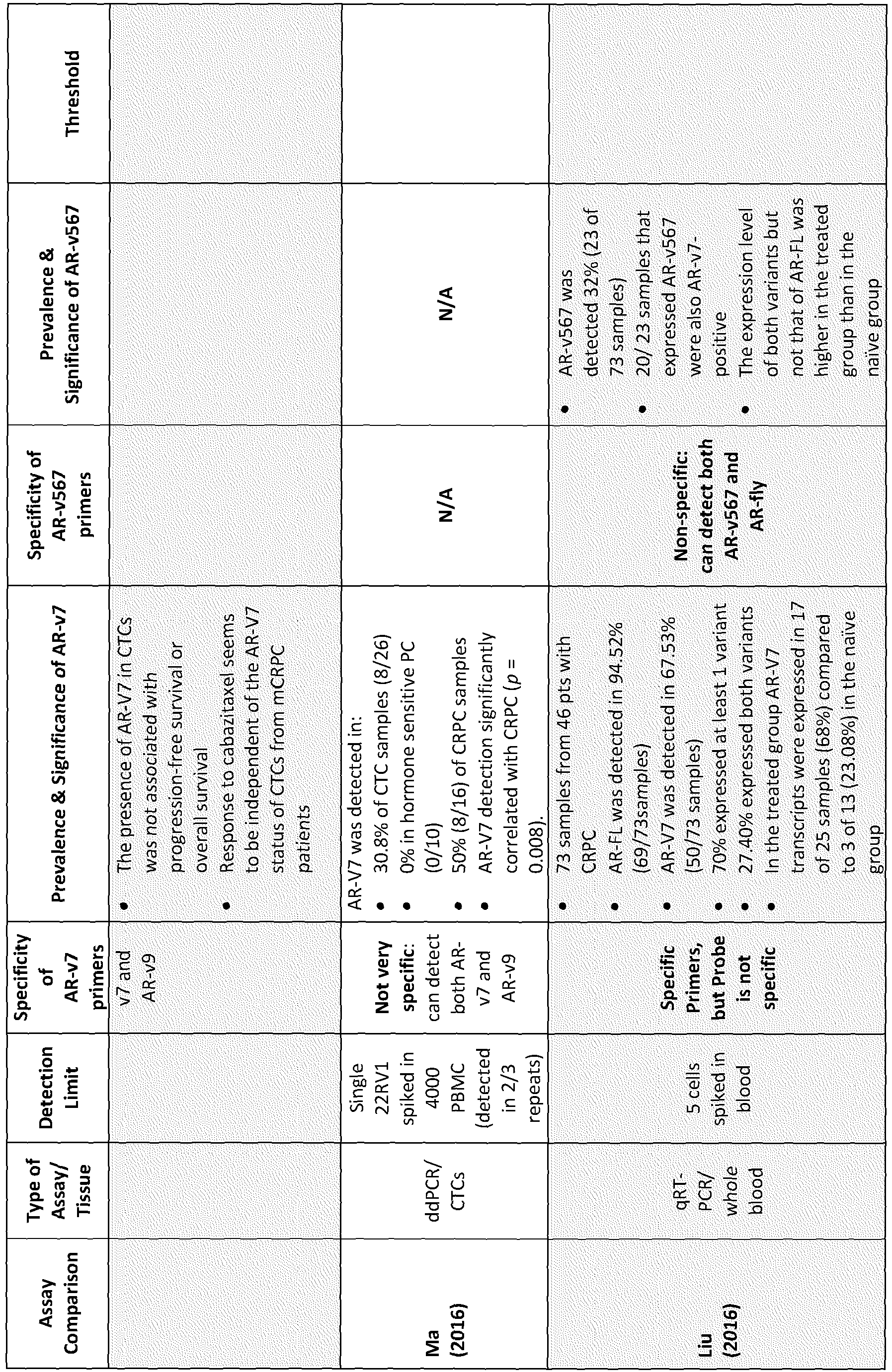

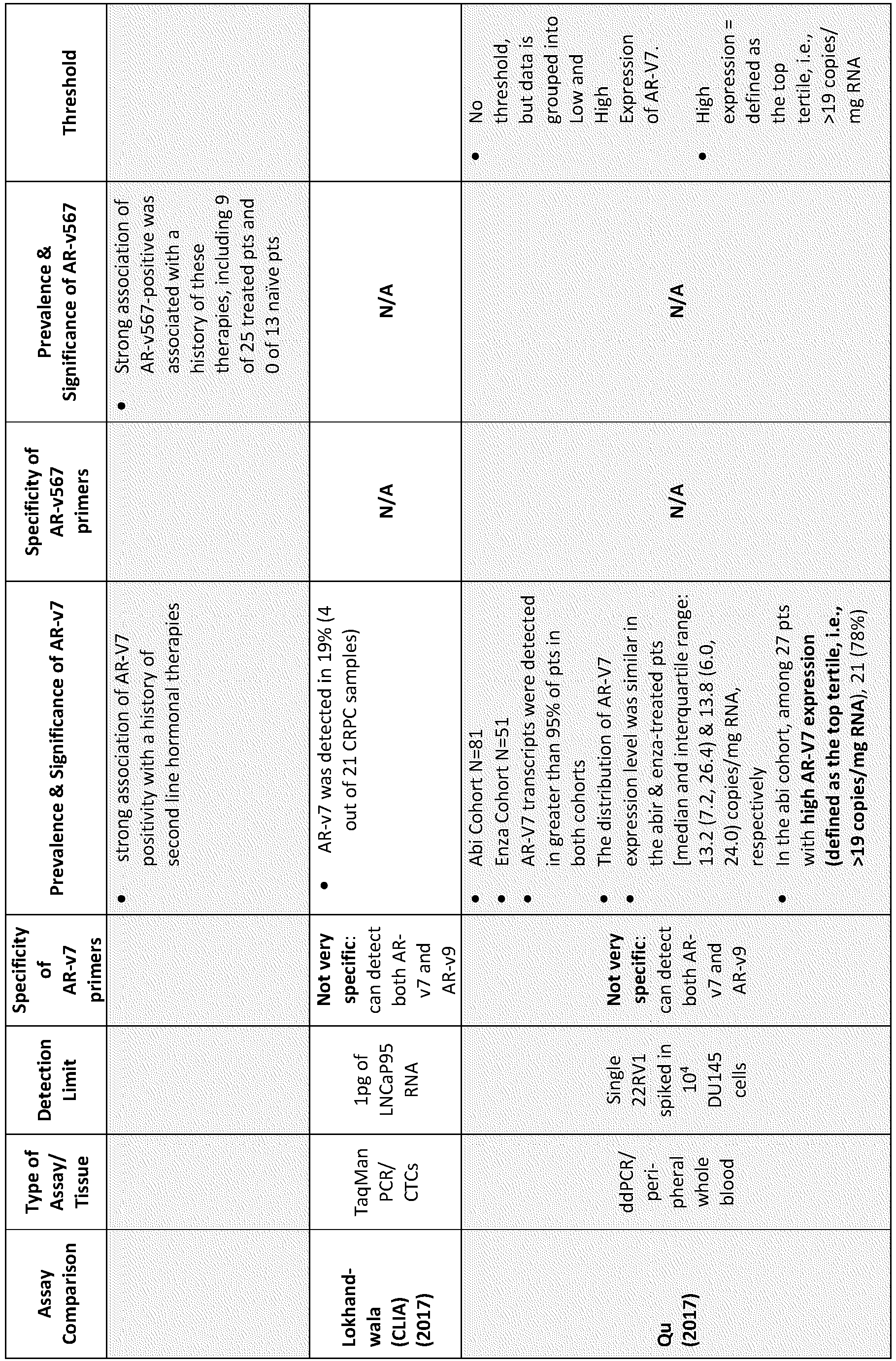

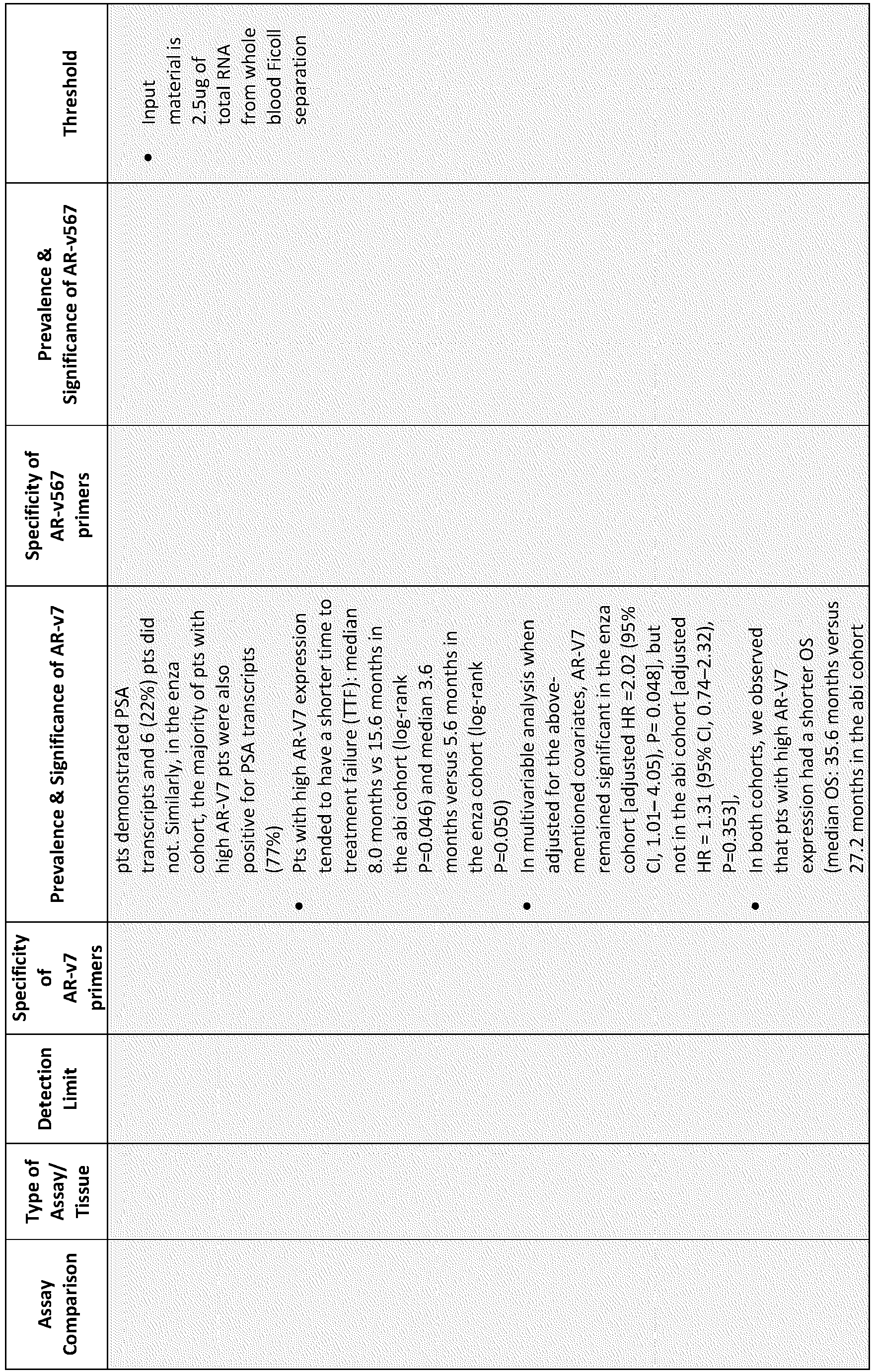

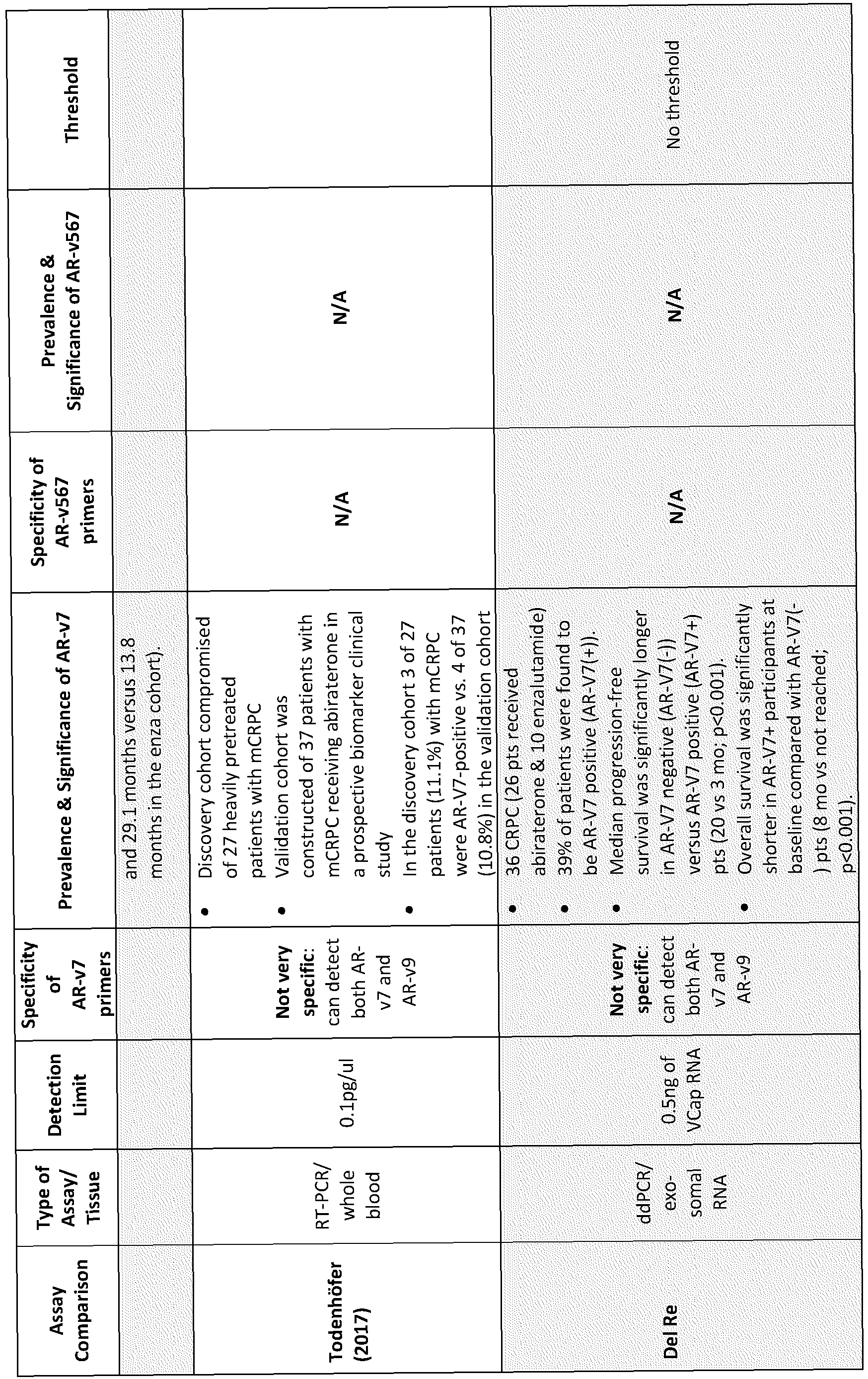

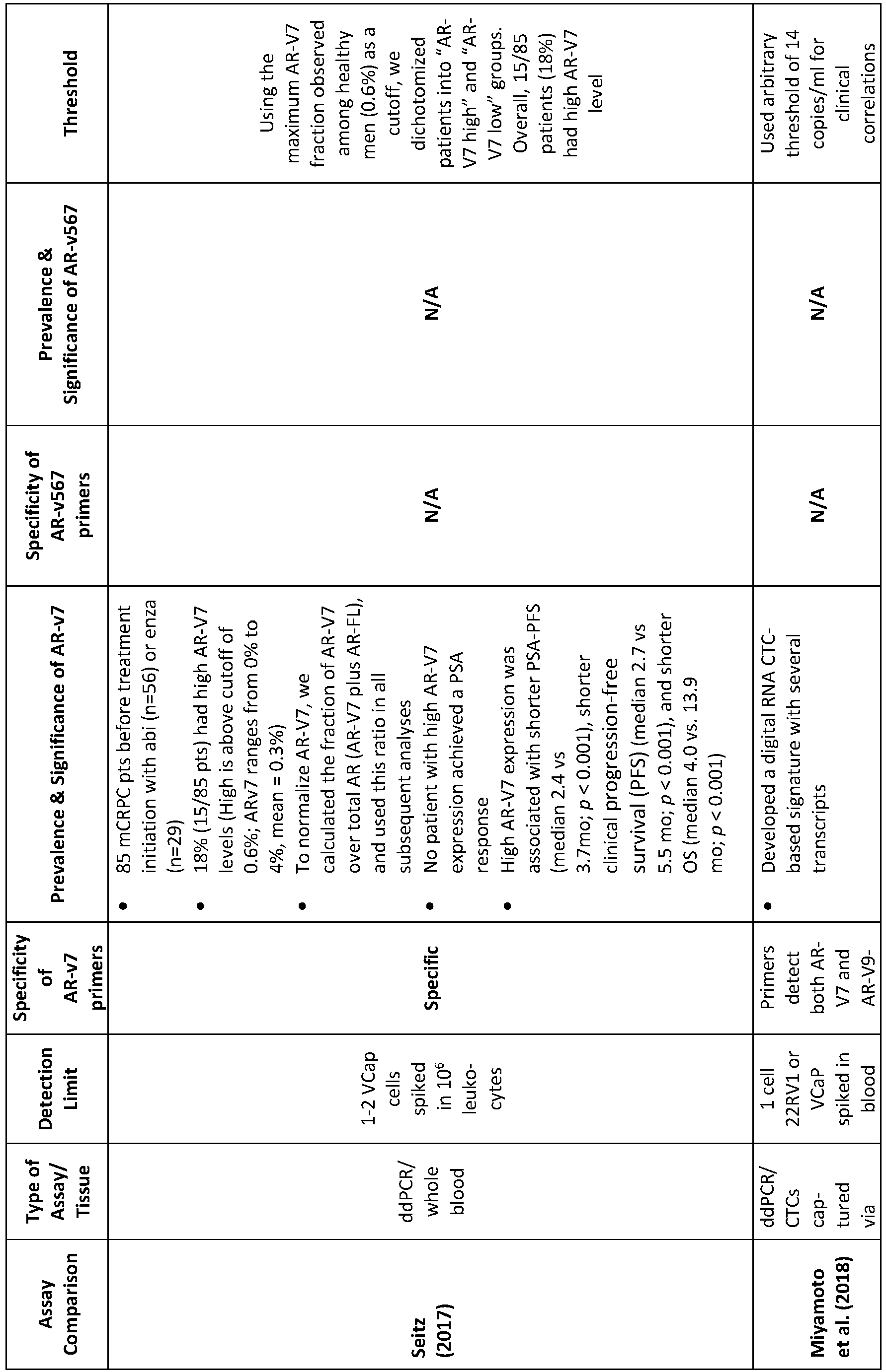

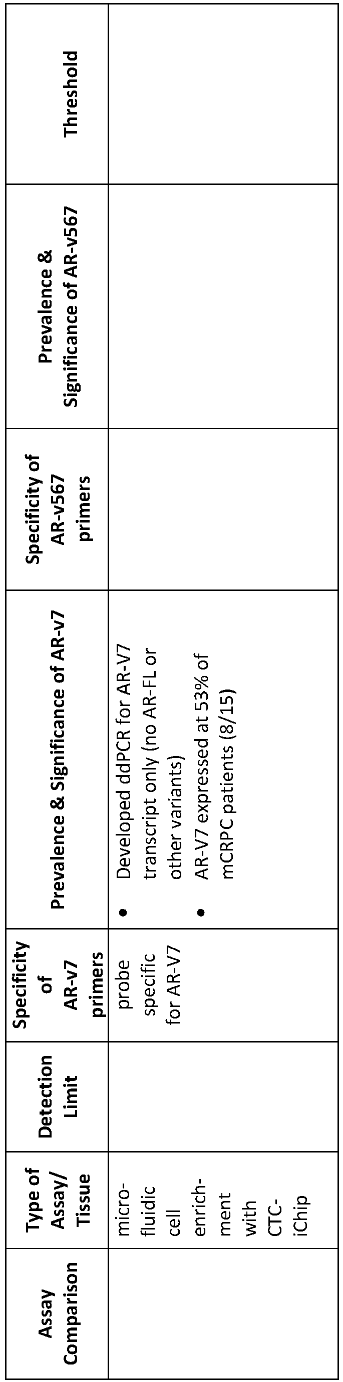

The methods and kits described herein can be used for any of the following:
- Use for detection of AR-FL, AR-V7 and AR-V567 from miniscule amounts of human samples (CTCs, tumor biopsies, organoids, rare single cells etc.);
- Use in the clinic to assist the optimal clinical disease management of diseases where expression of these AR variants is important (e.g. prostate cancer);
- Use for the real time and longitudinal monitoring of AR-V expression and correlating responses to AR-targeted therapies, taxane chemotherapy, or other AR-V targeted therapies;
- Use as a companion diagnostic tool for the clinical development of any new drugs/antibodies that involve modulation of the AR signaling axis and AR variants;
- Use for the selection of cohorts of patients with different expression levels or positive/negative for AR-Vs and for AR-V targeted therapies development.

The assay methods described herein have been used in two prospective multi-institutional clinical trials.

### Kits

Kits are also disclosed herein that are useful for detecting and/or quantifying full-length and/or variant androgen receptors.

For example, such a kit, disclosed herein (but not claimed), can include at least one primer or probe specific for one or more androgen receptor in a biological sample. One example of such a kit, disclosed herein (but not claimed), can include: at least one set of primers comprising a forward primer and a reverse primer, wherein each forward primer and reverse primer includes an oligonucleotide of between 10 and 30 nucleotides in length and of at least 10 contiguous nucleotides of a nucleotide sequence located in
Set 1: SEQ ID NO: 7 and 8 (for detecting / quantifying AR-FL);
Set 2: SEQ ID NO: 11 and 12 (for detecting / quantifying AR-V7);
Set 3: SEQ ID NO: 15 and 16 (for detecting / quantifying AR-v567es); or combinations of such sets of primers.

The kits can also include probes that can include one or more of the following sequences:
SEQ ID NO:9 (for detecting / quantifying AR-FL);
SEQ ID NO:13 (for detecting / quantifying AR-V7);
SEQ ID NO:17 (for detecting / quantifying AR-v567es); or
combinations thereof.

The kits can include combinations of primer sets and/or probes in a single composition or container. Alternatively, the kits can include separate primer sets and/or probes in separate compositions or containers.

The primer sets and/or probes can be covalently attached to a solid surface or be aliquoted into wells arrayed in a solid substrate. For example, the primer sets and/or probes can be distributed on or within a microarray.

The kits can also include nucleotides, enzymes, cofactors, salts, buffers, and combinations thereof that are useful for performing methods for detecting and/or quantifying the full-length androgen receptor and/or the androgen receptor variants.

### Treatment

Patients can be informed of the assay results. As used herein "informing the patient" or "informing the test subject" can involve reporting test results to a hospital, medical clinic, or doctor who may provide medical care for the patient or test subject. As used herein the terms "patient" and "test subject" are used interchangeably.

For example, patients can be informed of the quantity of full-length androgen receptor (AR-FL), androgen receptor variant 7 (AR-V7), and/or androgen receptor variant 5,6,7 (AR-V567) transcripts.

A subject or patient can have cancer (e.g., prostate cancer) and/or may be resistant to currently administered therapeutics (drug-resistant), when the quantities of full-length androgen receptor (AR-FL), androgen receptor variant 7 (AR-V7), and/or androgen receptor variant 5,6,7 (AR-V567) transcripts are different from control quantities of full-length androgen receptor (AR-FL), androgen receptor variant 7 (AR-V7), and/or androgen receptor variant 5,6,7 (AR-V567) transcripts. Controls can include the amounts of full-length androgen receptor (AR-FL), androgen receptor variant 7 (AR-V7), and/or androgen receptor variant 5,6,7 (AR-V567) transcripts detected or quantified in healthy subjects, or subjects without cancer.

The methods provided herein for detecting androgen receptor variants (and full-length androgen receptors) can be combined with providing for treatment of diseases associated with expression of such androgen receptor variants (and full-length androgen receptors). For example, in some cases detection of androgen receptor variant (and/or full-length androgen receptor) expression is an indicator of drug resistance. Patients or test subjects that exhibit drug resistance (e.g., as detected by the assay methods described herein) can benefit from the provision of different treatment regimens and/or providing for administration alternative drugs or therapeutic agents.

Patients or test subjects who can be provided for treatment include cancer patients, for example, patients with prostate cancer or drug-resistant prostate cancer.

When drug-resistant androgen (variant and/or full-length) receptor expression is detected, a patient can be provided for treatment with a variety of other therapeutic agents or therapeutic procedures that may not include use of the drug to which the patient is resistant. A drug-resistant cancer can be provided for treatment with a variety of other therapies useful in the treatment of drug-resistant cancer. For example, elevated prohibitin levels have been shown to play a role in taxane-resistant cancers, and inhibition of prohibitin can reduce taxane-resistance (see e.g., US2009/0312405). Thus, any method for inhibiting prohibitin (e.g., US2009/0312405) can be used in the treatment of a taxane-resistant cancer. Exemplary inhibitors of prohibitin are known in the art and are described e.g., in US2009/0312405.

Other agents that prevent or reverse drug-resistance can include, but are not limited to, an inhibitor of glutathione-S-transferase π, or an inhibitor of p-glycoprotein.

Non-limiting examples of other chemotherapeutic agents that can be provided for administration to patients or test subjects include alkylating agents such as thiotepa and CYTOXAN^{®} cyclophosphamide; alkyl sulfonates such as busulfan, improsulfan and piposulfan; aziridines such as benzodopa, carboquone, meturedopa, or uredopa; ethylenimines and methylamelamines including altretamine, triethylenemelamine, trietylenephosphoramide, triethiylenethiophosphoramide or trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gamma1I and calicheamicin omega1I (see, e.g., Agnew, Chem. Intl. Ed. Engl., 33: 183-186 (1994)); dynemicin, including dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; chloranbucil; GEMZAR^{®} gemcitabine; 6-thioguanine; mercaptopurine; platinum analogs such as cisplatin, oxaliplatin and carboplatin; vinblastine; platinum; etoposide (VP-16); ifosfamide; mitoxantrone; vincristine; NAVELBINEO. vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluoromethylornithine (DMFO); retinoids such as retinoic acid; capecitabine; combretastatin; leucovorin (LV); oxaliplatin, including the oxaliplatin treatment regimen (FOLFOX); lapatinib (Tykerb^{®}); inhibitors of PKC-alpha, Raf, H-Ras, EGFR (e.g., erlotinib (Tarceva@)) and VEGF-A that reduce cell proliferation and pharmaceutically acceptable salts, acids or derivatives of any of the above.

In addition, the methods of treatment disclosed herein can further include the use of radiation or radiation therapy. Further, the methods of treatment disclosed herein can further include the use of surgical treatments.

As described herein the provision of treatment can be varied depending on which types of AR variants are expressed in a patient. Taxanes are currently the only class of chemotherapy agents that extend survival in men with advanced prostate cancer. These drugs bind β-tubulin and stabilize cellular microtubules, leading to inhibition of microtubule-dependent intracellular trafficking and signaling, mitotic arrest, and apoptotic cell death. Although taxanes are generally considered antimitotic agents, they also inhibit tumor growth via several different mechanisms. Prostate cancer cells rely heavily on sustained androgen receptor (AR) nuclear signaling, which drives progression despite androgen-deprivation therapy. AR binds to cellular microtubules via the microtubule-associated motor protein dynein to facilitate its nuclear translocation. Taxanes can inhibit AR nuclear trafficking via stabilization of microtubules. Taxane-induced microtubule stabilization (termed drug-target engagement [DTE]) results in microtubule bundling (MTB), cytoplasmic sequestration of AR, inhibition of AR transcriptional activity, and inhibition of prostate cancer cell growth. Hence, in some cases taxane treatment can be helpful for treatment of prostate cancer.

Taxane treatment inhibits microtubule dynamics, it has been shown to differentially affect the nuclear localization of AR splice variants. The data shown herein demonstrates that AR-V7-negative patients have a greater reduction in AR percent nuclear localization when treated with taxanes compared with AR-V7-positive 315 patients (Table 9 and FIG. 10). AR-FL is kept inactive in the cytoplasm, whereas the nuclear localization of AR-V7 is not affected. Accordingly, tumors that express AR-V7 are less sensitive to taxanes and taxanes may not be successfully administered to patients exhibiting AR-v7. The hinge domain that is retained in ARv567es is the minimum functional domain required for microtubule binding, although it does not bind as extensively as the entire C-terminus of AR. Hence, taxane treatment can partially impair the nuclear localization of ARv567es *in vitro* and detecting ARv567es expression in patients can confer taxane sensitivity *in vivo.*

In 9% of patients with undetectable levels of either splice variant that were evaluated as described herein, but that also exhibited AR-FL expression, exceptionally high response rates to taxane treatment of 80-100% were observed.

Hence, when patient samples are evaluated as described herein, expression of AR-FL in such samples indicates that a patient can successfully be provided for treatment with taxanes. Patient with samples exhibiting expression of ARv567es can in some cases be successfully provided for treatment with taxanes, but in other cases may not be successfully provided for treatment with taxanes. However, when significant quantities of AR-7 are detected in patient samples, those patients should be provided for receipt of treatment other than taxanes because taxane treatment may not be effective.

The following Examples illustrate procedures and results used and obtained in the development of the invention.

### Example 1: Materials and Methods

This Example illustrates some of the materials and methods used in the development and methods described herein.

### Cell culture

22Rv1 (Cat # CRL-2505) and VCaP (Cat # CRL-2876) cells were obtained from ATCC. ATCC authenticates human cancer cell lines using short tandem repeat analysis. These cell lines were expanded, and earlier passages were frozen in liquid nitrogen. 22Rv1 were maintained in RMPI1640 (Corning) with 10% FBS, 100 U/mL penicillin, and 100µg/mL streptomycin (penicillin/streptomycin) in a 5% CO₂ incubator at 37°C. VCaP cells were cultured in DMEM (Corning) with 10% FBS and penicillin/streptomycin in a 5% CO₂ incubator at 37°C.

### Plasmid transfections

pEGFP-C1*-AR-FL,* pEGFP-C1-*AR*-*V7* and pEGFP-C2-*AR-567* plasmid DNA were used as positive controls for the development of the multiplex ddPCR assay. 6 ng of each plasmid was transfected in AR null HEK293T cells using FuGENE^{®} 6 Transfection Reagent according to the manufacturer's instructions (Roche, Germany). Post 24h transfection total RNA was isolated using *RNeasy Mini Kit* (Qiagen, Germany, Cat. # 74104), cDNA was generated from 1 ug of total RNA using *ProtoScript First Strand cDNA Synthesis* (NEB, Cat. # E6300L) and cDNA samples were used for evaluating the specificity of the methodology.

### Patient sample processing and RNA extraction

In this study, peripheral blood samples were collected from mCRPC patients in EDTA tubes and processed within 24 hours of the time of blood withdraw. All patients provided written informed consent. CTCs from the whole blood of mCRPC patients were enriched and isolated via two methods:
1. using the Rosette Sep negative depletion kit according to the manufacturer's instructions *(STEMCELL Technologies Inc., Canada);* and
2. using the prostate-specific membrane antigen (PSMA)-based geometrically enhanced immunocapture (GEDI) as described by Kirby et al. (PLoS One. 7(4):e35976 (2012) and/or Galletti et al. (LabChip 14(1): 147-156 (2014)).

Total RNA was extracted from the enriched CTCs pool using the *RNAeasy Plus Micro kit* (Qiagen) as per manufacturer's instructions. And then arrayed in 96-well format for PCR using commercially available multiplexed master mixes of PCR enzyme/buffer from the *One-Step RT ddPCR Advanced Kit for Probes* (Bio-Rad), amplicons for a GUSB control DNA region, and primers that specifically detect the AR- FL and AR-variants.

### Healthy Donors

We included 10 healthy male subjects to determine background levels of AR- FL, AR-V7 and AR-v567es transcripts in peripheral whole blood and whole blood processed in a similar manner as for CTC enrichment from the blood of prostate cancer patients. Samples from healthy subjects were obtained and stored under the same conditions as for patient samples to minimize any bias.

### Primers and Probes

Each primer set was designed to recognize unique and distinguished regions of each of the variants using Primer3Plus based on the direction of the ddPCR Application Guide Bulletin 6407 (Bio-Rad). AR-FL assay recognizes unique junction between exon 7 and 8, AR-v7 assay recognizes the junction of exon 3 and cryptic exon 3, and AR-v567es assay recognizes the junction between exon 4 and exon 8. The primers were designed such at least one primer from a pair spans on the exon-exon junction to avoid unspecific amplification from other variants and synthesizes from genomic DNA. Specificity of the primer design was assessed by a Nucleotide BLAST (blastn) search against the up-to-date version the human genome reference (hg38) database and IGV (Integrative Genomics Viewer). The primers and probes were designed and purchased from Bio-Rad for use in all droplet digital PCR (ddPCR) reactions as shown in Table 2.

**Table 2: Primers and Probes**

| **Transcript** | **Type** | **Sequence** | **SEQ ID NO:** |
|---|---|---|---|
| AR-FL | Forward | 5'-AATCCCACATCCTGCTCAAG-3' | 7 |
| | Reverse | 5'-GCAGCCTATTGCGAGAGAG-3' | 8 |
| | Probe | 5'-ACCAGCTCACCAAGCTCCTGG-3' | 9 |
| | Fluorophore | FAM | 10 |
| AR-V7 | Forward | 5'-AGGGATGACTCTGGGAGAAA-3' | 11 |
| | Reverse | 5'-AAAGGCTGACTTGCCTCATT-3' | 12 |
| | Probe | 5'-TCCGGGTTGGCAATTGCAAGC-3' | 13 |
| | Fluorophore | FAM | 14 |
| AR-v567es | Forward | 5'-CTTTGCAGCCTTGCTCTCTA-3' | 15 |
| | Reverse | 5'-CTTGCCTGATTGCGAGAGAG-3' | 16 |
| | Probe | 5'-ACACGTGGTCAAGTGGGCCA-3' | 17 |
| | Fluorophore | FAM | |

### Digital droplet PCR (ddPCR) platform and reactions

Droplet Digital PCR (ddPCR) is a digital PCR method based on the water-oil emulsion droplet technology (Vogelstein B, Kinzler KW. Digital PCR. Proc Natl Acad Sci U S A 96(16):9236-41 (1999); Pinheiro LB, Coleman VA, Hindson CM, Herrmann J, Hindson BJ, Bhat S, et al. Evaluation of a droplet digital polymerase chain reaction format for DNA copy number quantification, Anal Chem 84(2):1003-11 (2012)). The Droplet Digital PCR System partitions nucleic acid samples into 20,000 nanoliter-sized droplets and PCR amplification is carried out within each droplet.

AR-FL, AR-V7 and AR-v567es transcript quantifications were carried out on a QX200 Droplet Digital PCR (ddPCR) system with automated droplet generation (Bio-Rad Laboratories). Reactions were carried out in ddPCR Plates 96-Well, Semi-Skirted (twin.tec PCR, Eppendorf). Each well contained 5.5µl of ddPCR Supermix for Probes, 2.2ul Reverse Transcriptase and 1.1ul of 300mM DTT and other components of a One-Step RT-ddPCR Advanced Kit for Probes (Cat. # 186-4022, from Bio-Rad), 1.1 µl of target-specific primers, and 11 µl of sample RNA, for a total volume of 22 µl. Plates were sealed, spun down and loaded into a QX200 automated droplet generator (Bio-Rad). Immediately after droplet generation, 96-well plates containing droplet-partitioned samples were heat-sealed and PCR was carried out on a C1000 Touch Thermal Cycler (Bio-Rad) using the following cycling protocol: reverse transcription at 42 °C for 60min, enzyme activation at 95 °C for 10 minutes followed by 40 cycles of 95 °C for 30 seconds (for denaturation) and 60 °C for 60 seconds (for annealing/extension), followed by a final 10-minute incubation at 98 °C for enzyme deactivation. Ramp rate was 2 °C per second. Plates were then kept at 4 °C for at least 4 hours prior to being analyzed in the BioRad QX200 droplet reader because this has been shown to improve acceptable droplet counts. Included on each plate were positive and negative "plate controls": no template control (NTC), HEK 293 cells transfected with either AR-FL, AR-V7 or AR-v567es. Purified nuclease-free water was used as negative, no-template control (NTC). Plates were sealed on the robotic platform using a plate sealing stage. All robotic procedures were performed in a pre-PCR clean area.

Data acquisition and analysis was performed with the system's pre-installed software QuantaSoft from Bio-Rad. The fluorescence amplitude threshold, distinguishing the positive from the negative droplets was set manually by the technician in between the fluorescence amplitude of the positive and negative droplets taking into consideration all the positive and negative controls within each plate.

### ddPCR Quality Controls

Each plate and each well contained controls to ensure assay validity and performance. Wells or plates that did not meet the expected criteria were flagged and reran. Plates were flagged for evaluation and/or reran if any of the following conditions was not met: 1) positive droplets in NTC; 2) positive droplets in the no-mastermix control; and 3) an average of less than 15,000 droplets/well across the plate.

### Example 2: the ddPCR Assay and Data Analysis

AR-FL and AR-Vs expression was detected and/or quantified using ddPCR. The ddPCR method can reliably measure, with high precision, extremely low concentrations of specific DNA sequences even when a complex mixture of templates is present. Moreover, the ddPCR method does not require development of or use of a standard curve. Digital PCR is a method of absolute nucleic acid quantification based on the partitioning a qPCR reaction sample into tens of thousands of nano-reactions (droplets) of defined volume. Each droplet either contains or does not contain a template molecule (Vogelstein, 1999; Pinheiro, 2012; Hindson, Anal Chem. 2011 Nov 15;83(22):8604-10 (2011)). After PCR, droplets that contained a template will have a fluorescent signal (positive droplets) that distinguishes them from the droplets without a template (negative droplets). It is the ratio of detected "positive" droplets to total droplets that allows the number of target molecules per droplet to be calculated from a Poisson distribution.

### Example 3: Assay Specificity and Sensitivity

The analytical specificity of the ddPCR assay was determined by transfecting HEK293T cells with plasmids encoding AR-FL, AR-v7 or AR-v567es. The HEK293T cells express very low endogenous levels of AR-FL and are negative for AR-v7 and AR-v567 transcripts. Each primer pair and probe was designed to be at the position of exon junctions to ensure specificity for each respective transcript and avoid non-specific signals from other variants. See, e.g., FIG. 1A.

FIG. 1B shows signal detection expressed as copies/sample, specific for each transcript, while the water control was completely negative for all three transcripts. No variant signal was detected in the non-transfected HEK293T cells, while low levels of endogenous AR-FL was present, as expected. In addition, when we performed this assay using genomic DNA as input, no signal was detected, confirming the specificity of the assay.

FIG. 1C illustrates the analytical sensitivity of the assay as determined for each individual AR splice variant using a calibration curve that was generated using serial dilutions of the respective DNA plasmids (1, 0.1, 0.01 ng) in triplicate for each concentration. These results showed linearity over the entire quantification range and correlation coefficients greater than 0.99 in all cases, indicating a precise log-linear relationship. FIG. 1D shows that the assay does not detect genomic DNA.

The sensitivity of the ddPCR assay was also determined by spiking 1, 5, 25 or 50 VCAP or 22RV prostate cancer cells into 1 million PBMCs isolated via Ficoll gradient separation from the peripheral blood of a male healthy donor. Following RNA extraction, the total RNA was split into three different reactions for the detection of each the AR variants. As shown in FIG. 2A, the AR-FL and AR-v7 mRNA transcripts were detected in the VCaP spiked cells at levels as low as the quantity of RNA in a single cell. As also illustrated in FIG. 2A, the AR-v567es transcript was not detected in the VCaP cells.

The limit of detection of these androgen receptors was also evaluated by isolating single cells using the Cell Celector system from ALS (FIG. 2B). AR-FL and AR-v7 mRNA transcripts were detected in RNA obtained from just half a VCaP cell.

As shown in FIG. 2C, the expression levels of the AR-FL and AR-v7 were varied among the different single VCaP and 22RV cells tested, with some cells expressing higher levels of AR-FL than AR-v7 and *vice versa* emphasizing that the population of CTCs is heterogeneous.

### Example 4: Assay Reproducibility

Intra-assay variance (repeatability) of the ddPCR assay was evaluated by repeatedly analyzing 10ng of RNA from transfected AR-FL, ARv7 and AR-v567es DNA plasmids in HEK293T cell line and a non-transfected HEK293T null cell line in 5 parallel experimental set-ups.

Each experimental replicate was carried out under the following repeatability conditions. The same RNA batch from the transfected plasmids expressing the AR-FL, AR-v7 and AR-v567es, the same pre-mix from the One Step kit, cartridges from the same batch, the same instrument, but randomly positioned on the 96-well PCR plate. Intra-assay variance was expressed as the standard deviation (SD) of the Copies/µl. The intra-assay variance in Copies/µl for AR-FL, ranged from 0.05 to 1.2. The intra-assay variance in Copies/µl for AR-v7 ranged from 0 to 0.45. The intra-assay variance in Copies/µl for AR-v567es ranged from 0 to 0.09. See Table 3.

**Table 3: Intra-assay precision (n=5)**

| **Assay 1: AR-FL** | | | |
|---|---|---|---|
| ***ARFL*** | **Copies (SD)** | | **CV%** |
| HEK293-NT | 13.8 (±1.1) | | 8.4 |
| HEK293-FL | 1.5 (±0.05) x 10² | | 3.4 |
| HEK293-V7 | 12.2 (±1.1) | | 9.1 |
| HEK293-v567es | 13.8 (±1.2) | | 8.4 |

| **Assay 2:AR-V7** | | | |
|---|---|---|---|
| ***AR-V7*** | **Copies (SD)** | | **CV%** |
| HEK293-NT | 0 (0) | | 0 |
| HEK293-FL | 0 (0) | | 0 |
| HEK293-V7 | 9.1 (±0.45) x 10 | | 5.0 |
| HEK293-v567es | 0 (0) | | 0 |

| **Assay 3: AR-v567es** | | | |
|---|---|---|---|
| ***AR-567*** | **Copies (SD)** | | **CV%** |
| HEK293-NT | 0 (0) | | 0 |
| HEK293-FL | 0 (0) | | 0 |
| HEK293-V7 | 0 (0) | | 0 |
| HEK293-v567es | 1.67 (0.09) x 10² | | 5.4 |

As shown the Intra-assay variance expressed as within-run coefficient of variation (CV) of copies/µL ranged for AR-FL, from 3.4% to 9.1%, for AR-v7 from 0% to 5%, and for AR-v567es from 0% to 5.4%.

The Inter-assay variance (reproducibility) was also evaluated by analyzing the same DNA plasmids of AR-FL, AR-v7, and AR-v567es transfected in the HEK293 cell line on 5 separate assays performed on 5 different days. The results were expressed as between-run standard deviations (SDs). As shown in the chart below, between-run SDs of the copies variance for AR-FL ranged from 0.13 to 0.9, for AR-v7 ranged from 0 to 0.2, for AR-v567es ranged from 0 to 0.1, while CVs were for AR-FL from 6.1 to 7.4%, for AR-v7 from 0 to 2.9%, for AR-v567es from 0 to 7.1%. See Table 4.

**Table 4: Inter-assay precision (n=5)**

| ***Assay 1: AR-FL*** | | | |
|---|---|---|---|
| ***ARFL*** | **Copies (SD)** | | **CV %** |
| HEK293-NT | 12.9 (±0.79) | | 6.1 |
| HEK 293-FL | 1.82 (±0.13) x10² | | 7.2 |
| HEK293-V7 | 12.6 (±0.9) | | 7.4 |
| HEK2 93-v567es | 11.9 (±0.8) | | 6.7 |

| ***Assay 2: AR-V7*** | | | |
|---|---|---|---|
| **AR-V7** | **Copies (SD)** | | **CV%** |
| HEK293-NT | 0 (0) | | 0 |
| HEK293-FL | 0 (0) | | 0 |
| HEK293-V7 | 9.6 (±0.2) x 10 | | 2.9 |
| HEK293-v567es | 0 (0) | | 0 |

| ***Assay 3: AR-v567es*** | | | |
|---|---|---|---|
| ***AR-567*** | | **Copies (SD)** | **CV%** |
| HEK293-NT | | 0 (0) | 0 |
| HEK293-FL | | 0 (0) | 0 |
| HEK293-V7 | | 0 (0) | 0 |
| HEK293-v567es | | 1.54 (0.1) x10² | 7.1 |

### Example 5: Assay Validation

This Example illustrates validation of the assay procedures as described in the foregoing Examples within healthy volunteers and metastatic castration-resistant prostate cancer (mCRPC) patients.

### AR-V expression in Healthy Volunteers

The specificity of the assay was tested in using peripheral blood mononuclear cell (PBMC) fractions of peripheral blood samples from healthy male donors. Using the Ficoll-hypaque density gradient separation method PBMCs were isolated from 10 healthy male individuals.

As shown in FIG. 3A, all the healthy donor control samples were negative for both AR-variants, but expression of the reference gene GUSB was detected. Only very low levels of the AR-FL were detected in normal, healthy persons.

### AR-V expression in captured CTCs and PBMCs of mCRPC patients

The performance of the ddPCR assay was evaluated in circulating tumor cells (CTCs) and PBMCs isolated from the peripheral blood of six mCRPC samples. CTCs were isolated from 7- 15ml of peripheral blood through use of the antigen-agnostic CD45 negative depletion Rosette Sep kit. In parallel, 1-2mls of matching blood from the six mCRPC patient was processed using the Ficoll gradient centrifugation for the isolation of the PBMC fraction.

As illustrated in FIG. 3B, heterogeneous expression of AR-FL, AR-v7 and AR-v567 transcripts was detected in the CTCs from these patient samples. The PBMC fraction from the same patient samples expressed very low levels of AR-FL, while the AR-V7 and AR-v567es were not detectable in PBMC samples (FIG. 3B).

### Example 6: Assay Reproducibility in Patient Samples

The reproducibility of the ddPCR assay in CTCs from mCRPC patient samples was evaluated.

Nearly identical results were obtained for the expression of each AR transcript when the CTCs from the same patient sample was split and run twice. CTCs were isolated as described in the foregoing Examples from 7-15ml of peripheral blood by the CD45 negative depletion method. The RNA was extracted and was split into two fractions. The libraries and the ddPCR experimental setup for each of the two runs were processed by two different operators.

FIG. 4 shows the expression levels of the AR-FL, AR-v7 and AR-v567es RNAs in each given sample, showing that the expression levels were similar between the two runs.

### Example 7: AR-V expression in captured CTCs from mCRPC patients

The absolute copy number of each of the AR-variants was quantified in each of the patient samples and used to determine expression pattern and prevalence of each of these variants in CTCs using the ddPCR assay described herein.

As shown in FIG. 5, AR-FL was the predominantly expressed transcript with a median and mean respectively of [x;y copies/ul] and range of [x;y] while AR-V7 was expressed at a median and mean of [x,y] and range of [x;y] and AR-v567es has a median and mean of [x,y] and range of [x;y].

Table 5 below shows that of the 38 mCRPC samples that were analyzed, 28/38 (74%) were AR-FL positive, 26/38 (69%) were ARV7-positive, 11/38 (29%) were AR-v567es-positive, 9/38 (24%) had both variants, and 7 of 38 (18%) expressed all three AR-FL, AR-V7 and AR-v567es.

**Table 5: AR Types Expressed in mCRPC Samples**

| **AR-FL-positive** | **AR-V7-positive** | **AR-v567-positive** | **AR-V7-positive, AR-v567-positive** | **AR-V7-negative, AR-v567-negative** |
|---|---|---|---|---|
| 28/38 | 26/38 | 11/38 | 9/38 | 7/38 |
| 74% | 69% | 29% | 24% | 18% |

### Example 8: AR-V expression is enriched in Transferrin receptor 1 (TfR) positive CTCs versus EpCAM-positive CTCs from mCRPC patients.

The epithelial cell adhesion molecule (EpCAM) is the most widely used antigen for the positive identification of CTCs in solid tumors, including in prostate cancer cases. However, as EpCAM is downregulated during epithelial to mesenchymal transition (EMT), which is a process that precedes metastasis, its validity in the molecular characterization of CTC from metastatic patients is questionable.

Transferrin receptor (TfR) is highly expressed in prostate cancer and overexpressed in metastatic CRPC patients. As TfR appears to not be affected by EMT the inventors tested TfR as an alternative positive selection antigen for mCRPC CTCs.

TfR protein expression was first analyzed by immunofluorescence in a panel of prostate cancer cell lines and in healthy donor leukocytes using methods described by Galletti et al. (AACR Cancer Research 77(13 Supplement): 1713-1713 (July 2017)). While all prostate cancer cells lines analyzed were positive for TfR expression, none of the leukocytes expressed the receptor. Moreover, TfR expression was detected also in EpCAM negative prostate cancer cell lines.

To determine the clinical applicability of this novel CTC identifier, TfR expression was determined in CTCs isolated from peripheral blood of 16 metastatic CRPC patients using the Cell Celector technology. Two pools of TFR+ and EpCAM+ CTCs were also subjected to the ddPCR assay.

The results are shown in Table 6, and in FIG. 6.

**Table 6: AR Types Expressed in CTCs Isolated from CRPC Patients Using TFR+ or EpCAM+**

| **AR-FL** | | **AR-V7** | | **AR-v567es** | |
|---|---|---|---|---|---|
| **TFR+** | **EpCAM+** | **TFR+** | **EpCAM+** | **TFR+** | **EpCAM+** |
| 12/16 | 11/16 | 9/16 | 6/16 | 6/16 | 3/16 |
| 75% | 69% | 56% | 38% | 38% | 19% |

As illustrated in Table 6 and in FIG. 6, higher enrichment for ARv567es and ARV7 expressing cells was observed in the TfR+ CTCs. No significant difference was observed in the detection of AR-FL transcripts within TFR-CTC positive cells (75% express AR-FL) and the EpCAM-CTC positive cells (69% express AR-FL).

However, there was a significant difference in the detection of both AR-V7 and Ar-v567es within TFR-CTCs and EpCAM-CTCs. As shown, 56% of TFR-CTCs express AR-V7 while only 38% of EpCAM-CTCs express AR-V7. The AR-v567es variant is expressed in 38% of TFR-CTCs, while only 19% of EpCAM-CTCs express AR-v567es.

These data indicate that TfR is a promising biomarker for the detection of CTCs in mCRPC patients, and potentially superior to EpCAM. Hence, rather than relying solely on EpCAM+ enrichment, TfR can be useful for CTC enrichment from prostate cancer patients.

In addition to prostate cancer patients, the applicants have successfully used TfR to identify CTCs from the peripheral blood of non-small cell lung cancer (NSCLC) patients. Currently, there is no existing assay able to reliably and consistently identify CTCs in NSCLC patients, including the FDA-cleared CellSearch (slide 1). The applicants have used TfR labeling and have identified CTCs from both early-stage (stage I-IIIA) and metastatic (stage IV) NSCLC patients. The TfR+-cells were deemed to be CTCs based on co-expression of pan-cytokeratin (epithelial cell marker) and TTF1 (established marker used clinically by pathologists to identify adenocarcinoma of the lung) (slide 4).

The assay methods described herein have been used in two prospective multi-institutional clinical trials.

Applicants believe that the methods described herein describe the first specific dd-PCR assay that reliably and concomitantly detects the expression of both AR-V7 and AR-v567 variants as well AR-FL expression in CTCs from mCRPC patients.

The assay specificity relates to the design of primers that lie on unique exon-exon spanning regions of each variant that avoid interference from other variants. Such interference can be present in currently available assay methods due to sequence similarities, such as that of AR-v9 with other variants.

There have four recent publications on the dd-PCR assay for the detection of the AR-v7, and only one of which has any specificity for the detection of the AR-v7 from the whole blood of prostate patient samples, while the other three are non-specific due to the lack of specificity in the primer design and because those assays detect both the AR-v7 and the AR-v9 variants. The inability to reliably distinguish AR-v7 and AR-v9 variant signals by such currently available assay methods may have led to misleading and inaccurate quantification of these variants, with an inappropriate correlation to patient outcome.

There has been only report (Hornberg 2011) on the quantification of both AR-v567 and AR-v7 from prostate patient tissues by qRT-PCR, in which the AR-v567 primers were deemed to be specific, but the role of AR-v7 was unclear due to AR-v9 detection (and the inability to distinguish AR-v9 from other variants). In that study, AR-v567 was prevalent in 23% of CRPC samples, but such expression data were acquired using much greater amounts of RNA (e.g., 200 ng of RNA) extracted from bone metastases of each patient. Hence, the source of the RNA and reliability of the results of such an assay are different (and less reliable) than the methods described herein.

The assay methods described in this application are therefore an improvement over assay methods previously described and/or currently available.

### Example 9: AR-V7 and ARv567es Expression in CTCs Correlates with Outcomes to Taxane Therapy in Men with Metastatic Prostate Cancer

Patients with metastatic castration-resistant prostate cancer (mCRPC) have several treatment options; however, intrinsic and acquired resistance to various treatment modalities is common. The association was evaluated between AR-V7 and ARv567es splice variant expression and response in patients receiving taxanes in the TAXYNERGY study (NCT01718353). TAXYNERGY evaluated the benefit of an early switch from docetaxel to cabazitaxel or vice versa in patients with chemotherapy-naive mCRPC patients. Absence of both variants at baseline was associated with the best prostate-specific antigen response and progression-free survival in patients receiving taxanes. AR nuclear localization did not change following taxane treatment in AR-V7-positive or double-negative patients, suggesting AR-V7 may be dominant over ARv567es. These results indicate that absence of AR splice variants may be associated with a superior response to taxane treatment in mCRPC patients.

### Materials and Methods

### Patients and methods

TAXYNERGY was a non-comparative randomized Phase II study that enrolled chemotherapy-naive patients with progressive mCRPC. Patients were randomly assigned 2:1 to initial treatment with docetaxel 75 mg/m² or cabazitaxel 25 mg/m² every 3 weeks, where the first administration is referred to as cycle 1, the second administration is referred to as cycle 2, etc. Patients achieving at least a 30% PSA decline from baseline by cycle 5, day 1 (C5D1) continued to receive the same taxane, whereas those who did not achieve at least a 30% PSA decline were switched to the alternative taxane at C5D1. Treatment continued until disease progression, death, unacceptable toxicity, or withdrawal of consent (Antonarakis et al. J Clin Oncol 35(28):3181-8 (2017)).

### Ethical Consideration

The protocol complied with recommendations of the 18th World Health Congress (Helsinki, 1964) and all applicable amendments. The study was approved by the institutional review board at each participating center and conducted in compliance with guidelines for Good Clinical Practice. Patients provided written informed consent before participation.

### Patient sample processing and RNA extraction

As part of this study, peripheral blood samples were collected from each patient at baseline prior to initiating protocol treatment and at various time points on and after treatment, in EDTA tubes and shipped to Weill Cornell Medicine within 24 hours of the time of blood draw (Antonarakis 2017). CTCs from the whole blood of patients with mCRPC were captured using the prostate-specific membrane antigen based geometrically enhanced differential immunocapture microfluidic device as previously described (Kirby et al. PLoS One 7(4):e35976 (2012); Galletti et al. Nat Commun 5:5548 (2014)). All patients provided written informed consent.

### Droplet digital PCR

ddPCR is a digital PCR method based on water-oil emulsion droplet technology. The ddPCR system partitions nucleic acid samples into 20,000 nanoliter-sized droplets and PCR amplification is carried out within each droplet. Unlike traditional qPCR, ddPCR allows for absolute quantification of the transcript without the need for normalization or external reference genes (Zhao et al. J Virol Methods 194(1-2):229-34 (2013); Doi et al. Environ Sci Technol 49(9):5601-8 (2015); Huggett et al. Thorax 63(2):154-9 (2008); Racki et al. Plant Methods 10(1):42 (2014)).

Total RNA was extracted from the enriched CTCs pool using the RNAeasy Plus Micro kit (Qiagen) as per manufacturer's instructions. After PCR, droplets that contained a template had a fluorescent signal (positive droplets) that distinguished them from the droplets without a template (negative droplets). The number of target molecules was calculated from the ratio of detected positive droplets to total droplets, using Poisson distribution analysis. CTC-derived mRNA was used as input for the ddPCR reactions arrayed in 96-well format using commercially available multiplexed master mixes of PCR enzyme/buffer from the One-Step RT ddPCR Advanced Kit for Probes (Bio-Rad). Primers and probes specific for AR-FL, ARv567es, and AR-V7 were used to generate amplicons for each transcript and can be found in Table 2. AR-FL, ARv567es, and AR-V7 transcript quantifications were carried out on a QX200 ddPCR system with automated droplet generation (Bio-Rad Laboratories), as described herein. As is standard practice, no threshold was applied to the ddPCR values (Qu et al., Clin Cancer Res 23(3):726-34 (2017); Ma et al. Int J Mol Sci 17(8):10 (2016); Seitz et al. Eur Urol 72:828-834 (2017)). Transcript copy numbers for each patient can be found in Table 7.

**Table 7: Copy number for AR-FL, AR-V7 and ARv567es for the evaluable population at baseline**

| **Patient** | **Timepoint** | **AR- FL** | **AR-V7** | **ARv567es** |
|---|---|---|---|---|
| 1 | 2 | 11 | 5.4 | 9 |
| 2 | 2 | 92 | 4.4 | 9.4 |
| 3 | 2 | 2.8 | 4.6 | 0 |
| 4 | 2 | 278 | 2 | 84 |
| 5 | 2 | 0 | 0 | 1.4 |
| 6 | 2 | 3.2 | 0 | 12.2 |
| 7 | 2 | 580 | 32 | 474 |
| 8 | 2 | 0 | 0 | 1.6 |
| 9 | 2 | 22.4 | 0 | 1.6 |
| 10 | 2 | 14 | 30 | 84 |
| 11 | 2 | 8.2 | 0 | 0 |
| 12 | 2 | 2.6 | 0 | 0 |
| 13 | 2 | 4.6 | 1.4 | 0 |
| 14 | 2 | 288 | 9 | 12.6 |
| 15 | 2 | 68 | 0 | 3 |
| 16 | 2 | 22.2 | 0 | 1.6 |
| 17 | 2 | 434 | 2.6 | 28 |
| 18 | 2 | 20 | 0 | 1.4 |
| 19 | 2 | 54 | 1.8 | 0 |
| 20 | 2 | 70 | 2 | 3 |
| 21 | 2 | 512 | 1.6 | 3.2 |
| 22 | 2 | 22 | 0 | 0 |
| 23 | 2 | 6.8 | 0 | 1.6 |
| 24 | 2 | 26 | 3 | 1.6 |
| 25 | 2 | 11.2 | 1.6 | 3 |
| 26 | 2 | 0 | 18 | 0 |
| 27 | 2 | 28 | 0 | 26 |
| 28 | 2 | 10.2 | 4.4 | 0 |
| 29 | 2 | 18 | 4 | 1.8 |
| 30 | 2 | 0 | 22 | 8.8 |
| 31 | 2 | 128 | 1.8 | 78 |
| 32 | 2 | 28 | 0 | 7 |
| 33 | 2 | 12.6 | 1.6 | 20 |
| 34 | 2 | 58 | 0 | 52 |
| 35 | 2 | 11.4 | 0 | 3.2 |
| 36 | 2 | 62 | 9 | 13 |
| 37 | 2 | 18 | 1.8 | 1.6 |
| 38 | 2 | 6.6 | 0 | 0 |
| 39 | 2 | 238 | 6.4 | 7.4 |
| 40 | 2 | 172 | 3.4 | 2.6 |
| 41 | 2 | 210 | 3.4 | 5.4 |
| 42 | 2 | 1.6 | 1.4 | 8.4 |
| 43 | 2 | 2.8 | 1.6 | 1.6 |
| 44 | 2 | 12.4 | 1.6 | 1.4 |
| 45 | 2 | 10 | 350 | 18 |
| 46 | 2 | 12.4 | 1.6 | 3.4 |

The assay is highly specific for each transcript, which was confirmed using HEK293T cells transfected with plasmids encoding AR-FL, AR-V7 or ARV567es. In these validation assays, each primer set specifically amplified its intended target. Positive and negative controls were included in every assay to ensure optimal primer performance. The primers/probes used for AR-V7 do not co-amplify the highly homologous AR-V9 variant transcript. The assay sensitivity was assessed in spike-in experiments demonstrating single-cell AR-V detection (FIG. 7). Briefly, one or more cells of the human prostate cancer cell line 22RV1, which expresses endogenous AR-FL and AR-V7, was spiked into healthy donor (HD) blood. The cell mixtures were processed through the GEDI device, following the same protocol as for the patient sample processing used herein. The results showed that the assay can reliably and repeatedly detect AR-FL and AR-V7 transcripts from a single prostate cancer cell in the presence of healthy donor peripheral blood mononuclear cells. No AR-V transcripts were detected in healthy donor (HD) blood run through the GEDI, while AR-FL was detected in 6/10 HD samples (FIG. 8).

### Efficacy assessments

PSA levels were measured before treatment administration at each cycle and at the end-of-treatment visit, and then every three months at each follow-up visit until progression, death or study cut-off. PSA response was recorded as at least a 50% (PSA50) reduction from baseline either by C5D1 (prior to switch) or at any point during the entire treatment continuum. Progression-free survival (PFS) was defined as the time between randomization and the first documentation of radiographic tumor progression (using RECIST 1.1), clinical progression (including skeletal-related events, increasing pain requiring escalation of narcotic analgesics, urinary obstruction, etc.), PSA progression, or death from any cause. Progression-free survival was required to be confirmed at least 3 weeks after initial assessment.

### Statistical considerations

Descriptive statistics were used to present the results: mean, standard deviation, median, range, number, and percentage of patients. To relate the presence of splice variants to response, standard chi-square procedures were used. To relate the presence of splice variants to progression-free survival (PFS), Kaplan-Meier, Cox regression, and log-rank techniques were employed.

### Results

### Study population

Of 63 patients enrolled, 61 received taxane treatment. No new safety concerns were identified (Antonarakis et al. J Clin Oncol 35(28):3181-8 (2017)). AR splice variant expression data at baseline and treatment response data were available for 54 patients. For the nine excluded patients, reasons for exclusion included no PSA results (n = 1), non-evaluable CTC mRNA at baseline (n = 6), and randomized but not treated patients (n = 2). Median age was 71 years (range 53-84); 37%, 59% and 4% had an Eastern Cooperative Oncology Group performance status of 0, 1 and 2, respectively; and 43% (23 patients) had received prior AR-targeted therapy (Table 8).

**Table 8. Baseline characteristics**

| | | **All** | **AR-V7-negative/ ARv567es-negative** | **AR-V7-negative / AR^{v567es}**-**positive** | **AR-V7+** |
|---|---|---|---|---|---|
| | | N=54 | n=5 | n = 13 | n= 36 |
| Median age, years (range) | | 71 (53-84) | 64 (57-80) | 71 (53-81) | 71 (53-84) |

| Race, n (%) | | | | | |
|---|---|---|---|---|---|
| Caucasian/White | | 46 (85.2) | 4 (80.0) | 12 (92.3) | 30 (83.3) |
| Black | | 7 (13.0) | 1 (20.0) | 1 (7.7) | 5 (13.9) |
| Asian | | 1 (1.9) | 0 | 0 | 1 (2.8) |

| ECOG PS, n (%) | | | | | |
|---|---|---|---|---|---|
| | 0 | 20 (37.0) | 3 (60.0) | 7 (53.8) | 10 (27.8) |
| | 1 | 32 (59.3) | 2 (40.0) | 6 (46.2) | 24 (66.7) |
| | 2 | 2 (3.7) | 0 | 0 | 2 (5.6) |

| Gleason Score at diagnosis, n (%) | | | | | |
|---|---|---|---|---|---|
| ≤6 | | 7 (13.7) | 1 (25.0) | 3 (23.1) | 3 (8.8) |
| 7 | | 13 (25.5) | 1 (25.0) | 3 (23.1) | 9 (26.5) |
| 8-10 | | 31 (60.8) | 2 (50.0) | 7 (53.8) | 22 (64.7) |
| Prior prostatectomy, n (%) | | 24 (44.4) | 2 (40.0) | 6 (46.2) | 16 (44.4) |
| Prior new-generation AR-targeted therapy, n (%) | | 23 (42.6) | 1 (20.0) | 5 (38.5) | 17 (47.2) |
| Median PSA, ng/mL (range) | | 92.1 (2.4-1558.0) | 20.8 (3.9-832.1) | 89.0 (19.3-713.8) | 113.1 (2.4-1558.0) |
| Albumin, g/dL (SD) | | 39.0 (4.874) | 40.8 (2.388) | 39.2 (4.604) | 38.7 (5.242) |
| Hemoglobin, g/dL (SD) | | 12.24 (1.409) | 13.16 (1.336) | 12.23 (1.266) | 12.12 (1.465) |
| Alkaline phosphatase, U/L (SD) | | 217.8 (260.35) | 78.6 (23.33) | 232 (287.8) | 218.5 (266.13) |

| | | **All** | **AR-V7-negative/ ARv567es-negative** | **AR-V7-negative / AR^{v567es}**-**positive** | **AR-V7+** |
|---|---|---|---|---|---|
| LDH > ULN, n (%) | | 17 (32.7) | 0 | 6 (46.2) | 11 (32.4) |

| Metastases, n (%) | | | | | |
|---|---|---|---|---|---|
| | Bone | 49 (90.7) | 5 (100.0) | 6 (46.2) | 34 (94.4) |
| | Lymph nodes | 28 (51.9) | 3 (60.0) | 10 (76.9) | 14 (38.9) |
| | Visceral | 22 (40.7) | 3 (60.0) | 5 (38.5) | 14 (38.9) |
| | Other | 11 (20.4) | 9 (25.0 | Other | 11 (20.4) |

Baseline characteristics for this subgroup of patients from TAXYNERGY were generally similar to those published for the overall patient population in the TAXYNERGY study (Antonarakis et al. J Clin Oncol 35(28):3181-8 (2017)). Thirty-six patients were randomized to initial treatment, eighteen received initial treatment with docetaxel, and eighteen received initial treatment with cabazitaxel.

### Androgen receptor splice variant expression

Among the 54 patients, 67% (36 patients) and 78% (42 patients) were AR-V7-positive and ARv567es-positive as measured by ddPCR at baseline, respectively. Forty-nine (91%) were positive for either or both splice variants, and only five (9%) were double negative (FIG. 9). ddPCR splice variant expression appeared to be numerically more frequent in those patients who had received prior AR-targeted agents. Prior AR-targeted agents had been given in 33% vs 45% of patients who were ARv567es-negative vs ARv567es-positive, and 33% vs 47% of patients who were AR-V7-negative vs AR-V7-positve. Baseline characteristics by splice variant expression are shown in Table 8. Of note, patients expressing either AR-V-7 or ARv567es splice variant had a numerically higher median PSA level, and ARv567es-positve patients had a numerically higher frequency of visceral metastases (Table 8).

### Correlation between AR-V mRNA expression and AR protein nuclear localization

The inventors' analysis of TAXYNERGY results, revealed that a significant correlation exists between PSA response rate to taxane chemotherapy and changes in CTC AR nuclear localization (%ARNL). Patients with biochemical responses to taxanes had significant decreases in percent CTC AR nuclear localization at eight days after initial taxane treatment (C1D8) compared to the first day of initial taxane treatment (C1D1). As the ARNL immunofluorescence assay does not differentiate between AR-FL or AR-V, the inventors sought to correlate AR-V mRNA expression at baseline with changes in percent CTC AR nuclear localization (%ARNL) at eight days after initial taxane treatment (C1D8) compared to the first day of initial taxane treatment (C1D1). Twenty four of the 54 patients provided %ARNL data at both C1D1 and C1D8 (Tables 9 and 10; FIG. 10).

**Table 9: PSA outcomes and %ARNL according to AR-V7 and ARv567es expression**

| | | **AR-V7-positive** | **AR-V7-negtive** |
|---|---|---|---|
| | | **n=36** | **n = 18** |
| | | | |
| **PSA₅₀ at C5D1, n (%)** | | 13 (36) | 11 (61) |
| | p-value | 0.09 | |
| | | | |
| **PSA₅₀ at any time, n (%)** | | 21 (58) | 14 (78) |
| | p-value | 0.23 | |
| | | | |

| | | **n = 16** | **n = 8** |
|---|---|---|---|
| **C1D1 %ARNL, mean (SD)** | | 62.5 (14.3) | 63. (14.6) |
| **C1D8 %ARNL, mean (SD)** | | 62.2 (15.2) | 42.1 (11.1) |
| **C1D8 - C1D1 %ARNL, mean (SD)** | | -0.4 (13.2) | -21.5 (22.1) |
| | | **p-value 0.0023** | |
| | | | |
| | | | |

| | | **AR^{v567es}-positive** | **AR^{v567es}-negative** |
|---|---|---|---|
| | | **n=42** | **n = 12** |
| **PSA₅₀ at C5D1, n (%)** | | 16 (38) | 8 (67) |
| | p-value vs Group 1 | 0.11 | |
| **PSA₅₀ at any time, n (%)** | | 24 (57) | 11 (92) |
| | p-value | 0.04 | |
| | | | |

| | | **n = 18** | **n=6** |
|---|---|---|---|
| **C1D1 %ARNL, mean (SD)** | | 63.7 (16.8) | 62.6 (13.6) |
| **C1D8 %ARNL, mean (SD)** | | 56.3 (17.4) | 55.2 (17.0) |
| **C1D8 - C1D1 %ARNL, mean (SD)** | | -7.4 (11.8) | -7.4 (21.3) |
| | p-value | 0.9985 | |

**Table 10: PSA outcomes and %ARNL in AR-V7-positive patients and AR-V7-negative patients with or without ARv567es expression**

| | **Group 1** | **Group 2** | **Group 3** | **Total n = 54** |
|---|---|---|---|---|
| | **AR-V7-negative and AR^{v567es}**-**negative n=5** | **AR-V7-negative and AR^{v567es}**-**positive n=13** | **All AR-V7-positive n = 36** | |
| **PSA₅₀ at C5D1, n (%)** | 4 (80.0%) | 7 (53.9) | 13 (36.1) | 24 (44.4) |
| p-value vs Group 1 | | 0.31 | 0.26 | |
| p-value vs Group 2 | | | 0.06 | |
| Trend Group 1 > Group 2 > Group 3 | 0.1748 | | | |
| **PSA₅₀ at any time, n (%)** | 5 (100.0) | 9 (69.2) | 21 (58.3) | 35 (64.8) |
| p-value vs Group 1 | | 0.16 | 0.49 | |
| p-value vs Group 2 | | | 0.07 | |
| Trend Group 1 > Group 2 > Group 3 | 0.33 | | | |

| | **GROUP 1** | **GROUP 2** | **GROUP 3** | |
|---|---|---|---|---|
| | **AR-V7-negative and AR^{v567es}**-**negative n = 3** | **AR-V7-negative and AR^{v567es}**-**positive n= 5** | **All AR-V7-positive n = 16** | **Total n=24** |
| **C1D1 %ARNL,** mean **(SD)** | 56.9 (19.2) | 67.6 (11.6) | 62.5 (14.3) | 62.9 (14.1) |
| **C1D8 %ARNL, mean (SD)** | 44.1 (4.8) | 41.0 (14.1) | 62.2 (15.2) | 55.5 (16.8) |
| **C1D8 - C1D1 %ARNL, mean (SD)** | -12.9 (15.4) | -26.7 (25.4) | -0.4 (13.2) | -7.4 (19.1) |
| p-value vs Group 1 | | 0.52 | 0.08 | |
| p-value vs Group 2 | | | 0.52 | |
| Trend Group 1 > Group 2 > Group 3 | 0.08 | | | |

| | | | | |
|---|---|---|---|---|
| AR, androgen receptor; ARNL, androgen receptor nuclear localization; C1D1, Cycle 1 Day 1; C1D8, Cycle 1 Day 8; C5D1, Cycle 5 Day 1; PSA50, 50% reduction from baseline in prostate-specific antigen; SD, standard deviation. | | | | |

As many of the samples co-expressed both variants, to determine the relative impact of each variant, the samples were initially categorized into four groups (double positive, double negative, AR-V7-positive/ARv567es-negative, and AR-V7-negative/ARV567es-positive). Of these 24 patients, thirteen were double positive, three were double negative, three were AR-V7-positive/ARv567es-negative and five were AR-V7-negative/ARv567es-positive.

Patients who were AR-V7-positive /ARv567es-negative had a 1.9% decrease in %ARNL by C1D8, compared with a 26.7% decrease in patients who were AR-V7-negative /ARv567es-positive by C1D8 (not shown), while double-positive patients had a change of 0% by C1D8 (not shown).

These data taken together, show that AR-V7 can have a dominant role in driving taxane resistance. Due to the small number of patients in each group and since the presence of AR-V7 seemed to have a dominant effect over ARv567es, results are presented in three groups: AR-V7-positive (regardless of ARv567es status), ARv567es-positive /AR-V7-negative, and double negative in FIG. 10, and Table 10.

Patients who were double negative for AR-V7 and ARv567es expression had a 12.9% decrease in %ARNL by C1D8, compared with a 26.7% decrease in patients who were AR-V7-negative/ARv567es-positive by C1D8, and compared with a negligible 0.4% decrease in patients who were AR-V7-positive by C1D8 (p = 0.08 for trend) (Table 10). Comparison of the change in %ARNL between AR-V7-positive vs AR-V7-negative patients, regardless of ARv567es expression, revealed a 21.5% decrease in %ARNL in AR-V7-negative patients by C1D8 vs a 0.4% decrease in AR-V7-positive patients by C1D8 (p=0.0023) (Table 9).

### Efficacy outcomes

Splice variant expression and PSA outcomes are presented in Table 9 and Table 10. PSA₅₀ response at cycle 5, day 1 (C5D1) was observed in 61.1% of AR-V7-negative patients vs 36.1% of AR-V7-positive patients (p = 0.09) (Table 9). Similar trends were observed for PSA₅₀ response at any time during the study. For example, PSA₅₀ response at C5D1 was observed at any time in 77.8% of AR-V7-negative patients vs 58.3% of AR-V7-positve patients (p = 0.23).

PSA₅₀ responses at cycle 5, day 1 (C5D1) were observed in 38% of ARv567es-positive patients (regardless of AR-V7 status) vs 67% of ARv567es-negative patients (p=0.11).

For PSA₅₀ at any time (Table 9), responses were observed in 57% of ARv567es-positive patients vs 92% of ARv567es-negative patients (p=0.04). However, when patients were divided into three subgroups, in order to model a dominant role of AR-V7, 80% of AR-V7-negative/ ARv567es-negative patients had a greater than 50% PSA decline by Cycle 5 vs 53.9% in AR-V7-negative/ ARv567es-positve patients (p=0.31) (Table 10).

In double negative patients, all patients had a greater than 50% PSA decline at any time in the study vs 69.2% of AR-V7-negative/ARv567es-positive patients (p = 0.16) (Table 10). Median AR-V7 and ARv567es copy numbers at baseline were numerically lower in PSA responders than in PSA non-responders; however, the difference was not statistically significant, and overall variability was high (Table 11).

**Table 11: Copy number for AR-V7 and ARv567es stratified by PSA response**

| **PSA₅₀ at C5D1** | | **Total (n = 54)** | **Yes (n = 24)** | **No (n = 30)** |
|---|---|---|---|---|
| **AR-V7** absolute copy number at baseline | Mean (SD) | 10.49 (47.58) | 2.92 (6.19) | 16.55 (63.42) |
| | Median | 1.6 | 1.4 | 1.9 |
| | IQ Range | 0.0-4.4 | 0.0-3.5 | 1.4-6.4 |
| | Range | 0.0- 350.0 | 0.0-30.0 | 0.0-350.0 |
| | p-value | | 0.0842 | |
| | | | | |
| **ARv567es** absolute copy number at baseline | Mean (SD) | 1 9.57 (66.10) | 10.64 (27.67) | 26.72 (86.37) |
| | Median | 3.0 | 1.7 | 6.2 |
| | IQ Range | 1.4- 11.2 | 0.0- 7.4 | 1.6-12.2 |
| | Range | 0.0-474.0 | 0.0- 84.0 | 0.0- 474.0 |
| | p-value | | 0.0898 | |
| | | | | |

| **PSA₅₀ at any time** | | **Total (n = 54)** | **Yes (n = 35)** | **No (n = 1 9)** |
|---|---|---|---|---|
| | Mean (SD) | I 1 0.49 (47.58) | I 2.85 (5.80) | I 24.56 (79.25) |

| **PSA₅₀ at C5D1** | | **Total (n = 54)** | **Yes (n = 24)** | **No (n = 30)** |
|---|---|---|---|---|
| **AR-V7** absolute copy number at baseline | Median | 1.6 | 1.6 | 3.0 |
| | IQ Range | 0.0- 4.4 | 0.0- 3.4 | 1.4- 10.4 |
| | Range | 0.0- 350.0 | 0.0-30.0 | 0.0-350.0 |
| | p-value | | 0.0818 | |
| | | | | |
| **ARv567es** absolute copy number at baseline | Mean (SD) | 19.57 (66.10) | 10.56 (22.96) | 36.18 (106.87) |
| | Median | 3.0 | 1.6 | 8.4 |
| | IQ Range | 1.4- 11. 2 | 0.0- 7.0 | 1.6- 18.0 |
| | Range | 0.0-474.0 | 0.0- 84.0 | 0.0- 474.0 |
| | p-value | | 0.171 5 | |

Median PFS was 16.6 months for double negative patients compared with 11.2 months for AR-V7-negtive/ARv567es-positive (p = 0.18), and 8.5 months for AR-V7-positive patients (p = 0.004). (FIG. 11). For the AR-V7-negative vs ARV7-positive comparison, median PFS was 12.0 vs 8.5 months (hazard ratio = 0.38, p = 0.01). The p-value for the trend across AR-V7-negative / ARv567es-negative, AR-V7-negative / ARv567es-positive and AR-V7-positive was 0.0013. For the ARv567es-negative vs ARv567es-positive comparison, median PFS was 12.7 vs 7.3 months (hazard ratio = 0.37, p = 0.02) (FIG.11).

The results provided herein illustrate the association of treatment outcomes (PSA response and PFS) with the expression of ARv567es and AR-V7 as measured by ddPCR at baseline and after taxane treatment. Previous studies using RT-PCR to measure AR levels have not shown that AR-V7 presence is associated with primary taxane resistance (Antonarakis et al. JAMA Oncol 1(5):582-91 (2015)). However, as described herein, outcomes of AR-V7 and ARv567es double negative mCRPC patients that were enrolled in the TAXYNERGY study had PSA response rates that were numerically superior to ARv567es-positive / AR-V7-negative mCRPC patients compared to AR-V7-positive mCRPC patients. PFS was longest in double negative patients who did not express either of the AR-V7 and ARv567es splice variants. PFS was longer with taxane therapy in AR-V7-negative patients compared with AR-V7-positive patients. Absence of AR splice variants as measured by ddPCR was associated with superior response and PFS to cabazitaxel or docetaxel in patients with mCRPC.

### Example 10: Transferrin Receptor (TfR) Facilitates Lung Cancer Tumor Cell Detection and Isolation

This Example illustrates improved methods for identifying CTCs from lung cancer patients. No CTC identification methodology is currently used in non-small cell lung cancer (NSCLC) patients. For example, the CellSearch^{®} method detects and enumerates CTCs that are CD45-negative, EpCAM-positive, and positive for cytokeratins 8, 18, and/or 19. CellSearch^{®} has been FDA-approved for CTC detection in metastatic breast, prostate and colon cancer patients, but not in metastatic lung cancer patients. Hence, CTC identification and molecular characterization in NSCLC is an unmet clinical need.

### Methods

Samples were obtained from NSCLC patients and CTCs were enriched using an antigen agnostic process that included RosetteSep CD45 depletion, staining for TfR, staining for CK (a standard intracellular CTC identifier), staining for TTF1 (standard marker for epithelial cells of lung origin), staining for CD45 (leukocyte marker), and staining with DAPI (nuclear marker).

All TfR-positive CTCs were also TTF1-positive, confirming the lung origin of the cell population in the samples.

### Results

CK is a standard intracellular CTC identifier. As shown in Table 12 and FIG. 12, there is concordance between TfR and cytokeratin (CK) expression in CTCs. Note that CK as an intracellular marker cannot provide CTC enrichment when it is used alone.

Tables 12a and 12b shows that CK and TfR are generally co-expressed in CTCs.

**Table 12a: Concordance between CK and TfR expression in CTCs from early stage NSCLC patients**

| | **Ref. No.** | **Histology** | **CK-positive** | **TfR-positive** |
|---|---|---|---|---|
| 1 | BB 1090 | Adenoca | 9 | 10 |
| 2 | BB 1093 | Carcinoma | 11 | 11 |
| 3 | BB 1094 | Squamous | 9 | 18 |
| 4 | BB 1092 | Adenoca | 23 | 24 |
| 5 | BB 1095 | Adenoca | 20 | 20 |
| 6 | BB 1098 | Adenoca | 10 | 14 |
| 7 | BB 1102 | Biopsy | 6 | 5 |
| 8 | BB 1100 | Adenoca | 7 | 16 |
| 9 | BB 1104 | Adenoca | 21 | 19 |
| 10 | BB 1109 | Adenoca | 3 | 11 |
| 11 | BB 1099 | Adenoca | 37 | 43 |
| 12 | BB 1103 | Adenoca | 22 | 23 |
| 13 | BB 1107 | Adenoca | 35 | 28 |
| 14 | BB 1110 | Adenoca | 2 | 2 |
| 15 | BB 1111 | Adenoca | 5 | 3 |
| 16 | BB 1117 | Squamous | 16 | 22 |
| 17 | BB 1125 | Adenoca | 6 | 8 |
| 18 | BB 1119 | Adenoca | 20 | 20 |
| 19 | BB 1120 | Adenoca | 5 | 5 |
| 20 | BB 1129 | Adenoca | 7 | 10 |
| 21 | BB 1127 | Adenoca | 8 | 13 |

**Table 12b: Summary Concordance between CK and TfR expression in CTCs**

| | **CK-positive** | **TfR-positive** |
|---|---|---|
| **Mean CTC No./sample** | 13.4 | 15.5 |
| **Median CTC No./sample** | 9 | 14 |

FIG. 12 illustrates that TfR-positive labeling identifies CTCs across cancer stage, and across EGFR or K-Ras mutation status in early-stage NSCLC patients.

Approximately 10-15% of patients with non-small cell lung cancer in the United States and 35% in Asia have an EGFR positive mutation. KRAS gene mutations are found in 15 to 25 percent of all lung cancer cases but are more frequent in white populations than in Asian populations. For example, 25 to 50 percent of whites with lung cancer have KRAS gene mutations, whereas 5 to 15 percent of Asians with lung cancer have KRAS gene mutations.

These results show that TfR identifies CTCs in early stage NSCLC, a clinical scenario where the standard EpCAM-based CellSearch^{®} has always performed poorly.

### Example 11: Transferrin Receptor (TfR) Facilitates Pancreatic Cancer Tumor Cell Detection and Isolation

This Example illustrates improved methods for identifying CTCs from pancreatic cancer patients. CellSearch^{®} has been FDA-approved for CTC detection in metastatic breast, prostate and colon cancer patients, but it detects fewer CTCs than are actually present in samples from pancreatic cancer patients (see, e.g., Khoja et al. Br J Cancer 106(3): 508-516 (2012)). Hence, CTC identification and molecular characterization in pancreatic cancer patients is an unmet clinical need.

### Methods

Samples were obtained from pancreatic cancer patients and CTCs were enriched using an antigen agnostic process that included RosetteSep CD45 depletion. Forty-three samples from 33 patients were evaluated. Matched samples were separately stained for TfR and for EpCAM and the numbers of cells expressing TfR and EpCAM was evaluated.

One patient (patient 13) was further evaluated. Patient 13 had nine cycles of FOLFIRINOX but had a pathological progression in December 2017, so she was switched to Gem/ Abraxane and now has stable disease. Samples were obtained at different time points and evaluated using the transferrin receptor methods described herein to detect and quantify CTCs.

### Results

FIG. 13 shows the numbers of pancreatic CTCs from matched samples that express TfR and EpCAM. The median number of TfR-positive and EpCAM-negative cells detected was 148 (range : 2-4182) while the median number of EpCAM-positive and TfR-negative cells detected was 68 (range: 0-1552). As shown in FIG. 13, TfR-positive labeling identifies that more CTCs are generally present in these samples than EpCAM labeling does in these matching patient samples.

FIG. 14 shows that more CTCs were detected using transferrin receptor as the marker for pancreatic CTCs in samples obtained in December 2017 when the patient was undergoing pathological progression, than were detected before significant pathological progression began (in September 2017). Significantly, use of transferrin receptor was more effective for quantifying pancreatic CTCs than was EpCAM (see FIG. 14).

### References

Antonarakis, E. S., C. Lu, H. Wang, B. Luber, M. Nakazawa, J. C. Roeser, Y. Chen, T. A. Mohammad, Y. Chen, H. L. Fedor, T. L. Lotan, Q. Zheng, A. M. De Marzo, J. T. Isaacs, W. B. Isaacs, R. Nadal, C. J. Paller, S. R. Denmeade, M. A. Carducci, M. A. Eisenberger, and J. Luo. 2014. 'AR-V7 and resistance to enzalutamide and abiraterone in prostate cancer', N Engl J Med, 371: 1028-38.
Hornberg, E., E. B. Ylitalo, S. Crnalic, H. Antti, P. Stattin, A. Widmark, A. Bergh, and P. Wikstrom. 2011. 'Expression of androgen receptor splice variants in prostate cancer bone metastases is associated with castration-resistance and short survival', PLoS One, 6: e19059.
Kohli, M., Y. Ho, D. W. Hillman, J. L. Van Etten, C. Henzler, R. Yang, J. M. Sperger, Y. Li, E. Tseng, T. Hon, T. Clark, W. Tan, R. E. Carlson, L. Wang, H. Sicotte, H. Thai, R. Jimenez, H. Huang, P. T. Vedell, B. W. Eckloff, J. F. Quevedo, H. C. Pitot, B. A. Costello, J. Jen, E. D. Wieben, K. A. T. Silverstein, J. M. Lang, L. Wang, and S. M. Dehm. 2017. 'Androgen Receptor Variant AR-V9 Is Coexpressed with AR-V7 in Prostate Cancer Metastases and Predicts Abiraterone Resistance', Clin Cancer Res.
Liu, X., E. Ledet, D. Li, A. Dotiwala, A. Steinberger, A. Feibus, J. Li, Y. Qi, J. Silberstein, B. Lee, Y. Dong, O. Sartor, and H. Zhang. 2016. 'A Whole Blood Assay for AR-V7 and ARv567es in Patients with Prostate Cancer', J Urol, 196: 1758-63.
Lokhandwala, P. M., S. L. Riel, L. Haley, C. Lu, Y. Chen, J. Silberstein, Y. Zhu, G. Zheng, M. T. Lin, C. D. Gocke, A. W. Partin, E. S. Antonarakis, J. Luo, and J. R. Eshleman. 2017. 'Analytical Validation of Androgen Receptor Splice Variant 7 Detection in a Clinical Laboratory Improvement Amendments (CLIA) Laboratory Setting', J Mol Diagn, 19: 115-25.
Ma, Y., A. Luk, F. P. Young, D. Lynch, W. Chua, B. Balakrishnar, P. de Souza, and T. M. Becker. 2016. 'Droplet Digital PCR Based Androgen Receptor Variant 7 (AR-V7) Detection from Prostate Cancer Patient Blood Biopsies', Int J Mol Sci, 17*.*
Qu, F., W. Xie, M. Nakabayashi, H. Zhang, S. H. Jeong, X. Wang, K. Komura, C. J. Sweeney, O. Sartor, G. M. Lee, and P. W. Kantoff. 2017. 'Association of AR-V7 and Prostate-Specific Antigen RNA Levels in Blood with Efficacy of Abiraterone Acetate and Enzalutamide Treatment in Men with Prostate Cancer', Clin Cancer Res, 23: 726-34.
Thadani-Mulero, M., L. Portella, S. Sun, M. Sung, A. Matov, R. L. Vessella, E. Corey, D. M. Nanus, S. R. Plymate, and P. Giannakakou. 2014. 'Androgen receptor splice variants determine taxane sensitivity in prostate cancer', Cancer Res, 74: 2270-82.
Hu R, Dunn TA, Wei S, Isharwal S, Veltri RW, Humphreys E, et al. Ligand-independent androgen receptor variants derived from splicing of cryptic exons signify hormone-refractory prostate cancer. Cancer Res 2009;69(1):16-22.
Dehm SM, Schmidt LJ, Heemers HV, Vessella RL, Tindall DJ. Splicing of a novel androgen receptor exon generates a constitutively active androgen receptor that mediates prostate cancer therapy resistance. Cancer Res 2008;68(13):5469-77.
Qu Y, Dai B, Ye D, Kong Y, Chang K, Jia Z, et al. Constitutively active AR-V7 plays an essential role in the development and progression of castration-resistant prostate cancer. Sci Rep 2015; 5:7654.
Caffo O, Maines F, Veccia A, Kinspergher S, Galligioni E. Splice variants of androgen receptor and prostate cancer. Oncol Rev 2016;10(1):297.
National Comprehensive Cancer Network I. NCCN clinical practice guidelines in oncology (NCCN guidelines) prostate cancer version 2.2018. 2018.
Antonarakis ES, Lu C, Wang H, Luber B, Nakazawa M, Roeser JC, et al. AR-V7 and resistance to enzalutamide and abiraterone in prostate cancer. N Engl J Med 2014;371(11):1028-38.
Conteduca V, Wetterskog D, Sharabiani MTA, Grande E, Fernandez-Perez MP, Jayaram A, et al. Androgen receptor gene status in plasma DNA associates with worse outcome on enzalutamide or abiraterone for castration-resistant prostate cancer: A multi-institution correlative biomarker study. Ann Oncol 2017;28(7):1508-16.
Shore N, Heidenreich A, Saad F. Predicting response and recognizing resistance: Improving outcomes in patients with castration-resistant prostate cancer. Urology 2017.
Antonarakis ES, Lu 414 C, Luber B, Wang H, Chen Y, Zhu Y, et al. Clinical significance of androgen receptor splice variant-7 mRNA detection in circulating tumor cells of men with metastatic castration-resistant prostate cancer treated with first- and second-line abiraterone and enzalutamide. J Clin Oncol 2017;35(19):2149-56.
Qu F, Xie W, Nakabayashi M, Zhang H, Jeong SH, Wang X, et al. Association of AR-V7 and prostate-specific antigen RNA levels in blood with efficacy of abiraterone acetate and enzalutamide treatment in men with prostate cancer. Clin Cancer Res 2017;23(3):726-34.
Maughan BL, Xhou XC, Suzman DL, Nadal R, Bassi S, Schweizer MT, et al. Optimal sequencing of docetaxel and abiraterone in men with metastatic castration-resistant prostate cancer. Prostate 2015;75(15):1814-20.
Thadani-Mulero M, Portella L, Sun S, Sung M, Matov A, Vessella RL, et al. Androgen receptor splice variants determine taxane sensitivity in prostate cancer. Cancer Res 2014;74(8):2270-82.
Darshan MS, Loftus MS, Thadani-Mulero M, Levy BP, Escuin D, Zhou XK, et al. Taxane-induced blockade to nuclear accumulation of the androgen receptor predicts clinical responses in metastatic prostate cancer. Cancer Res 2011;71(18):6019-29.
Antonarakis ES, Tagawa ST, Galletti G, Worroll D, Ballman K, Vanhuyse M, et al. Randomized, noncomparative, phase II trial of early switch from docetaxel to cabazitaxel or vice versa, with integrated biomarker analysis, in men with chemotherapy-naive, metastatic, castration-resistant prostate cancer. J Clin Oncol 2017;35(28):3181-8.
Robinson D, Van Allen EM, Wu YM, Schultz N, Lonigro RJ, Mosquera JM, et al. Integrative clinical genomics of advanced prostate cancer. Cell 2015;161(5):1215-28.
Hornberg E, Ylitalo EB, Crnalic 435 S, Antti H, Stattin P, Widmark A, et al. Expression of androgen receptor splice variants in prostate cancer bone metastases is associated with castration-resistance and short survival. PLoS One 2011;6(4):e19059.
Antonarakis ES, Nakazawa M, Luo J. Resistance to androgen-pathway drugs in prostate cancer. N Engl J Med 2014;371(23):2234.
Antonarakis ES, Lu C, Luber B, Wang H, Chen Y, Nakazawa M, et al. Androgen receptor splice variant 7 and efficacy of taxane chemotherapy in patients with metastatic castration-resistant prostate cancer. JAMA Oncol 2015;1(5):582-91.
Scher HI, Lu D, Schreiber NA, Louw J, Graf RP, Vargas HA, et al. Association of AR-V7 on circulating tumor cells as a treatment-specific biomarker with outcomes and survival in castration resistant prostate cancer. JAMA Oncol 2016;2(11):1441-9.
Zhang G, Liu X, Li J, Ledet E, Alvarez X, Qi Y, et al. Androgen receptor splice variants circumvent AR blockade by microtubule-targeting agents. Oncotarget 2015;6(27):23358-71.
Onstenk W, Sieuwerts AM, Kraan J, Van M, Nieuweboer AJ, Mathijssen RH, et al. Efficacy of cabazitaxel in castration-resistant prostate cancer is independent of the presence of AR-V7 in circulating tumor cells. Eur Urol 2015;68(6):939-45.
Kohli M, Ho Y, Hillman DW, Van Etten JL, Henzler C, Yang R, et al. Androgen receptor variant AR452 V9 is coexpressed with AR-V7 in prostate cancer metastases and predicts abiraterone resistance. Clin Cancer Res 2017;23(16):4704-15.
Kirby BJ, Jodari M, Loftus MS, Gakhar G, Pratt ED, Chanel-Vos C, et al. Functional characterization of circulating tumor cells with a prostate-cancer-specific microfluidic device. PLoS One 2012;7(4):e35976.
Galletti G, Matov A, 457 Beltran H, Fontugne J, Miguel MJ, Cheung C, et al. ERG induces taxane resistance in castration-resistant prostate cancer. Nat Commun 2014;5:5548.
Vogelstein B, Kinzler KW. Digital PCR. Proc Natl Acad Sci U S A 1999;96(16):9236-41.
Pinheiro LB, Coleman VA, Hindson CM, Herrmann J, Hindson BJ, Bhat S, et al. Evaluation of a droplet digital polymerase chain reaction format for DNA copy number quantification. Anal Chem 2012;84(2):1003-11.
Zhao H, Wilkins K, Damon IK, Li Y. Specific qPCR assays for the detection of orf virus, pseudocowpox virus and bovine papular stomatitis virus. J Virol Methods 2013; 194(1-2):229-34.
Doi H, Takahara T, Minamoto T, Matsuhashi S, Uchii K, Yamanaka H. Droplet digital polymerase chain reaction (PCR) outperforms real-time PCR in the detection of environmental DNA from an invasive fish species. Environ Sci Technol 2015;49(9):5601-8.
Huggett JF, Taylor MS, Kocjan G, Evans HE, Morris-Jones S, Gant V, et al. Development and evaluation of a real-time PCR assay for detection of pneumocystis jirovecii DNA in bronchoalveolar lavage fluid of HIV-infected patients. Thorax 2008;63(2):154-9.
Racki N, Dreo T, Gutierrez-Aguirre I, Blejec A, Ravnikar M. Reverse transcriptase droplet digital PCR shows high resilience to PCR inhibitors from plant, soil and water samples. Plant Methods 2014;10(1):42,014-0042-6. eCollection 2014.
Ma Y, Luk A, Young FP, Lynch D, Chua W, Balakrishnar B, et al. Droplet digital PCR based androgen receptor variant 7 (AR-V7) detection from prostate cancer patient blood biopsies. Int J 476 Mol Sci 2016;17(8):10.3390/ijms17081264.
Seitz AK, Thoene 477 S, Bietenbeck A, Nawroth R, Tauber R, Thalgott M, et al. AR-V7 in peripheral whole blood of patients with castration-resistant prostate cancer: Association with treatment-specific outcome under abiraterone and enzalutamide. Eur Urol 2017;72:828-834.
Galletti G, Leach BI, Lam L, Tagawa ST. Mechanisms of resistance to systemic therapy in metastatic castration-resistant prostate cancer. Cancer Treat Rev 2017;57:16-27.
Steinestel J, Luedeke M, Arndt A, Schnoeller TJ, Lennerz JK, Wurm C, et al. Detecting predictive androgen receptor modifications in circulating prostate cancer cells. Oncotarget 2015.
Lokhandwala PM, Riel SL, Haley L, Lu C, Chen Y, Silberstein J, et al. Analytical validation of androgen receptor splice variant 7 detection in a clinical laboratory improvement amendments (CLIA) laboratory setting. J Mol Diagn 2017;19(1):115-25.
Miyamoto DT, Lee RJ, Kalinich M, LiCausi J, Zheng Y, Chen T, et al. An RNA-based digital circulating tumor cell signature is predictive of drug response and early dissemination in prostate cancer. Cancer Discov 2018.
Brunetto GS, Massoud R, Leibovitch EC, Caruso B, Johnson K, Ohayon J, et al. Digital droplet PCR (ddPCR) for the precise quantification of human T-lymphotropic virus 1 proviral loads in peripheral blood and cerebrospinal fluid of HAM/TSP patients and identification of viral mutations. J Neurovirol 2014;20(4):341-51.
Sanders R, Mason DJ, Foy CA, Huggett JF. Considerations for accurate gene expression measurement by reverse transcription quantitative PCR when analysing clinical samples. Anal Bioanal Chem 2014;406(26):6471-83.
Sanders R, Mason DJ, Foy CA, Huggett JF. Evaluation of digital PCR for absolute RNA quantification. PLoS One 2013;8(9):e75296.
Liu X, Ledet E, Li D, Dotiwala A, Steinberger 499 A, Feibus A, et al. A whole blood assay for AR-V7 and AR(v567es) in patients with prostate cancer. J Urol 2016;196(6):1758-63.
Thadani-Mulero M, Nanus DM, Giannakakou P. Androgen receptor on the move: Boarding the microtubule expressway to the nucleus. Cancer Res 2012;72(18):4611-5.
Zhu ML, Horbinski CM, Garzotto M, Qian DZ, Beer TM, Kyprianou N. Tubulin-targeting chemotherapy impairs androgen receptor activity in prostate cancer. Cancer Res 2010;70(20):7992-8002.
Scher HI, Graf RP, Schreiber NA, McLaughlin B, Lu D, Louw J, et al. Nuclear-specific AR-V7 protein localization is necessary to guide treatment selection in metastatic castration-resistant prostate cancer. Eur Urol 2017;71(6):874-82.

As used herein and in the appended claims, the singular forms "a," "an," and "the" include plural reference unless the context clearly dictates otherwise. Thus, for example, a reference to "a nucleic acid" or "a primer" or "a cell" includes a plurality of such nucleic acids, primers, or cells (for example, a solution or dried preparation of nucleic acids or primers, or a population of cells), and so forth. In this document, the term "or" is used to refer to a nonexclusive or, such that "A or B" includes "A but not B," "B but not A," and "A and B," unless otherwise indicated.

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 5% or 10% of the particular term.

## Claims

1. A method comprising:
a. capturing circulating cancer cells from a sample from a test subject;
b. extracting mRNA to provide a RNA sample; and
c. detecting and quantifying each of full-length androgen receptor (AR-FL), androgen receptor variant 7 (AR-V7), and androgen receptor variant 5,6,7 (AR-V567) in parallel digital droplet PCR assays;
wherein:
(i) the parallel digital droplet PCR assay(s) for detecting and quantifying for AR-FL optionally comprises the following set of primers or probes: a primer according to the sequence as set forth in SEQ ID NO: 7 and a primer according to the sequence as set forth in SEQ ID NO: 8, and optionally a probe according to the sequence as set forth in SEQ ID NO: 9;
(ii) the parallel digital droplet PCR assay(s) for detecting and quantifying for AR-V7 comprises the following set of primers or probes: a primer according to the sequence as set forth in SEQ ID NO: 11 and a primer according to the sequence as set forth in SEQ ID NO: 12, and optionally a probe according to the sequence as set forth in SEQ ID NO: 13; and
(iii) the parallel digital droplet PCR assay(s) for detecting and quantifying for AR-v567es optionally comprises the following set of primers or probes: a primer according to the sequence as set forth in SEQ ID NO: 15 and a primer according to the sequence as set forth in SEQ ID NO: 16, and optionally a probe according to the sequence as set forth in SEQ ID NO: 17.

2. The method of claim 1, comprising one or more digital droplet PCR assays for detecting and quantifying AR-FL, each digital droplet PCR assay for detecting and quantifying AR-FL performed in a separate droplet comprising a primer according to the sequence as set forth in SEQ ID NO: 7 and a primer according to the sequence as set forth in SEQ ID NO: 8; wherein the droplet can optionally further comprise a probe according to the sequence as set forth in SEQ ID NO: 9.

3. The method of any one of claims 1 or 2, comprising one or more digital droplet PCR assays for detecting and quantifying for AR-V7, each digital droplet PCR assay for detecting and quantifying for AR-V7 performed in a separate droplet comprising a primer according to the sequence as set forth in SEQ ID NO: 11 and a primer according to the sequence as set forth in SEQ ID NO: 12; wherein the droplet can optionally further comprise a probe according to the sequence as set forth in SEQ ID NO: 13.

4. The method of any one of claims 1, 2 or 3, comprising one or more digital droplet PCR assays for detecting and quantifying AR-v567es, each digital droplet PCR assay for detecting and quantifying AR-v567es performed in a separate droplet comprising a primer according to the sequence as set forth in SEQ ID NO: 15 and a primer according to the sequence as set forth in SEQ ID NO: 16; wherein the droplet optionally further comprises a probe according to the sequence as set forth in SEQ ID NO: 17.

5. The method of any one of claims 1-3 or 4, wherein the primers or probes are covalently or non-covalently bonded to one or two labels fluorescent, chemiluminescent, or radioactive labels.

6. The method of any one of claims 1-4 or 5, wherein capturing circulating cancer cells from the sample comprises isolating cells that express transferrin receptor, optionally further comprising depletion of CD45+ cells from the sample before isolating cells that express transferrin receptor.

7. The method of any one of claims 1-5 or 6, wherein the test subject is healthy.

8. The method of any one of claims 1-5 or 6, wherein the test subject is suspected of having cancer, or metastatic cancer, or prostate cancer.

9. The method of any one of claims 1-7 or 8, further comprising informing the test subject or medical personnel providing medical care for the test subject of detection, quantities, and/or quantification of full-length androgen receptor (AR-FL), androgen receptor variant 7 (AR-V7), or androgen receptor variant 5,6,7 (AR-V567) levels detected or quantified in the parallel digital droplet PCR assays.

10. A taxane for use in a method of treating prostate cancer in a subject, wherein the method comprises:
(i) detecting the absence of AR-V7 in a sample by the method of claim 1;
(ii) providing the taxane for administration to the subject.

11. A composition comprising two or more types of primers or probes with at least 15 nucleotides of SEQ ID NO: 7, 8, 9, 11, 12, 13, 15, 16, and 17, wherein one or more type of primer or probe is covalently or non-covalently bound to a label, wherein the composition comprises:
a primer having the sequence as set forth in SEQ ID NO: 11 and a primer comprising at least 15 nucleotides according to the sequence as set forth in SEQ ID NO: 12, wherein the composition can optionally further comprise a probe comprising at least 15 nucleotides according to the sequence as set forth in SEQ ID NO: 13.

12. The composition of claim 11, comprising a primer comprising at least 15 nucleotides according to the sequence as set forth in SEQ ID NO: 7 and a primer comprising at least 15 nucleotides according to the sequence as set forth in SEQ ID NO: 8, wherein the composition can optionally further comprise a probe comprising at least 15 nucleotides according to the sequence as set forth in SEQ ID NO: 9.

13. The composition of any one of claims 11 or 12, comprising a primer comprising at least 15 nucleotides according to the sequence as set forth in SEQ ID NO: 15 and a primer comprising at least 15 nucleotides according to the sequence as set forth in SEQ ID NO: 16, wherein the composition can optionally further comprise a probe comprising at least 15 nucleotides according to the sequence as set forth in SEQ ID NO: 17.

14. The composition of any one of claims 11-13, wherein one or more primers or probes are covalently or non-covalently bonded to one or two fluorescent, chemiluminescent, or radioactive labels.

15. A method according to any one of claims 1-9, wherein capturing circulating cancer cells from the sample comprises:
a. depleting the sample obtained from the test subject of CD45+ cells to obtain a CD45+ depleted sample; and
b. staining the CD45+ depleted sample with a labeled reagent that binds to transferrin receptor (TfR) to obtain a TfR-labeled sample;
c. optionally quantifying the TfR-labeled cells in the TfR-labeled sample.

## Patentansprüche

1. Verfahren, umfassend:
a. Einfangen zirkulierender Krebszellen aus einer Probe von einem Untersuchungssubjekt;
b. Extrahieren von mRNA, um eine RNA-Probe bereitzustellen; und
c. Nachweisen und Quantifizieren jedes von Androgenrezeptor in voller Länge (AR-FL), Androgenrezeptorvariante 7 (AR-V7) und Androgenrezeptorvariante 5,6,7 (AR-V567) in parallelen Digital-Droplet-PCR-Tests;
wobei:
(i) der/die parallele(n) Digital-Droplet-PCR-Test(s) zum Nachweisen und Quantifizieren für AR-FL optional den folgenden Satz von Primern oder Sonden umfassen: einen Primer gemäß der Sequenz wie dargelegt in SEQ ID NO: 7 und einen Primer gemäß der Sequenz wie dargelegt in SEQ ID NO: 8 und optional eine Sonde gemäß der Sequenz wie dargelegt in SEQ ID NO: 9;
(ii) der/die parallele(n) Digital-Droplet-PCR-Test(s) zum Nachweisen und Quantifizieren für AR-V7 den folgenden Satz von Primern oder Sonden umfassen: einen Primer gemäß der Sequenz wie dargelegt in SEQ ID NO: 11 und einen Primer gemäß der Sequenz wie dargelegt in SEQ ID NO: 12 und optional eine Sonde gemäß der Sequenz wie dargelegt in SEQ ID NO: 13; und
(iii) der/die parallele(n) Digital-Droplet-PCR-Test(s) zum Nachweisen und Quantifizieren für AR-v567es optional den folgenden Satz von Primern oder Sonden umfassen: einen Primer gemäß der Sequenz wie dargelegt in SEQ ID NO: 15 und einen Primer gemäß der Sequenz wie dargelegt in SEQ ID NO: 16 und optional eine Sonde gemäß der Sequenz wie dargelegt in SEQ ID NO: 17.

2. Verfahren nach Anspruch 1, umfassend einen oder mehrere Digital-Droplet-PCR-Tests zum Nachweisen und Quantifizieren von AR-FL, wobei jeder Digital-Droplet-PCR-Test zum Nachweisen und Quantifizieren von AR-FL in einem separaten Droplet durchgeführt wird, das einen Primer gemäß der Sequenz wie dargelegt in SEQ ID NO: 7 und einen Primer gemäß der Sequenz wie dargelegt in SEQ ID NO: 8 umfasst; wobei das Droplet optional ferner eine Sonde gemäß der Sequenz wie dargelegt in SEQ ID NO: 9 umfassen kann.

3. Verfahren nach einem der Ansprüche 1 oder 2, umfassend einen oder mehrere Digital-Droplet-PCR-Tests zum Nachweisen und Quantifizieren für AR-V7, wobei jeder Digital-Droplet-PCR-Test zum Nachweisen und Quantifizieren für AR-V7 in einem separaten Droplet durchgeführt wird, das einen Primer gemäß der Sequenz wie dargelegt in SEQ ID NO: 11 und einen Primer gemäß der Sequenz wie dargelegt in SEQ ID NO: 12 umfasst; wobei das Droplet optional ferner eine Sonde gemäß der Sequenz wie dargelegt in SEQ ID NO: 13 umfassen kann.

4. Verfahren nach einem der Ansprüche 1, 2 oder 3, umfassend einen oder mehrere Digital-Droplet-PCR-Tests zum Nachweisen und Quantifizieren für AR-v567es, wobei jeder Digital-Droplet-PCR-Test zum Nachweisen und Quantifizieren für AR-v567es in einem separaten Droplet durchgeführt wird, das einen Primer gemäß der Sequenz wie dargelegt in SEQ ID NO: 15 und einen Primer gemäß der Sequenz wie dargelegt in SEQ ID NO: 16 umfasst; wobei das Droplet optional ferner eine Sonde gemäß der Sequenz wie dargelegt in SEQ ID NO: 17 umfasst.

5. Verfahren nach einem der Ansprüche 1-3 oder 4, wobei die Primer oder Sonden kovalent oder nicht kovalent an eine oder zwei Markierungen fluoreszierende, chemilumineszierende oder radioaktive Markierungen gebunden sind.

6. Verfahren nach einem der Ansprüche 1-4 oder 5, wobei das Einfangen zirkulierender Krebszellen aus der Probe Isolieren von Zellen, die einen Transferrinrezeptor exprimieren, umfasst, optional ferner umfassend Depletion von CD45+-Zellen aus der Probe vor dem Isolieren von Zellen, die einen Transferrinrezeptor exprimieren.

7. Verfahren nach einem der Ansprüche 1-5 oder 6, wobei das Untersuchungssubjekt gesund ist.

8. Verfahren nach einem der Ansprüche 1-5 oder 6, wobei bei dem Untersuchungssubjekt der Verdacht besteht Krebs oder metastasierenden Krebs oder Prostatakrebs aufzuweisen.

9. Verfahren nach einem der Ansprüche 1-7 oder 8, ferner umfassend Informieren des Untersuchungssubjekts oder des medizinischen Personals, das dem Untersuchungssubjekt medizinische Versorgung bereitstellt, über den Nachweis, die Mengen und/oder die Quantifizierung von Spiegeln von Androgenrezeptor in voller Länge (AR-FL), Androgenrezeptorvariante 7 (AR-V7) oder Androgenrezeptorvariante 5,6,7 (AR-V567), die in den parallelen Digital-Droplet-PCR-Tests nachgewiesen oder quantifiziert wurden.

10. Taxan zur Verwendung in einem Verfahren zum Behandeln von Prostatakrebs bei einem Subjekt, wobei das Verfahren Folgendes umfasst:
(i) Nachweisen der Abwesenheit von AR-V7 in einer Probe durch das Verfahren nach Anspruch 1;
(ii) Bereitstellen des Taxans zur Verabreichung an das Subjekt.

11. Zusammensetzung, umfassend zwei oder mehr Arten von Primern oder Sonden mit mindestens 15 Nukleotiden von SEQ ID NO: 7, 8, 9, 11, 12, 13, 15, 16 und 17, wobei eine oder mehrere Arten von Primern oder Sonden kovalent oder nicht kovalent an eine Markierung gebunden sind, wobei die Zusammensetzung Folgendes umfasst:
einen Primer mit der Sequenz wie dargelegt in SEQ ID NO: 11 und einen Primer, der mindestens 15 Nukleotide gemäß der Sequenz wie dargelegt in SEQ ID NO: 12 umfasst, wobei die Zusammensetzung optional ferner eine Sonde umfassen kann, die mindestens 15 Nukleotide gemäß der Sequenz wie dargelegt in SEQ ID NO: 13 umfasst.

12. Zusammensetzung nach Anspruch 11, umfassend einen Primer, der mindestens 15 Nukleotide gemäß der Sequenz wie dargelegt in SEQ ID NO: 7 umfasst, und einen Primer, der mindestens 15 Nukleotide gemäß der Sequenz wie dargelegt in SEQ ID NO: 8 umfasst, wobei die Zusammensetzung optional ferner eine Sonde umfassen kann, die mindestens 15 Nukleotide gemäß der Sequenz wie dargelegt in SEQ ID NO: 9 umfasst.

13. Zusammensetzung nach einem der Ansprüche 11 oder 12, umfassend einen Primer, der mindestens 15 Nukleotide gemäß der Sequenz wie dargelegt in SEQ ID NO: 15 umfasst, und einen Primer, der mindestens 15 Nukleotide gemäß der Sequenz wie dargelegt in SEQ ID NO: 16 umfasst, wobei die Zusammensetzung optional ferner eine Sonde umfassen kann, die mindestens 15 Nukleotide gemäß der Sequenz wie dargelegt in SEQ ID NO: 17 umfasst.

14. Zusammensetzung nach einem der Ansprüche 11-13, wobei ein oder mehrere Primer oder Sonden kovalent oder nicht kovalent an eine oder zwei fluoreszierende, chemilumineszierende oder radioaktive Markierungen gebunden sind.

15. Verfahren gemäß einem der Ansprüche 1-9, wobei das Einfangen zirkulierender Krebszellen aus der Probe Folgendes umfasst:
a. Depletieren der von dem Untersuchungssubjekt erhaltenen Probe von CD45+-Zellen, um eine CD45+-depletierte Probe zu erhalten; und
b. Anfärben der CD45+-depletierten Probe mit einem markierten Reagens, das an den Transferrinrezeptor (TfR) bindet, um eine TfR-markierte Probe zu erhalten;
c. optional Quantifizieren der TfR-markierten Zellen in der TfR-markierten Probe.

## Revendications

1. Procédé comprenant :
a. la capture de cellules cancéreuses circulantes à partir d'un échantillon provenant d'un sujet testé ;
b. l'extraction d'ARNm pour fournir un échantillon d'ARN ; et
c. la détection et la quantification de chacun parmi le récepteur des androgènes pleine longueur (AR-FL), de la variante 7 du récepteur des androgènes (AR-V7) et de la variante 5, 6, 7 du récepteur des androgènes (AR-V567) dans des dosages PCR en gouttelettes numériques parallèles ;
dans lequel :
(i) le ou les dosages PCR en gouttelettes numériques parallèles pour la détection et la quantification d'AR-FL comprennent éventuellement l'ensemble suivant d'amorces ou de sondes : une amorce selon la séquence telle que définie dans SEQ ID NO : 7 et une amorce selon la séquence telle que définie dans SEQ ID NO : 8, et éventuellement une sonde selon la séquence telle que définie dans SEQ ID NO : 9 ;
(ii) le ou les dosages PCR en gouttelettes numériques parallèles pour la détection et la quantification d'AR-V7 comprennent l'ensemble suivant d'amorces ou de sondes : une amorce selon la séquence telle que définie dans SEQ ID NO : 11 et une amorce selon la séquence telle que définie dans SEQ ID NO : 12, et éventuellement une sonde selon la séquence telle que définie dans SEQ ID NO : 13, et
(iii) le ou les dosages PCR en gouttelettes numériques parallèles pour la détection et la quantification d'AR-v567es comprennent éventuellement l'ensemble suivant d'amorces ou de sondes : une amorce selon la séquence telle que définie dans SEQ ID NO : 15 et une amorce selon la séquence telle que définie dans SEQ ID NO : 16, et éventuellement une sonde selon la séquence telle que définie dans SEQ ID NO : 17.

2. Procédé de la revendication 1, comprenant un ou plusieurs dosages PCR en gouttelettes numériques pour la détection et la quantification d'AR-FL, chaque dosage PCR en gouttelettes numériques pour la détection et la quantification d'AR-FL étant effectué dans une gouttelette distincte comprenant une amorce selon la séquence telle que définie dans SEQ ID NO : 7 et une amorce selon la séquence telle que définie dans SEQ ID NO : 8 ; dans lequel la gouttelette peut éventuellement comprendre en outre une sonde selon la séquence telle que définie dans SEQ ID NO : 9.

3. Procédé de l'une quelconque des revendications 1 ou 2, comprenant un ou plusieurs dosages PCR en gouttelettes numériques pour la détection et la quantification d'AR-V7, chaque dosage PCR en gouttelettes numériques pour la détection et la quantification d'AR-V7 étant effectué dans une gouttelette distincte comprenant une amorce selon la séquence telle que définie dans SEQ ID NO : 11 et une amorce selon la séquence telle que définie dans SEQ ID NO : 12 ; dans lequel la gouttelette peut éventuellement comprendre en outre une sonde selon la séquence telle que définie dans SEQ ID NO : 13.

4. Procédé de l'une quelconque des revendications 1, 2 ou 3, comprenant un ou plusieurs dosages PCR en gouttelettes numériques pour la détection et la quantification d'AR-v567es, chaque dosage PCR en gouttelettes numériques pour la détection et la quantification d'AR-v567es étant effectué dans une gouttelette distincte comprenant une amorce selon la séquence telle que définie dans SEQ ID NO : 15 et une amorce selon la séquence telle que définie dans SEQ ID NO : 16 ; dans lequel la gouttelette comprend éventuellement en outre une sonde selon la séquence telle que définie dans SEQ ID NO : 17.

5. Procédé de l'une quelconque des revendications 1 à 3 ou 4, dans lequel les amorces ou sondes sont liées de manière covalente ou non covalente à un ou deux marqueurs fluorescents, chimioluminescents ou radioactifs marqueurs.

6. Procédé de l'une quelconque des revendications 1 à 4 ou 5, dans lequel la capture de cellules cancéreuses circulantes à partir de l'échantillon comprend l'isolement de cellules qui expriment le récepteur de la transferrine, comprenant éventuellement en outre l'épuisement de cellules CD45+ de l'échantillon avant l'isolement de cellules qui expriment le récepteur de la transferrine.

7. Procédé de l'une quelconque des revendications 1 à 5 ou 6, dans lequel le sujet testé est en bonne santé.

8. Procédé de l'une quelconque des revendications 1 à 5 ou 6, dans lequel le sujet testé est suspecté de comporter un cancer, un cancer métastatique ou un cancer de la prostate.

9. Procédé de l'une quelconque des revendications 1 à 7 ou 8, comprenant en outre l'information du sujet testé ou du personnel médical fournissant des soins médicaux au sujet testé de la détection, des quantités et/ou de la quantification de niveaux de récepteur des androgènes pleine longueur (AR-FL), de la variante 7 du récepteur des androgènes (AR-V7) ou de la variante 5, 6, 7 du récepteur des androgènes (AR-V567) détectés ou quantifiés dans les dosages PCR en gouttelettes numériques parallèles.

10. Taxane pour une utilisation dans un procédé de traitement du cancer de la prostate chez un sujet, le procédé comprenant :
(i) la détection de l'absence d'AR-V7 dans un échantillon par le procédé de la revendication 1 ;
(ii) la fourniture du taxane pour administration au sujet.

11. Composition comprenant deux ou plus types d'amorces ou de sondes avec au moins 15 nucléotides de SEQ ID NO : 7, 8, 9, 11, 12, 13, 15, 16 et 17, dans lequel un ou plusieurs types d'amorces ou de sondes sont liés de manière covalente ou non covalente à un marqueur, dans laquelle la composition comprend :
une amorce comportant la séquence telle que définie dans SEQ ID NO : 11 et une amorce comprenant au moins 15 nucléotides selon la séquence telle que définie dans SEQ ID NO : 12, dans laquelle la composition peut éventuellement comprendre en outre une sonde comprenant au moins 15 nucléotides selon la séquence telle que définie dans SEQ ID NO : 13.

12. Composition de la revendication 11, comprenant une amorce comprenant au moins 15 nucléotides selon la séquence telle que définie dans SEQ ID NO : 7 et une amorce comprenant au moins 15 nucléotides selon la séquence telle que définie dans SEQ ID NO : 8, dans laquelle la composition peut éventuellement comprendre en outre une sonde comprenant au moins 15 nucléotides selon la séquence telle que définie dans SEQ ID NO : 9.

13. Composition de l'une quelconque des revendications 11 ou 12, comprenant une amorce comprenant au moins 15 nucléotides selon la séquence telle que définie dans SEQ ID NO : 15 et une amorce comprenant au moins 15 nucléotides selon la séquence telle que définie dans SEQ ID NO : 16, dans laquelle la composition peut éventuellement comprendre en outre une sonde comprenant au moins 15 nucléotides selon la séquence telle que définie dans SEQ ID NO : 17.

14. Composition de l'une quelconque des revendications 11 à 13, dans laquelle une ou plusieurs amorces ou sondes sont liées de manière covalente ou non covalente à un ou deux marqueurs fluorescents, chimioluminescents ou radioactifs.

15. Procédé de l'une quelconque des revendications 1 à 9, dans lequel la capture de cellules cancéreuses circulantes à partir de l'échantillon comprend :
a. l'épuisement de l'échantillon obtenu à partir du sujet testé de cellules CD45+ pour obtenir un échantillon appauvri en CD45+ ; et
b. la coloration de l'échantillon appauvri en CD45+ avec un réactif marqué qui se lie au récepteur de la transferrine (TfR) pour obtenir un échantillon marqué au TfR ;
c. la quantification éventuelle des cellules marquées au TfR dans l'échantillon marqué au TfR.
